(19) Europäisches Patentamt
European Patent Office
Office européen des brevets

(11) EP 4 685 144 A1

(12) **EUROPEAN PATENT APPLICATION**
published in accordance with Art. 153(4) EPC

(43) Date of publication:
**28.01.2026 Bulletin 2026/05**

(21) Application number: **24800181.0**

(22) Date of filing: **17.04.2024**

(51) International Patent Classification (IPC):
***C07D 413/06*** (2006.01) ***C07D 417/06*** (2006.01)
***C07D 403/06*** (2006.01) ***C07D 403/14*** (2006.01)
***C07D 401/14*** (2006.01) ***C07D 487/04*** (2006.01)
***C07D 513/04*** (2006.01) ***C07D 495/04*** (2006.01)
***C07F 9/572*** (2006.01) ***C07D 498/04*** (2006.01)

(52) Cooperative Patent Classification (CPC):
**C07D 401/14; C07D 403/06; C07D 403/14; C07D 413/06; C07D 417/06; C07D 487/04; C07D 495/04; C07D 498/04; C07D 513/04; C07F 9/572**

(86) International application number:
**PCT/KR2024/005155**

(87) International publication number:
**WO 2024/228496 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.05.2023 KR 20230058488**
**13.07.2023 KR 20230091008**
**01.09.2023 KR 20230116058**
**12.04.2024 KR 20240049453**

(71) Applicant: **SFC Co., Ltd.**
**Cheongju-si, Chungcheongbuk-do 28122 (KR)**

(72) Inventors:
- **LEE, Do Min**
  **Cheongju-si, Chungcheongbuk-do 28122 (KR)**
- **MASANTA, Goutam**
  **Cheongju-si, Chungcheongbuk-do 28122 (KR)**
- **EUM, Min Su**
  **Cheongju-si, Chungcheongbuk-do 28122 (KR)**
- **KANG, Ji Soo**
  **Cheongju-si, Chungcheongbuk-do 28122 (KR)**
- **SI, Ho Young**
  **Cheongju-si, Chungcheongbuk-do 28122 (KR)**
- **KOLAY, Tina**
  **Cheongju-si, Chungcheongbuk-do 28122 (KR)**
- **JANG, Eun Chong**
  **Cheongju-si, Chungcheongbuk-do 28122 (KR)**
- **LIM, Hyun Ju**
  **Cheongju-si, Chungcheongbuk-do 28122 (KR)**
- **SONG, Ju Man**
  **Cheongju-si, Chungcheongbuk-do 28122 (KR)**
- **KIM, Seo Young**
  **Cheongju-si, Chungcheongbuk-do 28122 (KR)**
- **CHOI, Sang Don**
  **Cheongju-si, Chungcheongbuk-do 28122 (KR)**
- **SAHU, Sudipkumar**
  **Cheongju-si, Chungcheongbuk-do 28122 (KR)**

(74) Representative: **BCKIP Part mbB**
**MK1**
**Landsbergerstraße 98, 3.Stock**
**80339 München (DE)**

Remarks:
The complete document including Reference Table(s) and the Sequence Listing(s) can be downloaded from the EPO website

# (54) NON-FLUORESCENT COMPOUND AND USES THEREOF

(57) The present invention relates to a non-fluorescent compound that is useful as a quencher for a fluorescent compound exhibiting emission properties at an excited energy level, and to various applications thereof.

EP 4 685 144 A1

FIG. 1

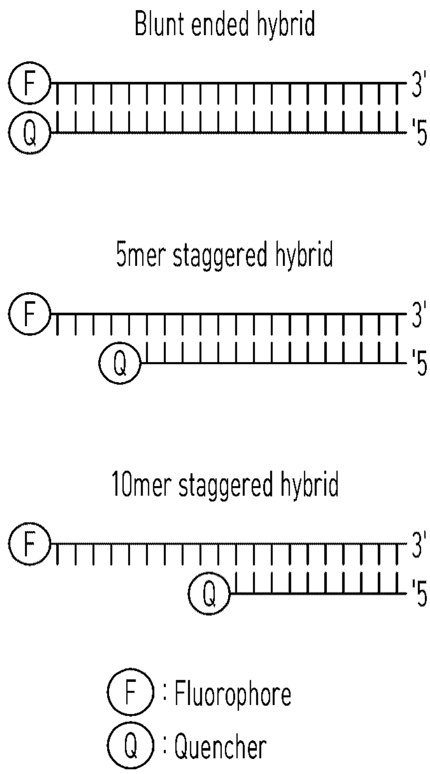
Blunt ended hybrid
F
3'
Q
'5
5mer staggered hybrid
F
3'
Q
'5
10mer staggered hybrid
F
3'
Q
'5
F : Fluorophore
Q : Quencher

## Description

BACKGROUND

### 1. Field of the Invention

The present invention relates to a non-fluorescent compound that is useful as a quencher for a fluorescent compound exhibiting emission properties at an excited energy level, and to various applications thereof.

### 2. Discussion of Related Art

The term "quencher" refers to a non-fluorescent compound capable of quenching the emission properties of a fluorescent compound at a specific wavelength range.

The quenching phenomenon caused by the non-fluorescent compound and/or a chemical moiety included in the non-fluorescent compound can occur via various mechanisms such as static quenching or dynamic quenching.

A representative example of static quenching is ground state quenching, while representative examples of dynamic quenching include fluorescence resonance energy transfer (FRET) and photo-induced electron transfer.

Among the aforementioned quenching mechanisms, FRET typically occurs when the emission spectrum of a fluorescent compound overlaps with the absorption spectrum of the quencher and the fluorescent compound and the quencher are in close proximity (i.e., within the Förster distance) sufficient to induce quenching. In other words, quenching by FRET occurs only within a wavelength range where the emission spectrum of the fluorescent compound and the absorption spectrum of the quencher overlap. As a result, a single quencher designed to induce FRET-mediated quenching generally exhibits quenching properties only within a narrow wavelength range or primarily in the emission spectrum range of fluorescent compounds that are relatively easy to quench.

For example, the commercially available quencher BHQ-1 absorbs light in the 500-550 nm range and can therefore quench the emission properties of fluorescein, which emits at approximately 520 nm. In contrast, BHQ-3 absorbs light in the 650-700 nm range, making it ineffective at quenching fluorescein but effective at quenching Cy5, which emits at approximately 670 nm.

As such, the narrow effective wavelength range of a single quencher inherently limits the types of fluorescent compounds that can be used in combination with it.

Meanwhile, ground state quenching, unlike FRET, does not require spectral overlap between the emission spectrum of the fluorescent compound and the absorption spectrum of the quencher. Generally, ground state quenching, also referred to as contact quenching, occurs when the fluorescent compound and the quencher are in sufficiently close proximity to form a ground state complex. Such a complex can be formed, for example, in a dual-labeled oligonucleotide, prior to hybridization with a target nucleic acid sequence, when the fluorescent compound and the quencher at each terminus are close enough to each other.

Because ground state quenching does not require spectral overlap between the emission spectrum of the fluorescent compound and the absorption spectrum of the quencher, if a single quencher can form ground state complexes with multiple fluorescent compounds having various emission wavelengths, it may exhibit quenching properties across a wide wavelength range, even outside the emission spectrum region of a given fluorescent compound.

Accordingly, if a single non-fluorescent compound is capable of both static quenching and dynamic quenching, it is expected to exhibit quenching effects over a wide wavelength range and with various fluorescent compounds.

SUMMARY OF THE INVENTION

As described above, the present invention aims to provide a non-fluorescent compound that can be used as a quencher across various fluorescent compounds and over a broad wavelength range by enabling both static quenching and dynamic quenching with a single non-fluorescent compound.

In addition, the present invention aims to provide a conjugate, particularly a nucleic acid labeling conjugate, in which the above non-fluorescent compound is used as a quencher.

Further, the present invention aims to provide a composition for detecting nucleic acid, a support for detecting nucleic acid, and a nucleic acid detection method, in which the above conjugate comprising the non-fluorescent compound as a quencher is used.

According to one aspect of the present invention, there is provided a non-fluorescent compound represented by Chemical Formula 1 below, wherein the non-fluorescent compound is capable of reducing fluorescence of various fluorescent compounds or in a wide wavelength range through at least one mechanism selected from static quenching and dynamic quenching.

[Chemical Formula 1]

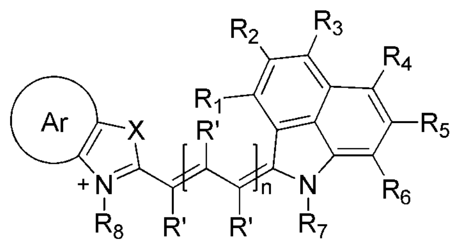

wherein,

Ar is a C5-C50 aryl, a C2-C50 heteroaryl, or a C5-C50 aliphatic-aromatic mixed ring, and is optionally substituted with at least one R",

$R_1$ to $R_6$, R' and R" are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido ($-O^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$,

$R_x$ is a reactive group, or a group comprising a backbone including a hydrocarbon having 1 to 40 carbon atoms to which at least one reactive group is bound,

wherein the reactive group is selected from carboxyl, carboxyl derivatives, carboxylate ($-CO_2^-$), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkane, haloacetamide, imido ester, glycol, halotriazine, hydrazine, acyl halide, alkyl halide and aryl halide,

$R_s$ is a carrier molecule, or a group comprising a backbone including a hydrocarbon having 1 to 40 carbon atoms to which at least one carrier molecule is bound,

$R_7$ and $R_8$ are each independently hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, optionally substituted C5-C50 aryl, optionally substituted C2-C50 heteroaryl, optionally substituted C5-C50 aralkyl, $R_x$ and $R_s$,

X is $CR_{20}R_{21}$, $NR_{22}$, O or S,

$R_{20}$ to $R_{22}$ are each independently hydrogen, optionally substituted C1-C40 alkyl, optionally substituted C1-C40 heteroalkyl, optionally substituted C3-C30 cycloalkyl, optionally substituted C3-C30 heterocycloalkyl, optionally substituted aryl, optionally substituted heteroaryl, $R_x$ and $R_s$, or are bonded to each other to form an optionally substituted 5- to 7-membered ring,

n is an integer of 0 to 5,

optionally, any two adjacent groups among $R_1$ to $R_6$ are bonded to each other to form an optionally substituted 5- to 7-membered ring, and

at least one $R_x$ or at least one $R_s$ is present in the non-fluorescent compound represented by Chemical Formula 1.

According to another aspect of the present invention, there is provided a conjugate comprising the non-fluorescent compound defined herein as a quencher.

According to still another aspect of the present invention, there is provided a composition for detecting nucleic acid comprising the conjugate defined herein.

According to still another aspect of the present invention, there is provided a support for detecting nucleic acid

comprising the non-fluorescent compound defined herein as a quencher.

According to still another aspect of the present invention, there is provided a method for detecting nucleic acid using the conjugate comprising the non-fluorescent compound defined herein as a quencher.

The non-fluorescent compound defined herein is capable of exhibiting quenching phenomena for various fluorescent compounds by being capable of both static quenching and dynamic quenching. In addition, the non-fluorescent compound defined herein is capable of exhibiting quenching phenomena by static quenching, unlike conventional quenchers that operate by dynamic quenching, and thus is capable of exhibiting quenching phenomena in a broader wavelength range.

Accordingly, the non-fluorescent compound defined herein has an advantage in that it can be combined with a wider variety of fluorescent compounds with a single quencher, because the wavelength range in which effective quenching properties can be exhibited is wide even with a single material.

## BRIEF DESCRIPTION OF THE DRAWINGS

FIG. 1 schematically illustrates the hybrid of a forward probe and a reverse probe prepared in Experimental Example 2.

FIGS. 2 and 3 show the PCR experimental results obtained using dual-labeled oligonucleotides according to Experimental Example 4.

FIGS. 4 to 6 show the PCR experimental results obtained using dual-labeled oligonucleotides according to Experimental Example 5.

FIGS. 7 and 8 show the PCR experimental results obtained using dual-labeled oligonucleotides according to Experimental Example 6.

FIGS. 9 to 12 show the PCR experimental results obtained using dual-labeled oligonucleotides according to Experimental Example 7.

FIG. 13 shows the PCR experimental results obtained using triple-labeled oligonucleotides according to Experimental Example 8.

## DETAILED DESCRIPTION OF EXEMPLARY EMBODIMENTS

For ease of understanding the present invention, certain terms are defined herein for convenience. Unless otherwise defined herein, scientific and technical terms used in the present invention shall have meanings as generally understood by those of ordinary skill in the art.

In addition, unless specifically indicated by the context, singular terms also include their plural forms, and plural terms may also include their singular forms.

Furthermore, unless otherwise defined herein, any functional group shall be construed according to the definitions commonly used in the technical field to which the present invention pertains.

### Non-fluorescent Compound

According to one aspect of the present invention, a non-fluorescent compound capable of reducing fluorescence through at least one mechanism selected from static quenching and dynamic quenching is provided.

The wavelength range in which the non-fluorescent compound can exhibit quenching properties may vary depending on the emission spectrum of a counterpart fluorescent compound, but quenching properties can be exhibited in a wavelength range of 200-1000 nm.

In addition, the non-fluorescent compound defined herein can quench the emission properties of fluorescent compounds through both static quenching and dynamic quenching.

For example, the non-fluorescent compound can reduce fluorescence of a fluorescent compound having an absorption or emission spectrum of 300-900 nm, preferably 550-900 nm, and more preferably 550-800 nm through dynamic quenching. Specifically, the dynamic quenching may be by a FRET mechanism. In addition, the non-fluorescent compound can reduce fluorescence of a fluorescent compound having an absorption or emission spectrum of 200-1000 nm, preferably 300-900 nm, through static quenching. Specifically, the static quenching may be induced by the non-fluorescent compound forming a ground state complex with the fluorescent compound.

Accordingly, since the non-fluorescent compound defined herein is capable of reducing fluorescence over a wide wavelength range (for example, 200-1000 nm) through two quenching mechanisms (dynamic quenching and static quenching), it has an advantage in that it can be used as a counterpart for a wider variety of fluorescent compounds than quenchers that reduce fluorescence through only a single quenching mechanism.

The non-fluorescent compound is represented by Chemical Formula 1 below. In addition, Chemical Formula 1 may exist as a resonance structure represented by Chemical Formula 1-1 below.

[Chemical Formula 1]

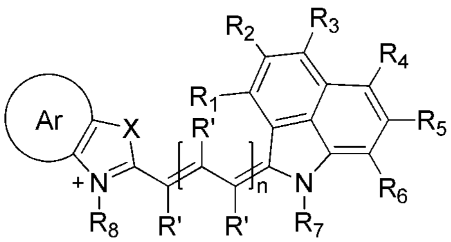

[Chemical Formula 1-1]

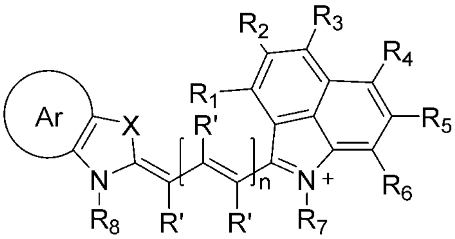

Herein, Ar is a C5-C50 aryl, a C2-C50 heteroaryl, or a C5-C50 aliphatic-aromatic mixed ring, and n is an integer of 0 to 5.

As used herein, the term "aryl" refers to an unsaturated aromatic ring including a single ring or multiple rings (preferably 1 to 6 rings) that are fused or linked through covalent bonds (for example, single bonds), and the term "heteroaryl" refers to an unsaturated aromatic ring in which at least one carbon atom in the aryl is replaced with a heteroatom (for example, nitrogen, oxygen, or sulfur).

Non-limiting examples of the aryl include phenyl, biphenyl, terphenyl, naphthyl, anthryl, phenanthrenyl, pyrenyl, fluoranthenyl, and analogs thereof that are fused.

Non-limiting examples of the heteroaryl include furyl, tetrahydrofuryl, pyrrolyl, pyrrolidinyl, thienyl, tetrahydrothienyl, oxazolyl, isoxazolyl, triazolyl, thiazolyl, isothiazolyl, pyrazolyl, pyrazolidinyl, oxadiazolyl, thiadiazolyl, imidazolyl, imidazolinyl, pyridyl, pyridazinyl, triazinyl, piperidinyl, morpholinyl, thiomorpholinyl, pyrazinyl, piperazinyl, pyrimidinyl, naphthyridinyl, benzofuranyl, benzothienyl, indolyl, indolinyl, indolizinyl, indazolyl, quinolizinyl, quinolyl, isoquinolyl, cinnolinyl, phthalazinyl, quinazolinyl, quinoxalinyl, pteridinyl, quinuclidinyl, carbazolyl, acridinyl, phenazinyl, phenothiazinyl, phenoxazinyl, purinyl, benzimidazolyl, benzothiazolyl, and analogs thereof that are fused.

As used herein, the term "aliphatic-aromatic mixed ring" refers to a multi-ring structure (preferably having 2 to 6 rings) in which an aliphatic ring and an aromatic ring are fused or connected to each other by a covalent bond (for example, a single bond). In addition, as used herein, the term "aliphatic ring" refers to a saturated hydrocarbon ring (cycloalkyl) or a saturated hydrocarbon ring (heterocycloalkyl) in which at least one carbon atom in the hydrocarbon ring is replaced with a heteroatom, and corresponds to a cyclic structure of an alkyl or heteroalkyl.

Non-limiting examples of the aliphatic ring include cyclopentyl, cyclohexyl, cyclohexenyl, cycloheptyl, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothiophen-2-yl, tetrahydrothiophen-3-yl, 1-piperazinyl, and 2-piperazinyl.

For example, Ar may be selected from [Ar-1] to [Ar-6] below.

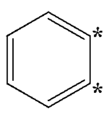

[Ar-1]

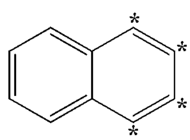

[Ar-2]

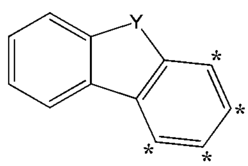

[Ar-3]

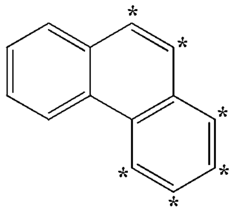

[Ar-4]

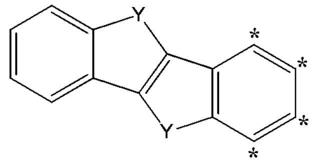

[Ar-5]

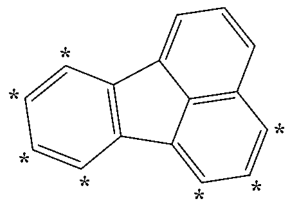

[Ar-6]

In [Ar-1] to [Ar-6], * denotes a carbon position capable of being bonded to the nitrogen-containing five-membered ring of Chemical Formula 1, and Y is each independently $CR_{23}R_{24}$, $NR_{25}$, O, or S.

$R_{23}$ to $R_{25}$ are each independently selected from optionally substituted C1-C40 alkyl, optionally substituted C1-C40 heteroalkyl, optionally substituted C3-C30 cycloalkyl, optionally substituted C3-C30 heterocycloalkyl, optionally substituted aryl, optionally substituted heteroaryl, $R_x$, and $R_s$, or are bonded to each other to form an optionally substituted 5- to 7-membered ring.

Ar may be optionally substituted with at least one R". That is, Ar is a substituted or unsubstituted ring. When Ar is a substituted ring, at least one carbon atom in Ar may be bound to R". In addition, when Ar is a substituted ring, R" bound to at least one carbon atom in Ar does not substantially reduce the quenching property of the non-fluorescent compound represented by Chemical Formula 1. Similarly, [Ar-1] to [Ar-6] may be optionally substituted with at least one R".

Various modifications of Ar in Chemical Formula 1 can be found from the structures of Dye 1 to Dye 97 attached herein.

$R_1$ to $R_6$, R' and R" are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido (-O-), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$.

The functional groups defined above do not substantially reduce the quenching property of the non-fluorescent compound represented by Chemical Formula 1.

In the non-fluorescent compound represented by Chemical Formula 1, when Ar has the structure of [Ar-1] or [Ar-2], it is preferable that the compound does not include water-soluble functional groups such as $NH_4^+$, $PO_4^-$, and $SO_3^-$ (including $SO_3H$, alkyl sulfonate, aryl sulfonate, and the like). Accordingly, the non-fluorescent compound in which Ar has the structure of [Ar-1] or [Ar-2] is substantially lipophilic. That the non-fluorescent compound is substantially lipophilic means that the compound exhibits a solubility in an aqueous solvent of 30% or less, 20% or less, or 10% or less, or is not soluble at all.

The hydrogen used herein may be protium ($^1H$), deuterium ($^2H$), or tritium ($^3H$).

The heteroatom used herein refers to an atom other than carbon and hydrogen, and more specifically, the heteroatom is an atom that can be used to substitute carbon in a backbone structure composed of carbon (more specifically, a hydrocarbon), and examples thereof include nitrogen, oxygen, sulfur, phosphorus, silicon, and selenium.

As used herein, a substituent denoted as Ca-Cb means a substituent having a carbon atom number of a to b.

For example, a Ca-Cb alkyl means a saturated aliphatic substituent having a carbon atom number of a to b, including straight-chain alkyl and branched alkyl. The term "branched alkyl" refers to a saturated aliphatic substituent in which another straight-chain alkyl is bound to any carbon of a straight-chain alkyl that is not branched. Specifically, examples of alkyl include methyl, ethyl, n-propyl, i-propyl, n-butyl, s-butyl, i-butyl, t-butyl, pent-1-yl, pent-2-yl, pent-3-yl, 3-methylbut-1-yl, 3-methylbut-2-yl, 2-methylbut-2-yl, 2,2,2-trimethyleth-1-yl, n-hexyl, n-heptyl, and n-octyl.

As used herein, heteroalkyl means a stable straight-chain or branched alkyl having the total number of specified atoms and at least one (e.g., 1 to 15) heteroatom selected from the group consisting of O, N, Si, and S.

The carbon and heteroatoms of the heteroalkyl group may be oxidized (for example, to form a ketone, N-oxide, sulfone, etc.), and a nitrogen atom may be quaternized. Non-limiting examples of heteroalkyl groups include $-CH_2-CH_2-O-CH_3$, $-CH_2-CH_2-NH-CH_3$, $-CH_2-CH_2-N(CH_3)_2$, $-C(=O)-NH-CH_2-CH_2-NH-CH_3$, $-C(=O)-N(CH_3)-CH_2-CH_2-N(CH_3)_2$, $-C(=O)-NH-CH_2-CH_2-NH-C(=O)-CH_2-CH_3$, $-C(=O)-N(CH_3)-CH_2-CH_2-N(CH_3)-C(=O)-CH_2-CH_3$, $-O-CH_2-CH_2-CH_2-NH(CH_3)$, $-O-CH_2-CH_2-CH_2-N(CH_3)_2$, $-O-CH_2-CH_2-CH_2-NH-C(=O)-CH_2-CH_3$, $-O-CH_2-CH_2-CH_2-N(CH_3)-C(=O)-CH_2-CH_3$, $-CH_2-CH_2-CH_2-NH(CH_3)$, $-O-CH_2-CH_2-CH_2-N(CH_3)_2$, - $CH_2-CH_2-CH_2-NH-C(=O)-CH_2-CH_3$, $-CH_2-CH_2-CH_2-N(CH_3)-C(=O)-CH_2-CH_3$, $-CH_2-S-CH_2-CH_3$, $-CH_2-CH_2-S(O)-CH_3$, $-NH-CH_2-CH_2-NH-C(=O)-CH_2-CH_3$, $-CH_2-CH_2-S(O)_2-CH_3$, $-CH_2-CH_2-0-CF_3$, and $-Si(CH_3)_3$.

As used herein, alkoxy includes both -O-(alkyl) groups and -O-(unsubstituted cycloalkyl) groups. Specifically, examples include methoxy, ethoxy, propoxy, isopropoxy, n-butoxy, tert-butoxy, sec-butoxy, n-pentoxy, n-hexyloxy, 1,2-dimethylbutoxy, cyclopropyloxy, cyclobutyloxy, cyclopentyloxy, and cyclohexyloxy, but are not limited thereto.

As used herein, acyl refers to -C(=O)-alkyl, -C(=O)-heteroalkyl, -C(=O)-cycloalkyl (substituted or unsubstituted), or -C(=O)-heterocycloalkyl (substituted or unsubstituted), and the alkyl, heteroalkyl, cycloalkyl, or heterocycloalkyl bound to the carbonyl carbon of the acyl is as defined herein. on-limiting examples of acyl include $-C(=O)CH_3$, $-C(=O)CH_2CH_3$, - $C(=O)CH(CH_3)_2$, $-C(=O)C(CH_3)_3$, -C(=O)-phenyl (substituted or unsubstituted), -C(=O)-cyclopropyl (substituted or unsubstituted), -C(=O)-cyclobutyl (substituted or unsubstituted), - C(=O)-cyclopentyl (substituted or unsubstituted), -C(=O)-cyclohexyl (substituted or unsubstituted), and -C(=O)-pyridyl (substituted or unsubstituted).

As used herein, halogen means fluoro (-F), chloro (-Cl), bromo (-Br), or iodo (-I), and an alkyl halide or haloalkyl means an alkyl substituted with the above-described halogen. For example, halomethyl means a methyl ($-CH_2X$, $-CHX_2$, or $-CX_3$) in which at least one hydrogen of methyl is replaced with a halogen. Thus, an alkyl halide means a substituent in which at least one halogen is bound to at least one carbon atom constituting an alkyl.

As used herein, aralkyl is a substituent in which an aryl is substituted on a carbon of an alkyl, and is a generic term for $-(CH_2)_nAr$. Examples of aralkyl include benzyl ($-CH_2C_6H_5$) and phenethyl ($-CH_2CH_2C_6H_5$).

As used herein, "optionally substituted" has the same meaning as the term "substituted or unsubstituted," and means that one or more hydrogen atoms present in any substituent may each independently be replaced with a non-hydrogen substituent. However, when any substituent is a substituted substituent, the substituent does not include any substituents or substitution patterns that are sterically impracticable.

When any substituent among $R_1$ to $R_6$, R', and R" is a substituted substituent, at least one substituent is bound to any carbon atom of the substituent (in the case of a substituent in which no carbon atom is present, to any atom to which the substituent can be bound, for example, nitrogen, oxygen, or sulfur).

The substituent is selected from C1-C40 alkyl, C1-C40 heteroalkyl, C2-C40 alkenyl, C2-C40 alkynyl, C3-C20 cycloalkyl, C3-C20 cycloalkenyl, C2-C20 heterocycloalkyl, optionally substituted C5-C50 aryl, C2-C50 heteroaryl, C5-C50 aralkyl, Rx, and Rs, preferably from C1-C40 alkyl, C1-C40 heteroalkyl, C2-C40 alkenyl, C2-C40 alkynyl, Rx, and Rs, and more preferably from C1-C40 alkyl, C1-C40 heteroalkyl, $R_x$, and $R_s$.

$R_x$ is a reactive group, or a group comprising a backbone including a hydrocarbon having 1 to 40 carbon atoms to which at least one reactive group is bound.

The backbone refers to a series of bonds for linking a reactive group or a carrier molecule to the base represented by Chemical Formula 1.

For example, the backbone may have an alkyl or heteroalkyl having 1 to 40 carbon atoms as a main chain. The main chain may further include multiple alkyl or heteroalkyl groups connected through an intermediate functional group such as an amide or ester.

The backbone may include at least one hetero atom selected from O, S, N, P, and Si. The backbone may be linear or non-linear, and may be composed of any combination of single, double, and triple bonds.

The backbone may, for example, be selected from alkyl, alkenyl, aryl, heteroaryl, cycloalkyl, heterocycloalkyl, -C(O)-, -C(O)O-, $-NR_L-$, -O-, -S-, $-C(O)NR_L-$, -S(O)o-, - $S(O)NR_L-$, $-S(O_2)NR_L-$, $-P(O)_P-$, and the structures (a) to (o) below, or may be composed of a combination thereof.

(a) $-(CH_2)_q-$

(b) $-(CH_2)_q-O-(CH_2)_q-$

(c) 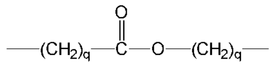

(d) 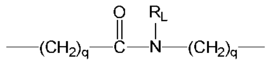

(e) 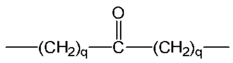

(f) 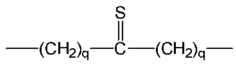

(8) $-(CH_2)_q-S-(CH_2)_q-$

(h) $-(CH_2)_q-O-(CH_2)_q-O-(CH_2)_q-$

(i) $-(CH_2)_q-S-(CH_2)_q-S-(CH_2)_q-$

(j) 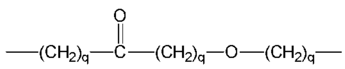

(k) 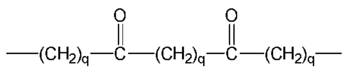

(l) 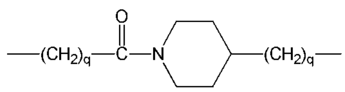

(m) 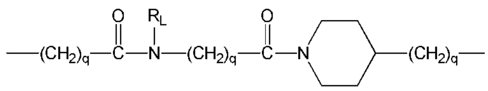

(n) 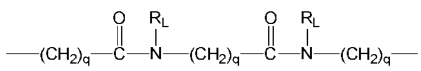

(o) 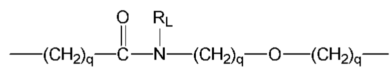

Herein, $R_L$ is selected from hydrogen, C1-C40 alkyl, and C1-C40 heteroalkyl, q is the same or different and is independently an integer of 0 to 10, o is an integer of 1 to 2, and p is an integer of 1 to 4.

The reactive group is a functional group selected from carboxyl, carboxyl derivatives, carboxylate (-CO2-), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkane, haloacetamide, imido ester, glycol, halotriazine, hydrazine, acyl halide, alkyl halide, and aryl halide.

In addition, the reactive group may not participate in any reaction as it is protected by a protecting group.

The protecting group is introduced by chemically converting the reactive group to provide reaction selectivity of at least some of the reactive groups during a series of chemical or biological reactions.

The protecting group includes a protecting group derived from an alcohol, a protecting group derived from an

amine, a protecting group derived from a carbonyl, a protecting group derived from a carboxylic acid, a protecting group derived from a phosphate, or a protecting group derived from an alkyne.

For example, when the reactive group is hydroxyl, the protecting group may be acetyl, benzoyl, benzyl, β-methoxyethoxymethyl ether, dimethoxytrityl, methoxymethyl ether, methoxytrityl, p-methoxybenzyl ether, p-methoxyphenyl ether, methylthiomethyl ether, silyl ether, trityl, or an analogue thereof. When the reactive group is amino, the protecting group may be tert-butyl carbamate, benzyl carbamate, acetamide, phthalimide, p-toluenesulfonamide, or an analogue thereof. When $R_x$ is carbonyl or carboxyl, the protecting group may be acetal, ketal, dithiane, methyl ester, benzyl ester, tert-butyl ester, silyl ester, or an analogue thereof. In addition, other preferred examples of protecting groups may be found in the following reference (Greene et al., PROTECTIVE GROUPS IN ORGANIC SYNTHESIS, Third Edition, John Wiley & Sons, New York, 1999; https://en.wikipedia.org/wiki/Protecting_group).

$R_s$ is a carrier molecule, or a group comprising a backbone including a hydrocarbon having 1 to 40 carbon atoms to which at least one carrier molecule is bound, and the definition of the backbone is the same as that of $R_x$.

The carrier molecule refers to a substance that carries the non-fluorescent compound represented by Chemical Formula 1.

The carrier molecule may be selected from amino acids, polymers of amino acids, peptides, proteins, neurotoxins, phallotoxins, cytokines, toxins, protease substrates, protein kinase substrates, enzymes, antibodies, antibody fragments, lectins, glycoproteins, histones, albumins, lipoproteins, avidin, streptavidin, protein A, protein G, protein L, phycobiliproteins, fluorescent proteins, hormones, growth factors, nucleic acid bases, nucleosides, nucleotides, nucleic acid polymers, nucleotide analogues, nucleoside analogues, nucleoside triphosphates, deoxynucleoside triphosphates (dNTPs), dideoxynucleoside triphosphates (ddNTPs), organic or inorganic nanoparticles, organic or inorganic microparticles, haptens, carbohydrates, polysaccharides, lipids, ion-complexing moieties such as crown ethers, PEG groups, organic polymers, and inorganic polymers.

In addition, the carrier molecule may be protected by a protecting group so as not to participate in any reaction. In this case, the carrier molecule being protected by a protecting group means that a functional group present in the carrier molecule is protected by a protecting group. The definition of the protecting group of the carrier molecule is the same as the protecting group of the reactive group described above.

Herein, a derivative refers to a compound similar to any compound that is obtained by chemically modifying a part of the compound.

A carboxyl derivative is a functional group that can be converted to a carboxyl group, and may be an N-hydroxysuccinimide ester, N-hydroxybenzotriazole ester, acyl halide, acyl imidazole, thioester, alkyl ester, alkenyl ester, alkynyl ester, or aromatic ester.

The carboxyl derivative may be optionally substituted, and examples thereof include trifluoromethyl ester, pentafluorophenyl ester, p-nitrophenyl ester, tetrafluorophenyl ester, sulfo-succinimidyl ester, sulfo-dichlorophenyl ester, and sulfo-tetrafluorophenyl ester.

A 1,2,4,5-tetrazine derivative may be 3,6-dimethyl-1,2,4,5-tetrazine, 3,6-diphenyl-1,2,4,5-tetrazine, or 3-methyl-6-phenyl-1,2,4,5-tetrazine.

A cycloalkyne derivative may be a compound that participates in a bipolar cycloaddition, an inverse-electron demand Diels-Alder reaction, or a strain-promoted azide-alkyne cycloaddition (SPAAC) reaction. For example, cyclooctynes such as OCT, COMBO (ALO), MOFO, DIFO, DIBO, BARAC, DIBAC (ADIBO), DBCO, DIMAC, BCN, or TMTH may be used as cycloalkyne derivatives participating in the SPAAC reaction.

In addition, examples of any derivative specified herein may be confirmed through documents known in the relevant technical field.

A dienophile is an alkene or alkyne capable of undergoing a Diels-Alder reaction with a diene, and may specifically be an alkene or alkyne in which an electron-withdrawing group is directly bonded to an $sp^2$-hybridized carbon or an sp-hybridized carbon.

The term "electron-withdrawing group" refers to a functional group that has a tendency to attract electrons through an inductive effect or a resonance effect and may also be referred to as a "deactivating group."

Examples of the electron-withdrawing group include trifluoromethylsulfonyl ($-SO_2CF_3$), substituted or unsubstituted ammonium ($-NR_3^+$), nitro, sulfonic acid ($-SO_3H$), sulfonyl ($-SO_2R$), nitrile, trihalomethyl ($-CF_3$, $-CCl_3$, $-CBr_3$, $-CI_3$), haloformyl (-COCl, -COBr, -COI), formyl (-CHO), acyl (-COR), carboxyl ($-CO_2H$), substituted ester ($-CO_2R$), substituted or unsubstituted aminocarbonyl ($-CONR_2$), and nitroso (-N=O).

In addition, unless otherwise defined herein, the electron-withdrawing group may further include functional groups other than those exemplified above that exhibit a tendency to attract electrons.

$R_7$ and $R_8$ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, optionally substituted C5-C50 aryl, optionally substituted C2-C50 heteroaryl, optionally substituted C5-C50 aralkyl, $R_x$, and $R_s$.

X is $CR_{20}R_{21}$, $NR_{22}$, O, or S. When X is $CR_{20}R_{21}$, $R_{20}$ and $R_{21}$ are each independently selected from optionally substituted C1-C40 alkyl, optionally substituted C1-C40 heteroalkyl, optionally substituted C3-C30 cycloalkyl, optionally substituted C3-C30 heterocycloalkyl, optionally substituted aryl, optionally substituted heteroaryl, $R_x$, and $R_s$.

When X is $NR_{22}$, $R_{22}$ is each independently selected from optionally substituted C1-C40 alkyl, optionally substituted C1-C40 heteroalkyl, optionally substituted C3-C30 cycloalkyl, optionally substituted C3-C30 heterocycloalkyl, optionally substituted aryl, optionally substituted heteroaryl, $R_x$, and $R_s$.

n is an integer of 0 to 5. When n is 0, one R' is present, and when n is 1 or greater, a plurality of R' groups are present. When a plurality of R' groups are present in the polymethine due to n being 1 or greater, each of R' is independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido ($-O^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$, and $R_s$.

Any two adjacent substituents among $R_1$ to $R_6$ may be bonded to each other to form an optionally substituted 5- to 7-membered ring.

The ring formed by bonding two adjacent substituents among $R_1$ to $R_6$ may be an aliphatic ring (cycloalkyl, heterocycloalkyl) or an aromatic ring (aryl, heteroaryl).

The ring may be a single ring or a polycyclic ring, and when the ring is a polycyclic ring, the ring may be an aliphatic-aliphatic mixed ring, an aliphatic-aromatic mixed ring, or an aromatic-aromatic mixed ring.

As described above, a mixed ring means a polycyclic ring (preferably having 2 to 6 rings) in which the same or different rings are fused or connected by a covalent bond (for example, a single bond).

Two adjacent substituents among $R_1$ to $R_6$ that are bonded to each other to form a ring may be substituted with a substituent selected from C1-C40 alkyl, C1-C40 heteroalkyl, C2-C40 alkenyl, C2-C40 alkynyl, C3-C20 cycloalkyl, C3-C20 cycloalkenyl, C2-C20 heterocycloalkyl, optionally substituted C5-C50 aryl, C2-C50 heteroaryl, C5-C50 aralkyl, $R_x$, and $R_s$.

Variants in which two adjacent substituents among $R_1$ to $R_6$ are bonded to each other to form a 5- to 7-membered ring can be found in the structures of Dye 1 to Dye 97 attached herein.

When Ar is substituted with a plurality of R" groups, two adjacent R" groups may be bonded to each other to form an optionally substituted 5- to 7-membered ring.

The ring formed by bonding two adjacent R" groups may be an aliphatic ring (cycloalkyl, heterocycloalkyl) or an aromatic ring (aryl, heteroaryl). The ring may be a single ring or a polycyclic ring, and when the ring is a polycyclic ring, it may be an aliphatic-aliphatic mixed ring, an aliphatic-aromatic mixed ring, or an aromatic-aromatic mixed ring. As described above, a mixed ring refers to a polycyclic ring (preferably having 2 to 6 rings) in which the same or different rings are fused or connected by a covalent bond (for example, a single bond).

The ring formed by bonding two adjacent R" groups may be substituted with a substituent selected from C1-C40 alkyl, C1-C40 heteroalkyl, C2-C40 alkenyl, C2-C40 alkynyl, C3-C20 cycloalkyl, C3-C20 cycloalkenyl, C2-C20 heterocycloalkyl, optionally substituted C5-C50 aryl, C2-C50 heteroaryl, C5-C50 aralkyl, $R_x$, and $R_s$.

Variants in which two adjacent R" groups are bonded to each other to form a 5- to 7-membered ring can be found in the structures of Dye 1 to Dye 97 attached herein.

Two adjacent substituents among $R_{20}$ to $R_{22}$ may be bonded to each other to form an optionally substituted 5- to 7-membered ring.

The ring formed by bonding two adjacent substituents among $R_{20}$ to $R_{22}$ may be an aliphatic ring (cycloalkyl, heterocycloalkyl) or an aromatic ring (aryl, heteroaryl). The ring may be a single ring or a polycyclic ring, and when the ring is a polycyclic ring, it may be an aliphatic-aliphatic mixed ring, an aliphatic-aromatic mixed ring, or an aromatic-aromatic mixed ring. As described above, a mixed ring refers to a polycyclic ring (preferably having 2 to 6 rings) in which the same or different rings are fused or connected by a covalent bond (for example, a single bond).

The ring formed by bonding two adjacent substituents among $R_{20}$ to $R_{22}$ may be substituted with a substituent selected from C1-C40 alkyl, C1-C40 heteroalkyl, C2-C40 alkenyl, C2-C40 alkynyl, C3-C20 cycloalkyl, C3-C20 cycloalkenyl, C2-C20 heterocycloalkyl, optionally substituted C5-C50 aryl, C2-C50 heteroaryl, C5-C50 aralkyl, $R_x$, and $R_s$.

Variants in which two adjacent substituents among $R_{20}$ to $R_{22}$ are bonded to each other to form a 5- to 7-membered ring can be found in the structures of Dye 1 to Dye 97 attached herein.

Thus, when two adjacent substituents of the non-fluorescent compound represented by Chemical Formula 1 are bonded to each other to form a ring, the ring may be phenyl, naphthyl, carbazole, fluorene, dibenzofuran, dibenzothiophene, fluoranthene, or the like, and is not necessarily limited thereto.

The non-fluorescent compound represented by Chemical Formula 1 is characterized in that at least one $R_x$ or at least one $R_s$ is present.

The non-fluorescent compound represented by Chemical Formula 1 may further comprise a counter ion. The counter ion is an organic or inorganic anion and may be appropriately selected in consideration of the solubility and stability of the quencher. Specifically, examples of the counter ion include inorganic acid anions such as hexafluorophosphate ion, halogen ion, phosphate ion, perchlorate ion, periodate ion, hexafluoroantimonate ion, hexafluorostannate ion, fluoroborate ion, and tetrafluoroborate ion; and organic acid anions such as thiocyanate ion, benzenesulfonate ion, naphthalenesulfonate ion, p-toluenesulfonate ion, alkylsulfonate ion, benzoate ion, alkylcarboxylate ion, trihaloalkylcarboxylate ion, alkylsulfonate ion, trihaloalkylsulfonate ion, and nicotinate ion. In addition, metal compound ions such as bisphenyldithiol, thiobisphenol chelate, and bisdiol-α-diketone, metal ions such as sodium and potassium, and quaternary ammonium salts may also be used as the counter ion.

In another embodiment, the non-fluorescent compound may be represented by Chemical Formula 2 below. The non-fluorescent compound represented by Chemical Formula 2 has a structure in which Ar in the non-fluorescent compound represented by Chemical Formula 1 is [Ar-1].

[Chemical Formula 2]

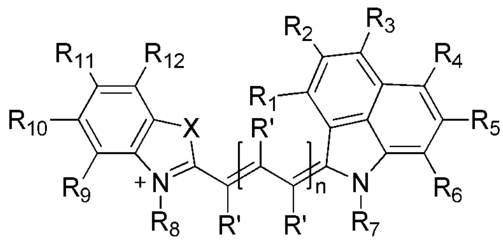

In Chemical Formula 2, the definitions of $R_1$ to $R_8$, R', $R_x$, $R_s$, n, X, and $R_{20}$ to $R_{22}$ are the same as those in Chemical Formula 1, and unless otherwise defined, are the same as those in Chemical Formula 1.

$R_9$ to $R_{12}$ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido (-O-), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$.

Two adjacent substituents among $R_9$ to $R_{12}$ may be bonded to each other to form an optionally substituted 5- to 7-membered ring.

At least one $R_x$ or at least one $R_s$ is present in the non-fluorescent compound represented by Chemical Formula 2.

In another embodiment, the non-fluorescent compound may be represented by Chemical Formula 3 below.

The non-fluorescent compound represented by Chemical Formula 2 has a structure in which $R_{10}$ and $R_{11}$ are bonded to each other to form ring A in the non-fluorescent compound represented by Chemical Formula 3.

[Chemical Formula 3]

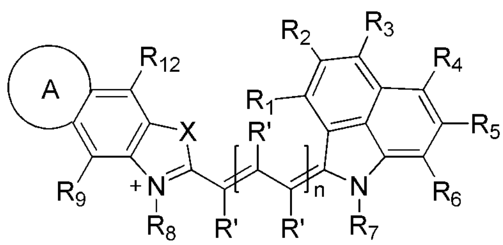

In Chemical Formula 3, the definitions of $R_1$ to $R_8$, R', R", $R_x$, $R_s$, n, X, and $R_{20}$ to $R_{22}$ are the same as those in Chemical Formula 1, and unless otherwise defined, are the same as those in Chemical Formula 1.

Ring A is a C5-C50 aryl, a C2-C50 heteroaryl, or a C5-C50 aliphatic-aromatic mixed ring, and is optionally substituted with at least one R".

$R_9$ and $R_{12}$ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido ($-O^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$.

Two adjacent substituents among R", $R_9$, and $R_{12}$ may be bonded to each other to form an optionally substituted 5- to 7-membered ring.

At least one $R_x$ or at least one $R_s$ is present in the non-fluorescent compound represented by Chemical Formula 3.

In another embodiment, the non-fluorescent compound may be represented by Chemical Formula 4 below. The non-fluorescent compound represented by Chemical Formula 4 has a structure in which $R_{11}$ and $R_{12}$ are bonded to each other to form ring B in the non-fluorescent compound represented by Chemical Formula 2.

[Chemical Formula 4]

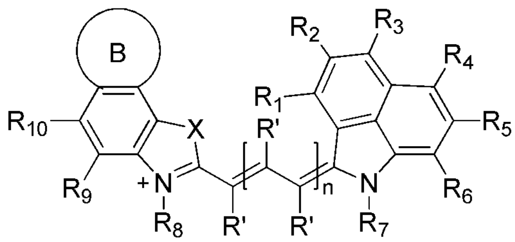

In Chemical Formula 4, the definitions of $R_1$ to $R_8$, R', R", $R_x$, $R_s$, n, X, and $R_{20}$ to $R_{22}$ are the same as those in Chemical Formula 1, and unless otherwise defined, are the same as those in Chemical Formula 1.

Ring B is a C5-C50 aryl, a C2-C50 heteroaryl, or a C5-C50 aliphatic-aromatic mixed ring, and is optionally substituted with at least one R".

$R_9$ and $R_{10}$ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido ($-O^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$.

R" and $R_{10}$ may be bonded to each other to form an optionally substituted 5- to 7-membered ring.

At least one $R_x$ or at least one $R_s$ is present in the non-fluorescent compound represented by Chemical Formula 4.

In another embodiment, the non-fluorescent compound may be represented by Chemical Formula 5 below. The non-fluorescent compound represented by Chemical Formula 5 has a structure in which $R_9$ and $R_{10}$ are bonded to each other to form ring C in the non-fluorescent compound represented by Chemical Formula 2.

[Chemical Formula 5]

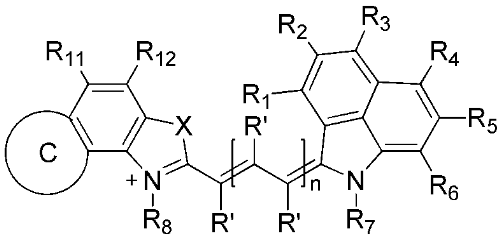

In Chemical Formula 5, the definitions of $R_1$ to $R_8$, R', R", $R_x$, $R_s$, n, X, and $R_{20}$ to $R_{22}$ are the same as those in Chemical Formula 1, and unless otherwise defined, are the same as those in Chemical Formula 1.

Ring C is a C5-C50 aryl, a C2-C50 heteroaryl, or a C5-C50 aliphatic-aromatic mixed ring, and is optionally substituted with at least one R".

$R_{11}$ and $R_{12}$ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido (-O$^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$.

R" and $R_{11}$ may be bonded to each other to form an optionally substituted 5- to 7-membered ring.

At least one $R_x$ or at least one $R_s$ is present in the non-fluorescent compound represented by Chemical Formula 5.

In another embodiment, the non-fluorescent compound may be represented by Chemical Formula 6 below. The non-fluorescent compound represented by Chemical Formula 6 has a structure in which Ar is a naphthalene ring bonded to X in the non-fluorescent compound represented by Chemical Formula 1.

[Chemical Formula 6]

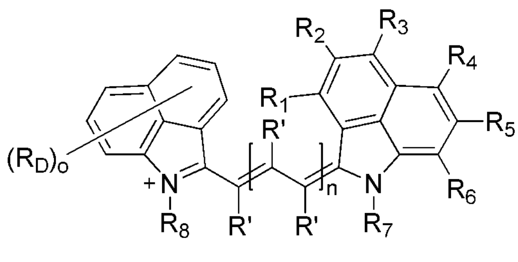

In Chemical Formula 6, the definitions of $R_1$ to $R_8$, R', $R_x$, $R_s$, and n are the same as those in Chemical Formula 1, and unless otherwise defined, are the same as those in Chemical Formula 1.

$R_D$ is each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido (-O$^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$, and $R_s$, and o is an integer of 0 to 6.

At least one $R_x$ or at least one $R_s$ is present in the non-fluorescent compound represented by Chemical Formula 6.

It is preferable that the non-fluorescent compound represented by Chemical Formula 6 does not include water-soluble functional groups such as $NH_4^+$, $PO_4^-$, and $SO_3^-$ (including $SO_3H$, alkyl sulfonate, arylsulfonate, etc.). Accordingly, the non-fluorescent compound represented by Chemical Formula 6 is substantially lipophilic. Being substantially lipophilic means that the non-fluorescent compound exhibits a solubility of 30% or less, 20% or less, or 10% or less in an aqueous solvent, or is completely insoluble.

In another embodiment, the non-fluorescent compound may be represented by Chemical Formula 7 below. The non-fluorescent compound represented by Chemical Formula 7 has a structure in which Ar is represented by Chemical Formula 8 below in the non-fluorescent compound represented by Chemical Formula 1.

[Chemical Formula 7]

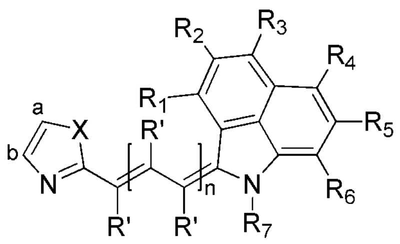

In Chemical Formula 7, the definitions of $R_1$ to $R_7$, R', $R_x$, $R_s$, n, X, and $R_{20}$ to $R_{22}$ are the same as those in Chemical Formula 1, and unless otherwise defined, are the same as those in Chemical Formula 1. a and b of Chemical Formula 7 are bonded to two adjacent substituents among $R_{31}$ to $R_{35}$ of Chemical Formula 8 below.

[Chemical Formula 8]

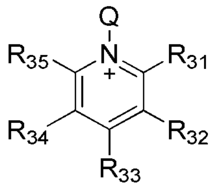

substituents among $R_{31}$ to $R_{35}$ of Chemical Formula 8 that are not bonded to a and b of Chemical Formula 7 are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido (-O$^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$.

Optionally, two adjacent substituents among $R_{31}$ to $R_{35}$ of Chemical Formula 8 that are not bonded to a and b of Chemical Formula 7 are bonded to each other to form an optionally substituted 5- to 7-membered ring,

Q is selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, optionally substituted C5-C50 aryl, optionally substituted C2-C50 heteroaryl, optionally substituted C5-C50 aralkyl, $R_x$ and $R_s$.

At least one $R_x$ or at least one $R_s$ is present in the non-fluorescent compound represented by Chemical Formula 7.

Specific examples of the compounds defined herein are as follows. However, the following exemplary compounds are provided for the purpose of assisting in the understanding of the compounds defined herein, and are not intended to limit the scope of the compounds defined herein. Based on the following exemplary compounds and the scope of the compounds defined herein, it can be expected that compounds recognized as being equivalent to the following exemplary compounds will all exhibit the effects of the compounds defined herein.

In addition, exemplary compounds other than those for which the synthesis steps are described in detail through

the preparation examples disclosed herein can also be synthesized by referring to the preparation examples disclosed herein or by referring to the definitions provided herein and using synthesis methods known to those skilled in the art.

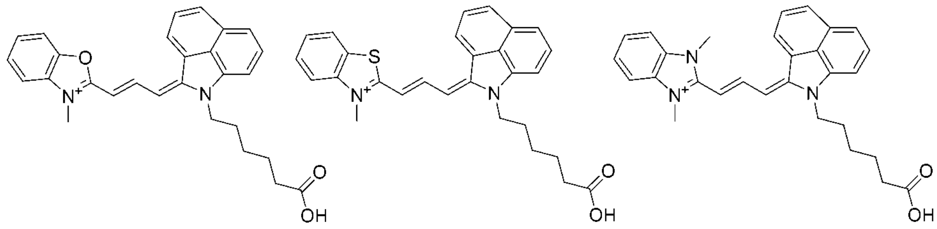

[Dye 1] [Dye 2] [Dye 3]

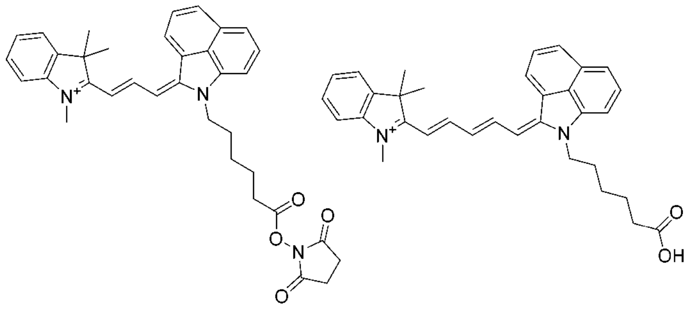

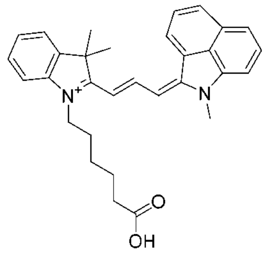

[Dye 4] [Dye 5] [Dye 6]

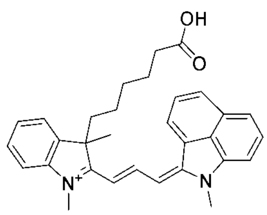

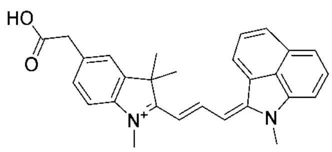

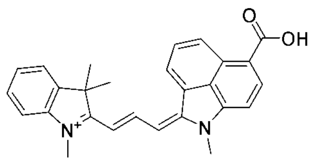

[Dye 7] [Dye 8] [Dye 9]

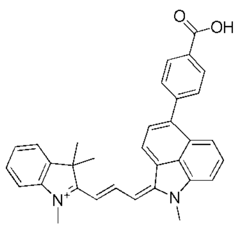

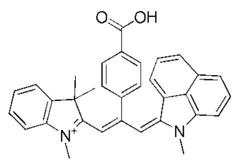

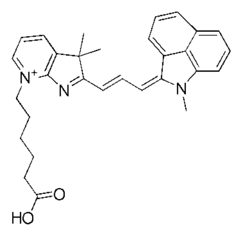

[Dye 10] [Dye 11] [Dye 12]

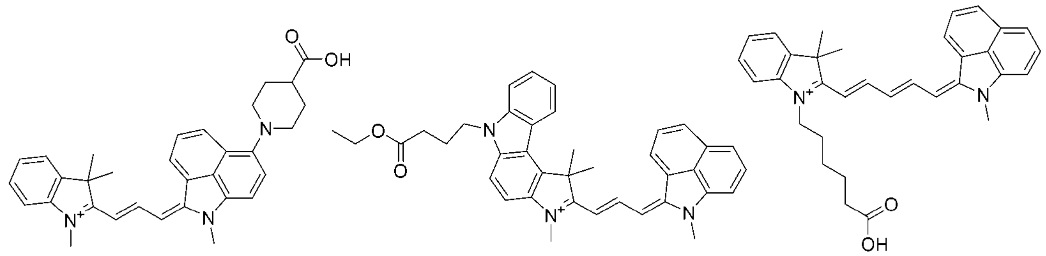

[Dye 13] [Dye 14] [Dye 15]

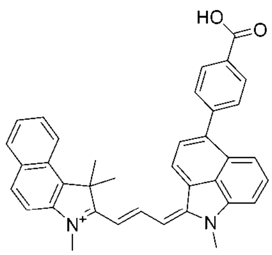

[Dye 16]

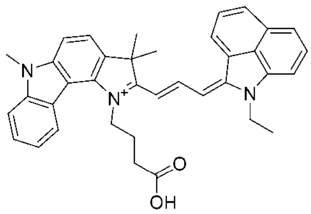

[Dye 17]

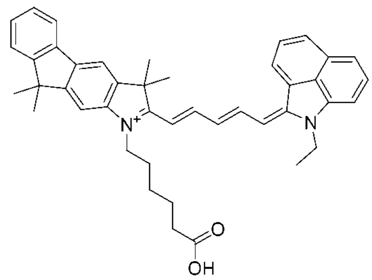

[Dye 18]

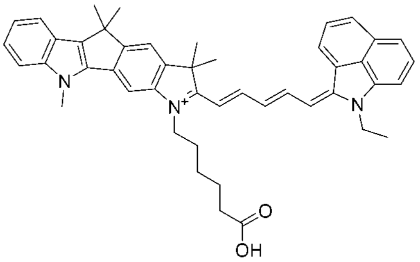

[Dye 19]

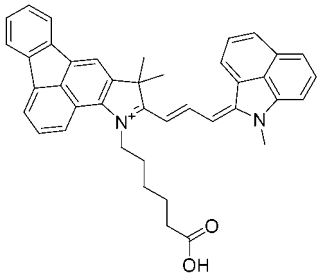

[Dye 20]

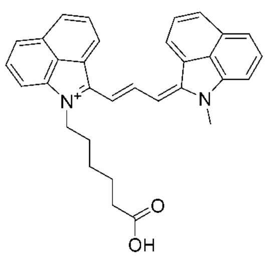

[Dye 21]

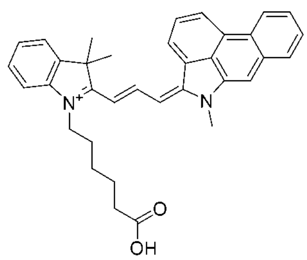

[Dye 22]

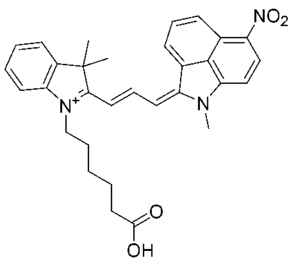

[Dye 23]

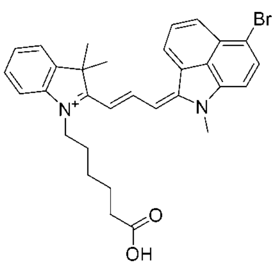

[Dye 24]

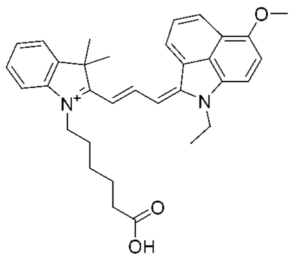

[Dye 25]

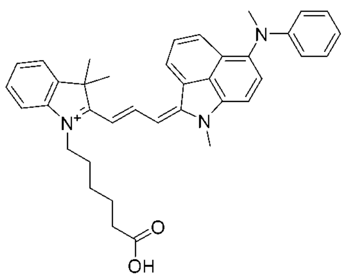

[Dye 26]

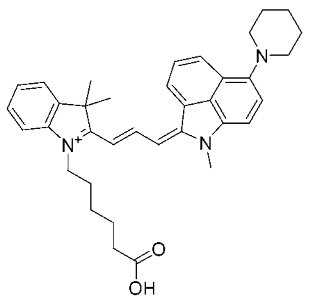

[Dye 27]

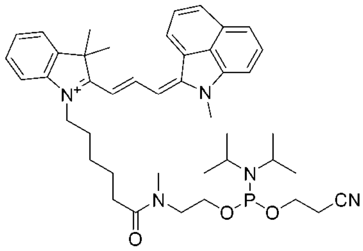

[Dye 28]

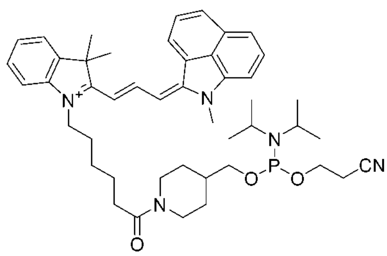

[Dye 29]

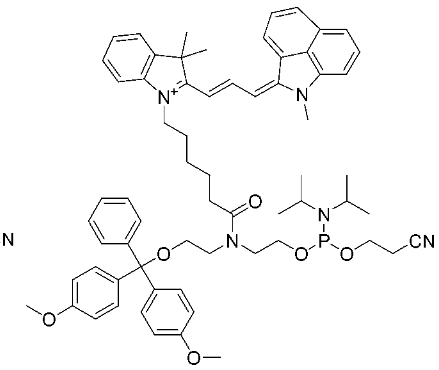

[Dye 30]

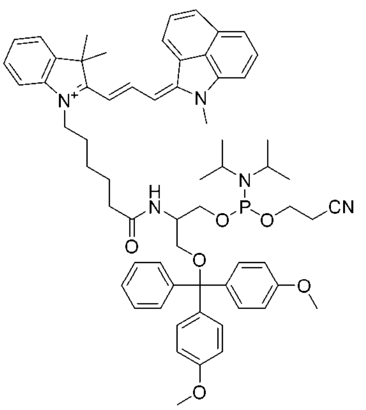

[Dye 31]

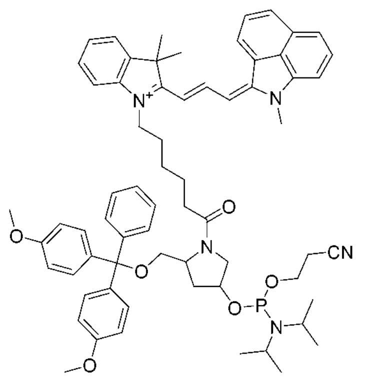

[Dye 32]

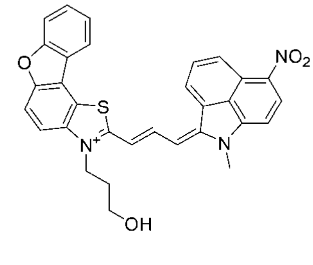

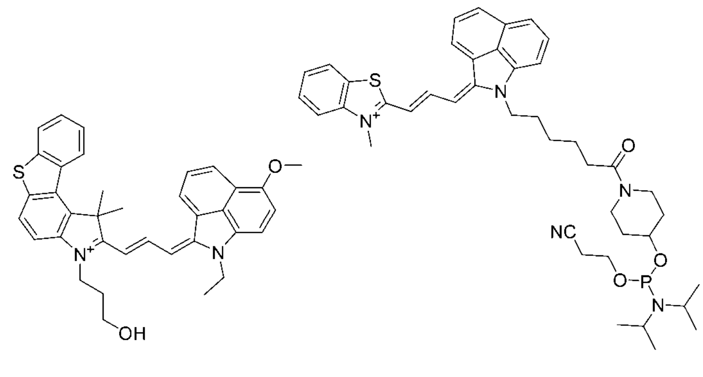

[Dye 33] [Dye 34] [Dye 35]

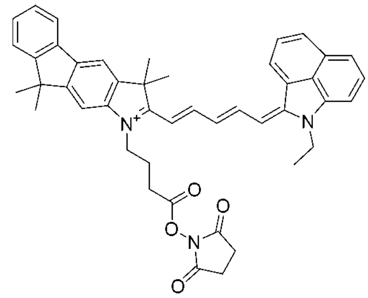

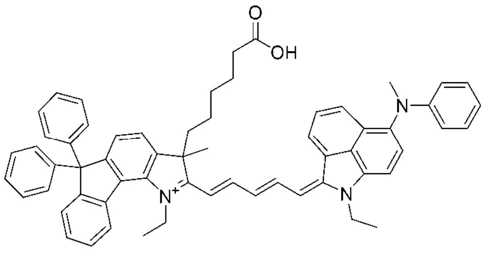

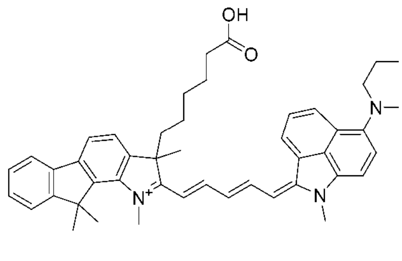

[Dye 36] [Dye 37] [Dye 38]

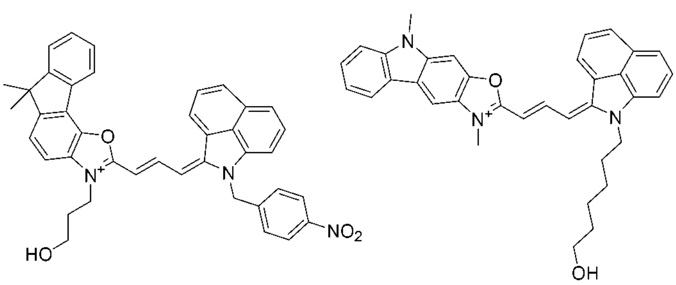

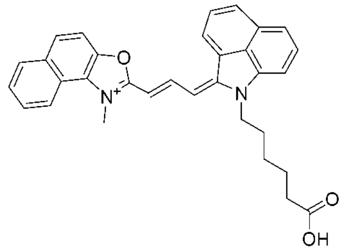

[Dye 39] [Dye 40] [Dye 41]

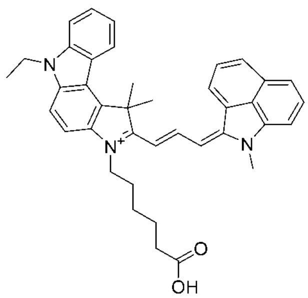

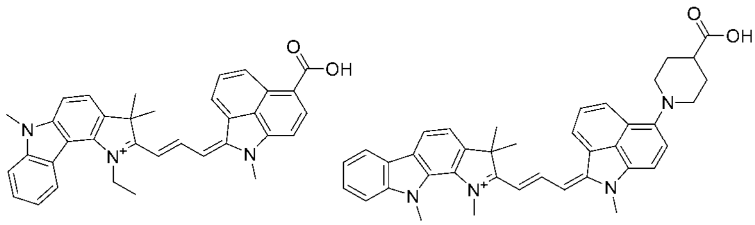

[Dye 42] [Dye 43] [Dye 44]

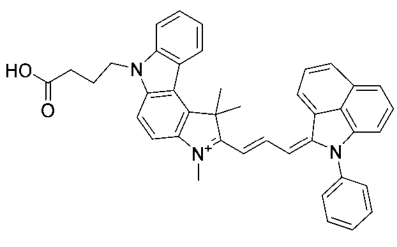

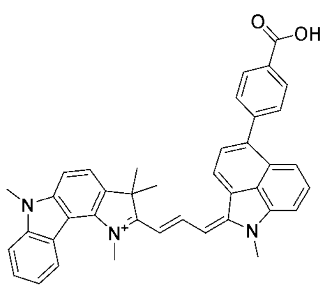

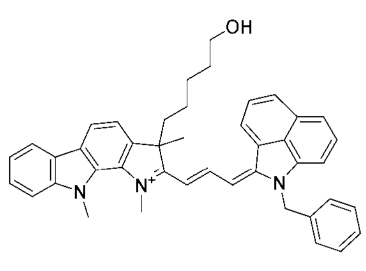

[Dye 45] [Dye 46] [Dye 47]

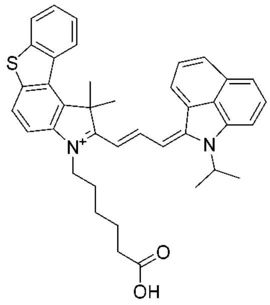

[Dye 48]

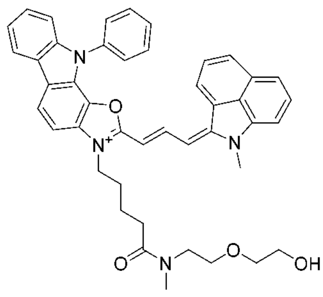

[Dye 49]

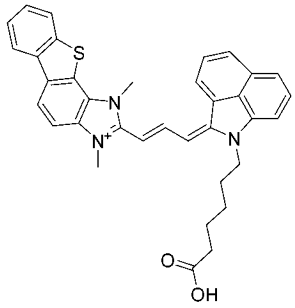

[Dye 50]

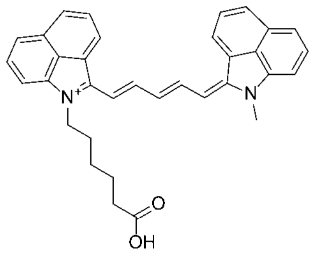

[Dye 51]

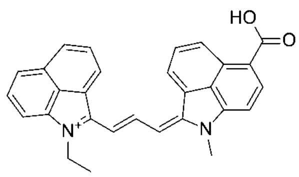

[Dye 52]

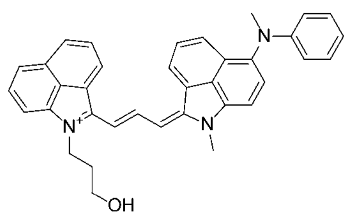

[Dye 53]

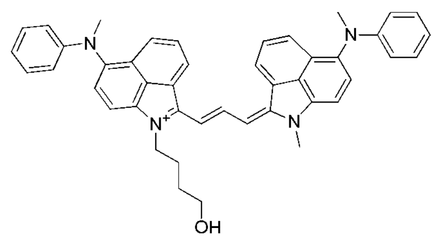

[Dye 54]

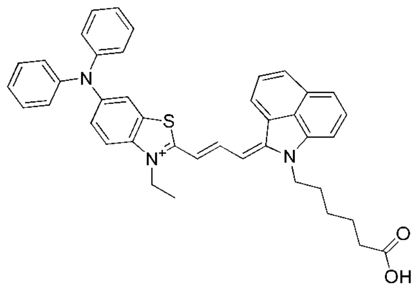

[Dye 55]

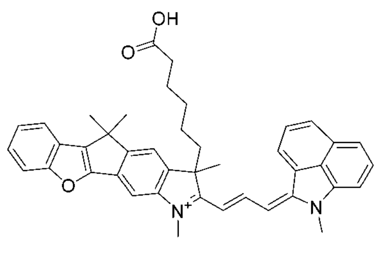

[Dye 56]

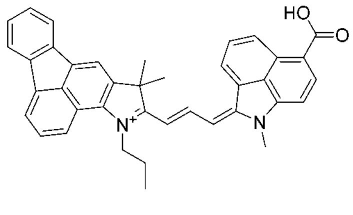

[Dye 57]

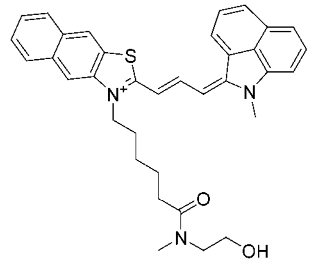

[Dye 58]

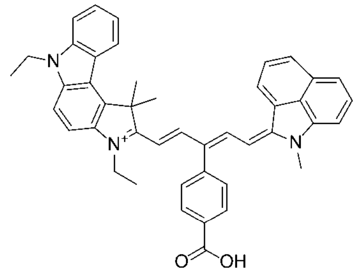

[Dye 59]

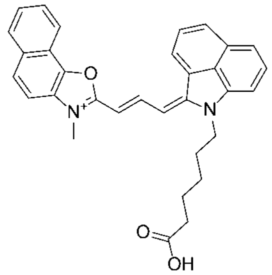

[Dye 60]

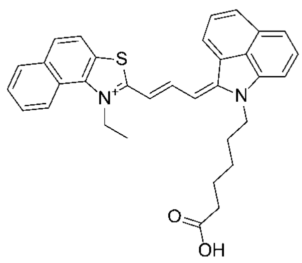

[Dye 61]

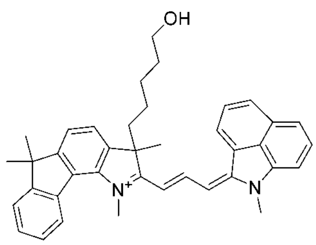

[Dye 62]

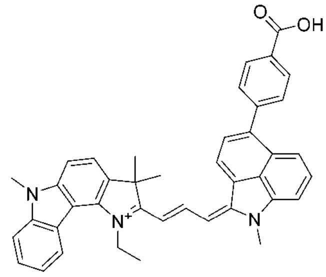

[Dye 63]

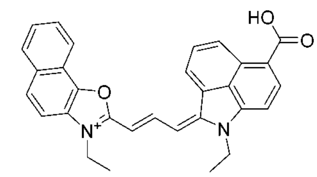

[Dye 64]

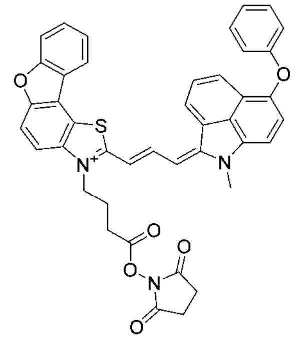

[Dye 65]

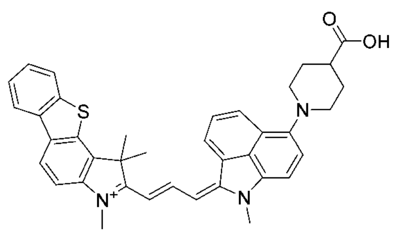

[Dye 66]

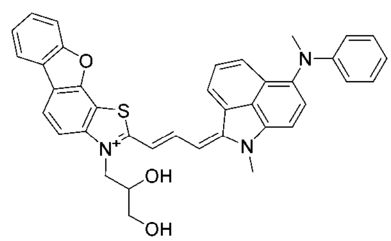

[Dye 67]

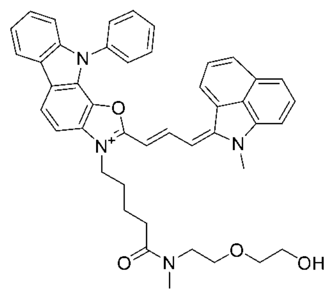

[Dye 68]

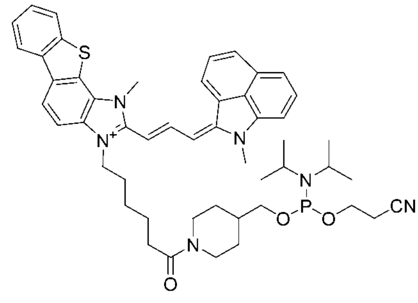

[Dye 69]

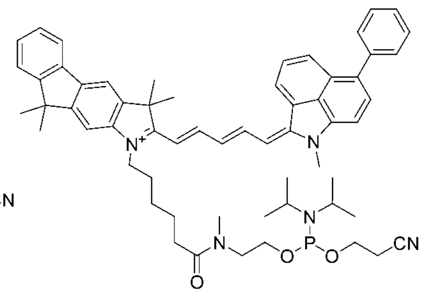

[Dye 70]

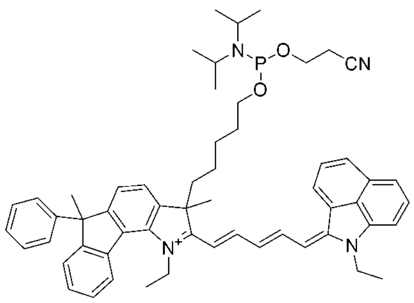

[Dye 71]

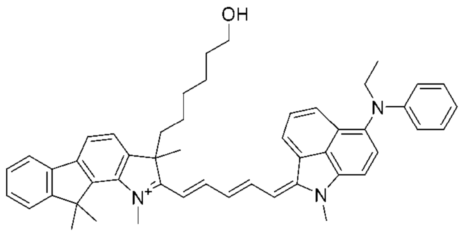

[Dye 72]

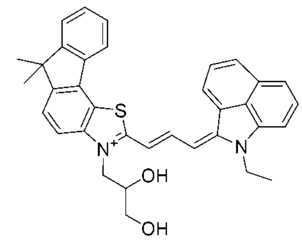

[Dye 73]

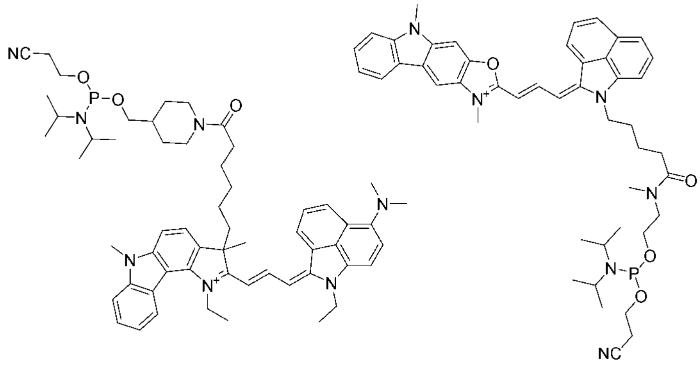

[Dye 74]

[Dye 75]

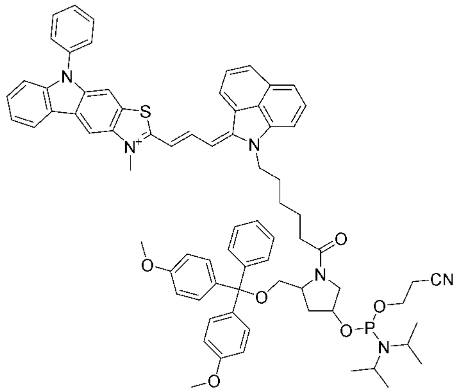

[Dye 76]

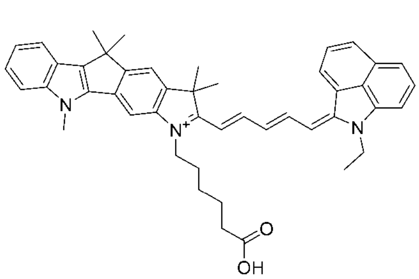

[Dye 77]

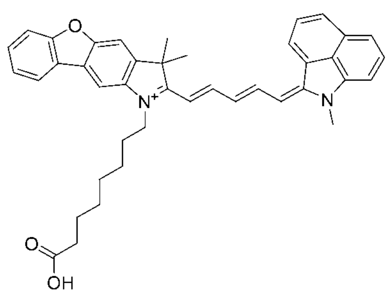

[Dye 78]

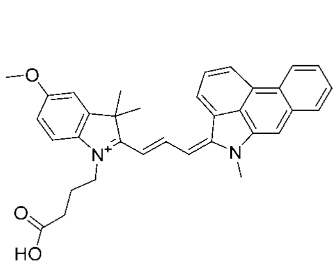

[Dye 79]

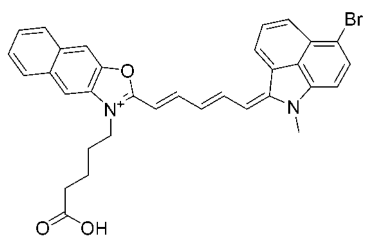

[Dye 80]

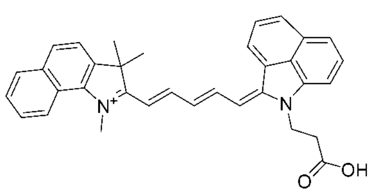

[Dye 81]

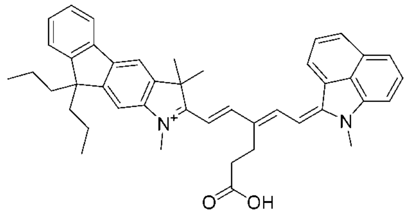

[Dye 82]

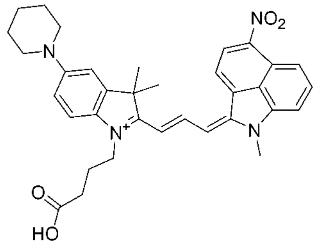

[Dye 83]

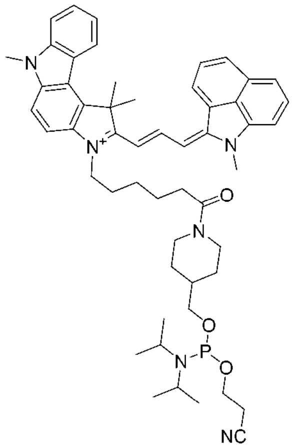

[Dye 84]

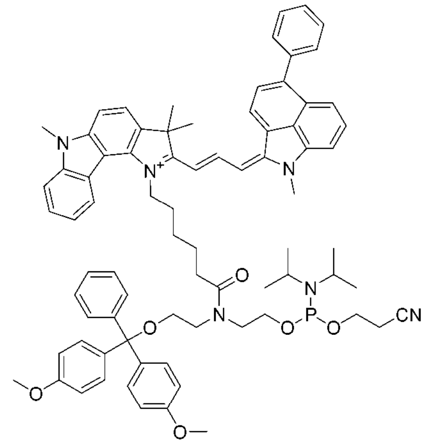

[Dye 85]

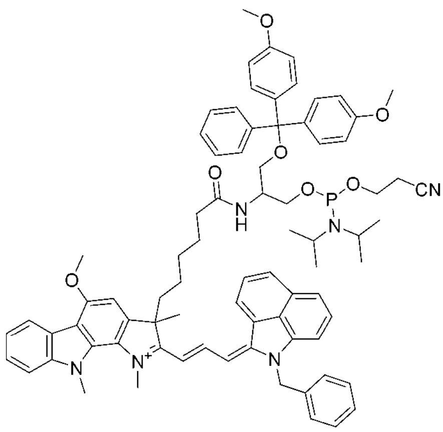

[Dye 86]

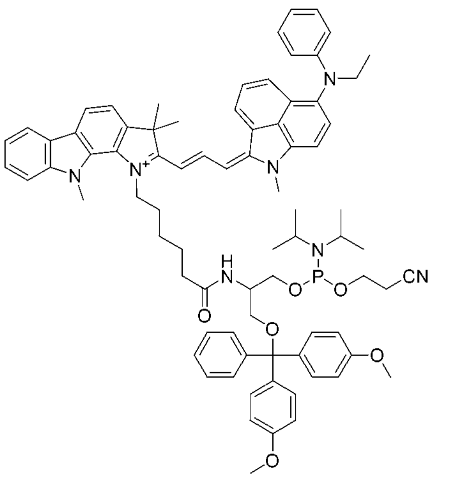

[Dye 87]

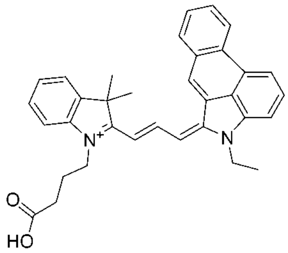

[Dye 88]

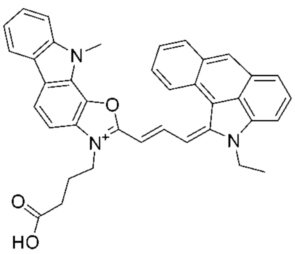

[Dye 89]

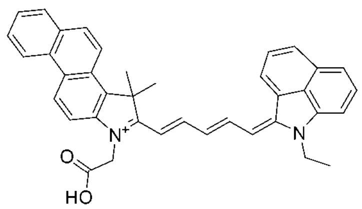

[Dye 90]

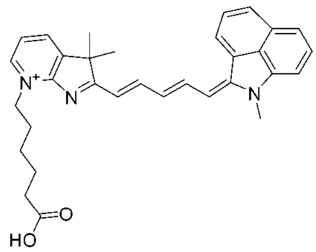

[Dye 91]

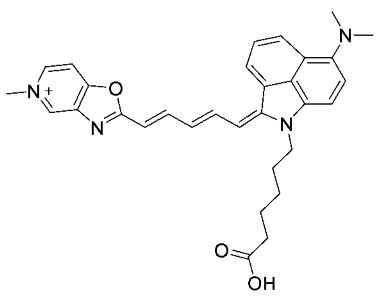

[Dye 92]

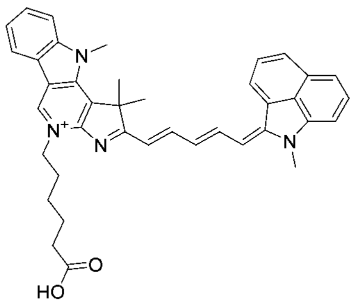

[Dye 93]

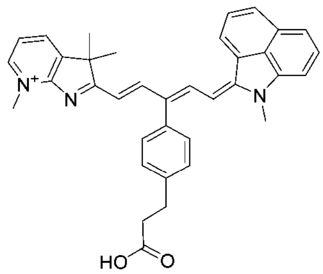

[Dye 94]

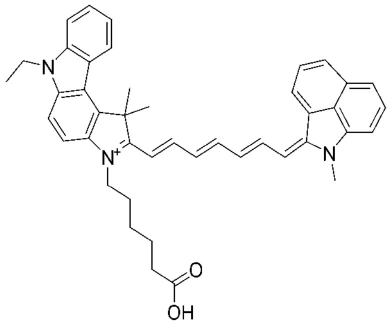

[Dye 95]

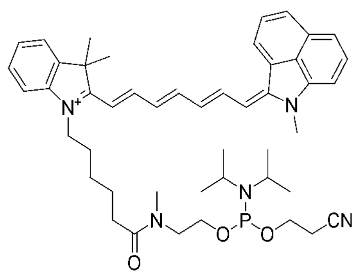

[Dye 96]

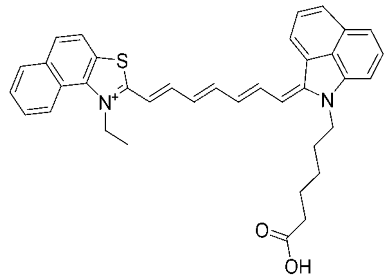

[Dye 97]

## Applications of the Non-Fluorescent Compound

The present invention relates to a non-fluorescent compound that is useful as a quencher for a fluorescent compound exhibiting luminescent properties at an excited energy level and to various uses thereof. According to another aspect of the present invention, various applications of a non-fluorescent compound capable of quenching luminescent properties of a fluorescent compound through static quenching and dynamic quenching are provided.

In one embodiment, a conjugate comprising the non-fluorescent compound defined herein as a quencher can be provided.

The conjugate can be used for biomolecule labeling. The conjugate can target biomolecules such as antibodies, lipids, proteins, peptides, carbohydrates, and nucleic acids (including nucleotides and nucleosides). Specific examples of lipids include fatty acids, phospholipids, and lipopolysaccharides, and specific examples of carbohydrates include monosaccharides, disaccharides, and polysaccharides (e.g., dextran).

The conjugate may be a nucleotide conjugate, probe, primer, and/or peptide. The non-fluorescent compound may be bound to any terminus (5'-terminus or 3'-terminus) or any internal position of the conjugate.

When the conjugate is a nucleotide conjugate, the conjugate may include an oligonucleotide.

The oligonucleotide refers to a polymer of 1 to several hundred nucleotides. Phosphodiester bonds in the oligonucleotide may be modified or substituted with methylphosphonate, phosphorothioate (PTO), boranophosphate, phosphoryl guanidine, guanidinopropyl phosphoramidate, thiazole, guanidinium, and the like. The oligonucleotide may also include one or more modified nucleotides. For example, modified nucleotides may include PNA, BNA (e.g., LNA, ENA), HNA, ANA, CeNA, and GNA having sugar modifications, or nucleotides in which any substituent (e.g., MOE, alkyl, halogen) is introduced into the sugar.

Further, modified nucleotides and nucleosides may include modified nucleobases derived from adenine (A), guanine (G), thymine (T), cytosine (C), and uracil (U), for example, inosine, xanthine, hypoxanthine, nebularine, isoguanosine, tubercidin, 2-aminoadenine, 2-haloadenine, 2-alkyladenine, 2-methylaminoadenine, 6-methyladenine, 8-haloadenine, 8-aminoadenine, 8-thioadenine, diazaadenine, 8-hydroxyadenine and other substituted adenines, 5-halouracil, 4-thiouracil, 5-trifluoromethyluracil, pseudouracil, 2-thiouracil, 5-halouracil and other substituted uracils, 5-halocytosine, 5-trifluorocytosine, 6-azacytosine and other substituted cytosines, 2-thiothymine, 6-azathymine and other substituted thymines, 8-haloguanine, 8-aminoguanine, 8-thioguanine, 8-thioalkylguanine, 8-hydroxyguanine, 8-azaguanine, 7-deazaguanine, 7-methylguanine and other substituted guanines, pyrazolo[3,4-d]pyrimidine, 2,6-diaminopurine, phenoxazine, 2-aminopurine, 3-nitropyrrole, 5-hydroxybutynyluridine, Super A®, Super G®, Super T®, and Super D™.

Examples of modified nucleobases may also be referred to in the following reference documents (U.S. Pat. Nos. 11,155,713; Current Topics in Medicinal Chemistry, Volume 7, Number 7, Wojciechowski, Filip, E. Hudson, Robert H, 2007, pp. 667-679).

As used herein, nucleobase may include modified nucleobase.

An oligonucleotide labeled with one non-fluorescent compound or fluorescent compound may be referred to as a single-labeled oligonucleotide, for example, an oligonucleotide labeled with the non-fluorescent compound at the 3'-terminus.

The conjugate may further comprise a quencher containing the non-fluorescent compound defined herein and a fluorophore. The fluorophore may be any known fluorescent compound suitable for biomolecule labeling. The fluorescent compound may be bound to any terminus (5'-terminus or 3'-terminus) or any internal position of the conjugate.

An oligonucleotide labeled with two non-fluorescent compounds or fluorescent compounds may be referred to as a double-labeled oligonucleotide, for example, an oligonucleotide labeled with a fluorescent compound at the 5'-terminus and with a non-fluorescent compound at the 3'-terminus.

The fluorescent compound may be at least one selected from coumarin, cyanine, BODIPY, fluorescein, rhodamine, pyrene, carbopyronine, oxazine, xanthene, thioxanthene, acridine, and derivatives thereof.

More specific examples of the fluorescent compound include 6-FAM™, TET™, JOE™, VIC®, HEX™, NED™, PET®, ROX™, TAMRA™, TET™, Texas Red®, SUN, MAX, ABY, JUN, LIZ, TAZ, CAL Fluor® Gold 540, CAL Fluor® Orange 560, CAL Fluor® Red 590, CAL Fluor® Red 610, CAL Fluor® Red 635, Cy® (cyanine) 3, Cy® 3.5, Cy® 5, Cy® 5.5, Cy® 7, Cy® 7.5, Quasar® 570, Quasar® 670, Quasar® 705, Rhodamine Green™, Rhodamine Red™, LightCycler® Cyan 500, LightCycler® Red 610, LightCycler® Red 640, LightCycler® Red 670, LightCycler® Red 705, Oregon Green® 488, Oregon Green® 500, Oregon Green® 514, the Alexa Fluor® dyes 350, 405, 488, 532, 546, 555, 568, 594, 610, 647, 680 and Alexa Fluor® 750, the BODIPY® dyes, Epoch Blue, AMCA, Marina Blue®, Pacific Blue™, Pacific Green™, Pacific Orange™, Yakima Yellow™, the ATTO dyes 390, 425, 465, 488, 495, 514, 520, 532, Rho6G, 542, 550, 565, Rho3B, Rho11, Rho12, Thio12, Rho101, 590, 594, Rho13, 610, 620, Rho14, 633, 643, 647, 647N, 655, Oxa12, 665, 680, 700, 725, ATTO 740, Texas Red, Oyster 645, SFC™-V, SFC™-N, SFC™574, SFC™647, SFC™-C610, SFC™620, SFC™670, SFC™705, Chamel™560, Chamel™610, Chamel™670, and Chamel™705.

The conjugate may include a first quencher comprising the non-fluorescent compound defined herein, a second quencher selected from azo, coumarin, cyanine, BODIPY, fluorescein, rhodamine, pyrene, carbopyronine, benzo[c,d]indole, oxazine, xanthene, thioxanthene, acridine, and derivatives thereof, and a fluorophore.

The first quencher, second quencher, and fluorophore may be bound to any terminus (5'-terminus or 3'-terminus) or any internal position of the conjugate.

An oligonucleotide labeled with three non-fluorescent compounds or fluorescent compounds may be referred to as a triple-labeled oligonucleotide, for example, an oligonucleotide in which the fluorescent compound is labeled at the 5'-terminus, the first quencher is labeled at the 3'-terminus, and the second quencher is inserted between oligonucleotides.

The second quencher may be at least one selected from azo, coumarin, cyanine, BODIPY, fluorescein, rhodamine, pyrene, carbopyronine, benzo[c,d]indole, oxazine, xanthene, thioxanthene, acridine, and derivatives thereof.

More specific examples of the second quencher include dabcyl, Eclipse™, Black Hole Quencher™ BHQ0 (BHQnova), Black Hole Quencher™ BHQ1, Black Hole Quencher™ BHQ2, Black Hole Quencher™ BHQ3, BlackBerry™ Quencher 650 (BBQ650™), Iowa Black™ FQ (IABkFQ), SFC™ Q1, SFC™ Q2, Iowa Black™ RQ-n1, Iowa Black™ RQ-n2, Iowa Black™ RQSp, TAMRA, Deep Dark Quencher I (DDQ I), Deep Dark Quencher II (DDQ II), QXL™520, QXL™570, QXL™610, QXL™670, IRQXL™, IRDye™, QC-1, QSY™, QSY™2, BMN-Q460, BMN-Q535, BMN-Q1, BMN-Q2, BMN-Q590, BMN-Q620, and BMN-Q651.

The conjugate may further include a minor groove binder (MGB) to improve binding affinity to nucleic acids. If a conjugate further comprising an MGB is used, PCR amplification efficiency can be enhanced.

The conjugate defined herein can be variously used in chemical and biological fields. In particular, it may be useful for real-time polymerase chain reaction or microassay, but is not limited thereto.

In another embodiment, a composition for detecting nucleic acids comprising the conjugate defined herein can be provided.

The nucleic acid detection composition according to above embodiment of the present invention may further include an enzyme for reaction with a target nucleic acid, a solvent (such as buffer), and other reagents, along with the conjugate defined herein.

Examples of the solvent include buffers selected from phosphate buffer, carbonate buffer, and Tris buffer; organic solvents selected from dimethyl sulfoxide, dimethylformamide, dichloromethane, methanol, ethanol, and acetonitrile; or water. The solubility of the quencher can be adjusted by introducing various functional groups into the quencher depending on the type of solvent.

In another embodiment, a support for nucleic acid detection comprising a quencher comprising the non-fluorescent compound defined herein, a support, and a linker connecting the quencher and the support can be provided. A biomolecule (nucleic acid) in a sample can be immobilized on the support through interaction with the quencher

immobilized on the support.

The support may be made of at least one selected from glass (e.g., CPG), cellulose, nylon, acrylamide gel, dextran, polystyrene, alginate, collagen, peptide, fibrin, hyaluronic acid, agarose, polyhydroxyethyl methacrylate, polyvinyl alcohol, polyethylene glycol, polyethylene oxide, polyethylene glycol diacrylate, gelatin, matrigel, polylactic acid, carboxymethyl cellulose, dextran, chitosan, latex, and Sepharose, and may be in the form of beads or a membrane.

The linker is a part that connects the quencher and the support, and any material capable of connecting the quencher and the support can be used as the linker intended herein.

For example, the linker may be selected from substituted or unsubstituted C1-C30 alkyl, substituted or unsubstituted C3-C30 cycloalkyl, substituted or unsubstituted C2-C30 heteroalkyl including at least one hetero atom, substituted or unsubstituted C2-C30 heterocycloalkyl including at least one hetero atom, substituted or unsubstituted C2-C30 alkenyl, substituted or unsubstituted C6-C30 aryl, substituted or unsubstituted C3-C30 heteroaryl, amide (-CONH-), ester (-COO-), ketone (-CO-), nucleoside, and any combination thereof. The linker connects the quencher and the support but does not substantially affect other reactions or the fluorescence and quenching actions of the quencher or the fluorophore.

Examples of connection structures of the support and the quencher via the linker are shown below. In the following examples, "Quencher" refers to a quencher (or non-fluorescent compound), and "dye" refers to a fluorophore.

[Conneting Structure 1]

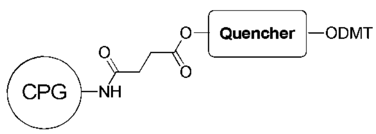

[Conneting Structure 2]

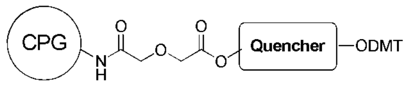

[Conneting Structure 3]

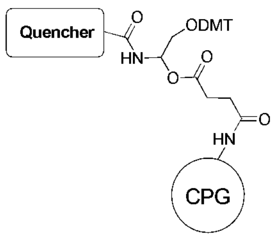

[Conneting Structure 4]

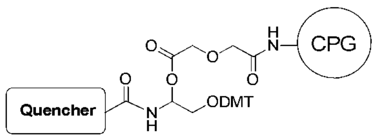

[Conneting Structure 5]

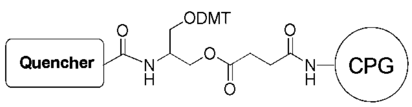

[Conneting Structure 6]

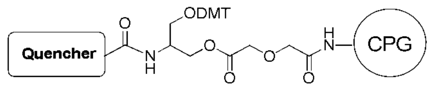

[Conneting Structure 7]

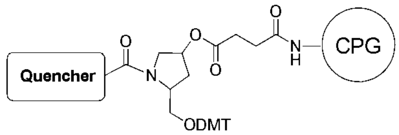

[Conneting Structure 8]

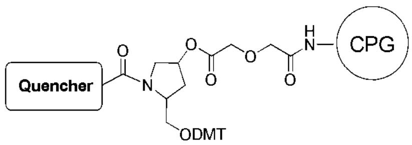

[Conneting Structure 9]

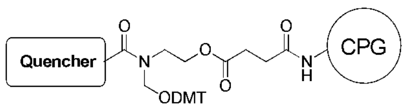

[Conneting Structure 10]

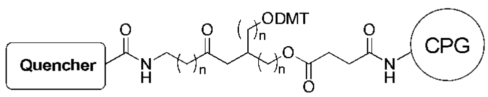

[Conneting Structure 11]

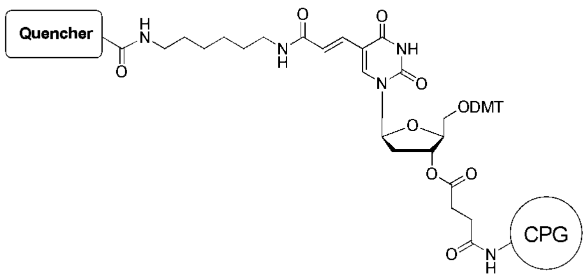

[Conneting Structure 12]

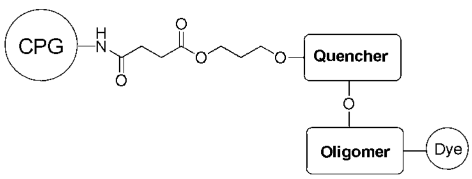

[Conneting Structure 13]

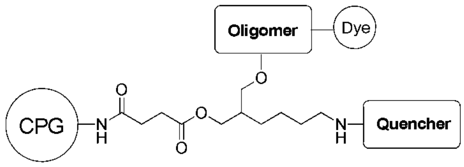

## Method for Detecting Nucleic Acid

According to another aspect of the present invention, a method for labeling a target biomolecule, which is nucleic acid, using a conjugate comprising the non-fluorescent compound and a fluorescent compound defined herein is provided.

In addition, depending on the type of the target biomolecule, a method for labeling a biomolecule through target-specific interaction may be implemented by introducing an appropriate reactive group into the non-fluorescent compound and/or the fluorescent compound. Further, a method for identifying a biomolecule labeled with the conjugate defined herein by electrophoresis may also be implemented.

In one embodiment, a method for detecting nucleic acid may comprise:

(a) preparing a reaction mixture comprising a target nucleic acid, reagents necessary for amplifying the target nucleic acid, and the conjugate defined herein;
(b) amplifying the target nucleic acid in the reaction mixture; and
(c) measuring fluorescence intensity of the reaction mixture.

The conjugate used in the embodiment comprises at least a quencher comprising the non-fluorescent compound defined herein and a fluorophore.

The step (b) may comprise:

(b-1) extending the nucleotide conjugate hybridized to the target nucleic acid by a polymerase;
(b-2) separating a quencher and a fluorophore of the nucleotide conjugate from the target nucleic acid by exonuclease activity of the polymerase; and
(b-3) allowing the fluorophore separated from the quencher to emit fluorescence.

In the step (b), the target nucleic acid may be amplified by a method selected from strand displacement amplification (SDA), polymerase chain reaction (PCR), reverse transcription polymerase chain reaction (RT-PCR), real-time polymerase chain reaction, allele-specific polymerase chain reaction, ligase chain reaction (LCR), rolling circle amplification (RCA), isothermal multiple displacement amplification (IMDA), recombinase polymerase amplification (RPA), self-sustained sequence replication (3SR), single primer isothermal amplification (SPIA), multiple displacement amplification (MDA), whole genome amplification (WGA), cross-priming amplification (CPA), signal mediated amplification of RNA technology (SMART), transcription mediated amplification (TMA), nucleic acid sequence based amplification (NASBA), loop-mediated isothermal amplification (LAMP), and helicase dependent amplification (HDA).

In addition, the method may further comprise (d) determining the amount of amplification of the target nucleic acid based on the fluorescence intensity measured in the step (c).

Hereinafter, specific embodiments of the present invention are provided. However, the embodiments described below are merely illustrative or explanatory of the present invention, and the present invention is not limited thereto. Furthermore, reporters defined in the claims and the detailed description of the specification, which are not synthesized in the following preparation examples, may be synthesized by reference to the preparation examples provided herein.

## Synthesis Example. Synthesis of non-fluorescent compound

### Synthesis of Dye 1

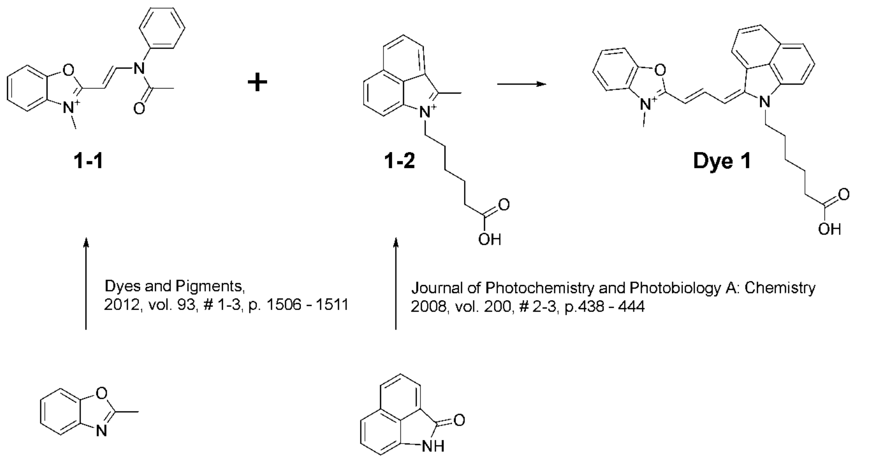


1-1 1-2 Dye 1

Synthesis of Intermediate 1-1

Intermediate 1-1 was synthesized with reference to Dyes and Pigments, 2012, vol. 93, no. 1-3, p. 1506-1511.

Synthesis of Intermediate 1-2

Intermediate 1-2 was synthesized with reference to Journal of Photochemistry and Photobiology A: Chemistry, 2008, vol. 200, no. 2-3, p. 438-444.

Synthesis of Dye 1

In a 100 mL single-necked reactor, intermediate 1-1 (2.0 g, 4.8 mmol), intermediate 1-2 (1.95 g, 4.8 mmol), and pyridine (20 mL) were added and stirred at 50°C for 1 hour. After concentration, Dye 1 was synthesized by column purification (1.2 g).

The $^1$H-NMR of the obtained Compound 1 is as follows:
$^1$H-NMR (400 MHz, DMSO-$d_6$) δ 8.72 (t, 1H, J = 12.8 Hz), 8.52 (d, 1H, J = 8.0 Hz), 8.40 (d, 1H, J = 8.22 (d, 1H, J = 8.4 Hz), 8.03 (t, 1H, J = 7.2 Hz), 7.79 (d, 1H, J = 8.4 Hz), 7.68 (t, 1H, J = 8.4 Hz), 7.57-7.55 (m, 2H), 7.45-7.29 (m, 2H), 7.00 (d, 1H, J = 13.6 Hz), 6.83 (d, 1H, J = 12.8 Hz), 4.50 (t, 2H, J = 7.6 Hz), 3.82 (s, 3H), 2.54 (m, 2H), 2.03-1.77 (m, 6H)

**Synthesis of Dye 2**

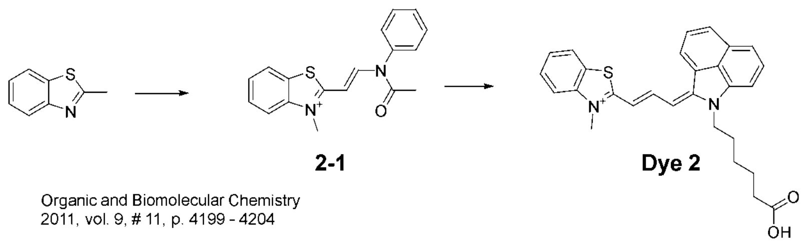


2-1 Dye 2

Synthesis of Intermediate 2-1

Intermediate 2-1 was synthesized with reference to Organic and Biomolecular Chemistry, 2011, vol. 9, no. 11, p. 4199-4204.

Synthesis of Dye 2

In a 100 mL single-necked reactor, intermediate 2-1 (3.0 g, 6.9 mmol), intermediate 1-2 (2.81 g, 6.9 mmol), and pyridine (30 mL) were added and stirred at 50°C for 1 hour. After concentration, Dye 2 was synthesized by column purification (2.2 g).

The $^1$H-NMR of the obtained Dye 2 is as follows:
$^1$H-NMR (400 MHz, DMSO-$d_6$) δ 8.75 (t, 1H, J = 12.8 Hz), 8.50 (d, 1H, J = 8.0 Hz), 8.39 (d, 1H, J = 8.21 (d, 1H, J = 8.4 Hz), 8.00 (t, 1H, J = 7.2 Hz), 7.76 (d, 1H, J = 8.4 Hz), 7.68 (t, 1H, J = 8.4 Hz), 7.59-7.51 (m, 2H), 7.49-7.25 (m, 2H), 7.01 (d, 1H, J = 13.6 Hz), 6.88 (d, 1H, J = 12.8 Hz), 4.51 (t, 2H, J = 7.6 Hz), 3.80 (s, 3H), 2.53 (m, 2H), 2.10-1.76 (m, 6H)

**Synthesis of Dye3**

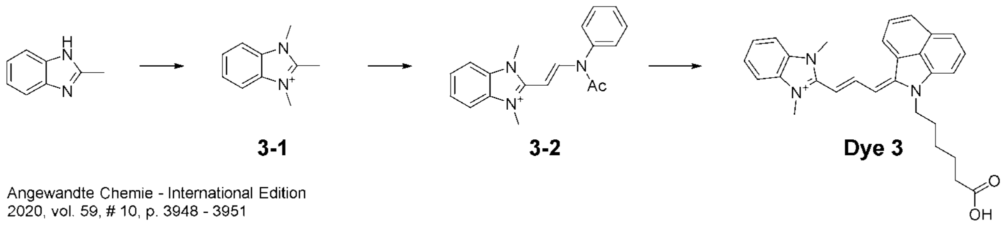

3-1

3-2

Dye 3

Angewandte Chemie - International Edition
2020, vol. 59, # 10, p. 3948 - 3951

Synthesis of Intermediate 3-1

Intermediate 3-1 was synthesized with reference to Angewandte Chemie - International Edition, 2020, vol. 59, no. 10, p. 3948-3951.

Synthesis of Intermediate 3-2

In a 250 mL single-necked reactor, intermediate 3-1 (5.0 g, 17 mmol), N,N-diphenylformamidine (4.1 g, 21 mmol), and acetic anhydride (40 mL) were added and stirred at 110°C for 1 hour. After cooling, ethyl acetate (100 mL) was added and vigorously stirred. The resulting solid was filtered to synthesize intermediate 3-2.

Synthesis of Dye 3

In a 100 mL single-necked reactor, intermediate 3-2 (2.0 g, 4.6 mmol), intermediate 1-2 (1.89 g, 4.6 mmol), and pyridine (20 mL) were added and stirred at 50°C for 1 hour. After concentration, Dye 3 was synthesized by column purification (0.8 g).

The $^1$H-NMR of the obtained Dye 3 is as follows:
$^1$H-NMR (400 MHz, DMSO-$d_6$) δ 8.70 (t, 1H, J = 12.8 Hz), 8.48 (d, 1H, J = 8.0 Hz), 8.37 (d, 1H, J = 8.21 (d, 1H, J = 8.4 Hz), 8.00 (t, 1H, J = 7.2 Hz), 7.74 (d, 1H, J = 8.4 Hz), 7.66 (t, 1H, J = 8.4 Hz), 7.55-7.50 (m, 2H), 7.49-7.25 (m, 2H), 7.00 (d, 1H, J = 13.6 Hz), 6.86 (d, 1H, J = 12.8 Hz), 4.50 (t, 2H, J = 7.6 Hz), 3.88 (s, 6H), 2.51 (m, 2H), 2.10-1.75 (m, 6H)

**Synthesis of Dye 4**

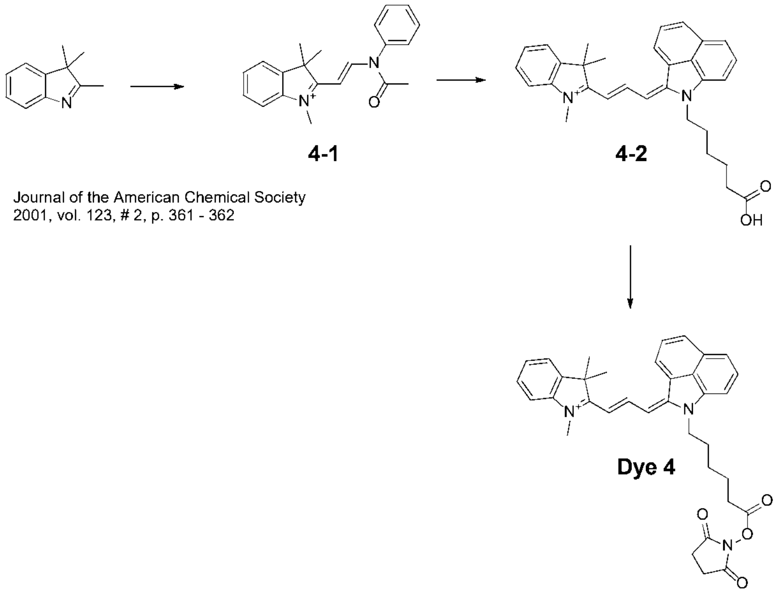


4-1

4-2

Dye 4

Synthesis of Intermediate 4-1

Intermediate 4-1 was synthesized with reference to Journal of the American Chemical Society, 2001, vol. 123, no. 2, p. 361-362.

Synthesis of Intermediate 4-2

In a 100 mL single-necked reactor, intermediate 4-1 (5.0 g, 17 mmol), intermediate 1-2 (4.1 g, 21 mmol), and pyridine (50 mL) were added and stirred at 50°C for 1 hour. After concentration, intermediate 4-2 was synthesized by column purification.

Synthesis of Dye 4

In a 100 mL single-necked reactor, intermediate 4-2 (2.7 g, 4.2 mmol), HSTU (O-(N-succinimidyl)-N,N,N',N'-tetramethyluronium hexafluorophosphate) (1.81 g, 5.0 mmol), triethylamine (1.76 mL, 12.6 mmol), and dimethylformamide (27 mL) were added and stirred at room temperature for 30 minutes. After concentration, Dye 4 was synthesized by column purification (2.1 g).

The $^1$H-NMR of the obtained Dye 4 is as follows:
$^1$H-NMR (400 MHz, DMSO-$d_6$) $\delta$ 8.77 (t, 1H, J = 12.8 Hz), 8.54 (d, 1H, J = 8.0 Hz), 8.38 (d, 1H, J = 8.29 Hz), 8.22 (d, 1H, J = 8.4 Hz), 8.03 (t, 1H, J = 7.2 Hz), 7.71 (d, 1H, J = 8.4 Hz), 7.65 (t, 1H, J = 8.4 Hz), 7.61-7.52 (m, 2H), 7.47-7.15 (m, 2H), 7.00 (d, 1H, J = 13.6 Hz), 6.87 (d, 1H, J = 12.8 Hz), 4.50 (t, 2H, J = 7.6 Hz), 3.90 (s, 4H), 3.82 (s, 3H), 2.53 (m, 2H), 2.12 (s, 6H), 2.10-1.76 (m, 6H)

**Synthesis of Dye 5**

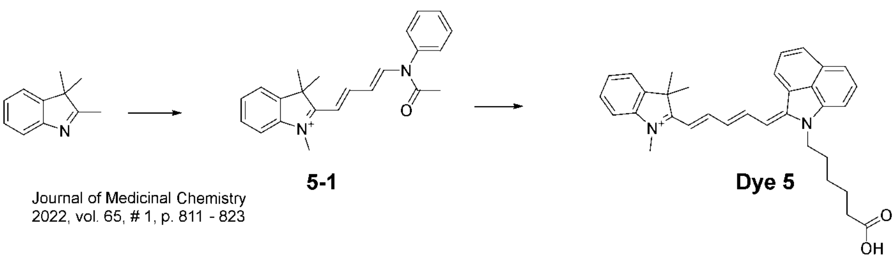


5-1

Dye 5

Synthesis of Intermediate 5-1

Intermediate 5-1 was synthesized with reference to Journal of Medicinal Chemistry, 2022, vol. 65, # 1, p. 811 - 823.

Synthesis of Dye 5

In a 100 mL single-necked reactor, intermediate 5-1 (3.0 g, 6.4 mmol), intermediate 1-2 (2.6 g, 6.4 mmol), and pyridine (30 mL) were added and stirred at 50°C for 1 hour. After concentration, Dye 5 was synthesized by column purification (1.7 g).

The $^1$H-NMR of the obtained Dye 5 is as follows:

$^1$H-NMR (400 MHz, DMSO-$d_6$) $\delta$ 8.56 (d, 1H, J = 8.0 Hz), 8.49 (t, 2H, J = 15.6 Hz), 8.33 (d, 1H, J = 8.30Hz), 8.22 (d, 1H, J = 8.4 Hz), 8.00 (t, 1H, J = 7.2 Hz), 7.75 (d, 1H, J = 8.4 Hz), 7.63 (t, 1H, J = 8.4 Hz), 7.60-7.50 (m, 2H), 7.45-7.17 (m, 2H), 6.90 (t, 1H, J = 13.6 Hz), 6.36 (d, 2H, J = 13.6 Hz), 4.52 (t, 2H, J = 7.6 Hz), 3.80 (s, 3H), 2.53 (m, 2H), 2.12 (s, 6H), 2.10-1.76 (m, 6H)

**Synthesis of Dye 6**

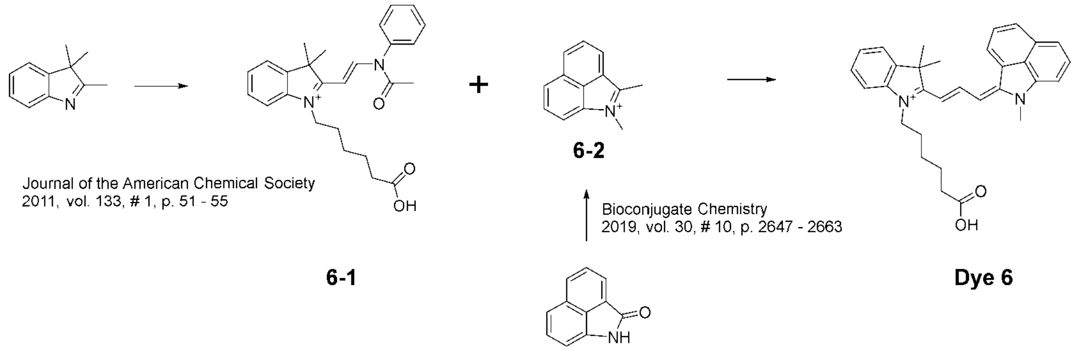


6-2

6-1

Dye 6

Synthesis of Intermediate 6-1

Intermediate 6-1 was synthesized with reference to Journal of the American Chemical Society, 2011, vol. 133, # 1, p. 51 - 55.

Synthesis of Intermediate 6-2

Intermediate 6-2 was synthesized with reference to Bioconjugate Chemistry, 2019, vol. 30, # 10, p. 2647 - 2663.

Synthesis of Dye 6

In a 100 mL single-necked reactor, intermediate 6-1 (2.0 g, 3.7 mmol), intermediate 6-2 (1.1 g, 3.7 mmol), and pyridine (30 mL) were added and stirred at 50°C for 1 hour. After concentration, Dye 6 was synthesized by column purification (1.1 g).

$^1$H-NMR of the obtained Dye 6 is as follows:

$^1$H-NMR (400 MHz, DMSO-$d_6$) $\delta$ 8.76 (t, 1H, J = 12.8 Hz), 8.51 (d, 1H, J = 8.0 Hz), 8.37 (d, 1H, J = 8.0 Hz), 8.22 (d, 1H, J = 8.4 Hz), 8.04 (t, 1H, J = 7.2 Hz), 7.70 (d, 1H, J = 8.4 Hz), 7.62 (t, 1H, J = 8.4 Hz), 7.59-7.50 (m, 2H), 7.45-7.13 (m, 2H), 6.98 (d, 1H, J = 13.6 Hz), 6.86 (d, 1H, J = 12.8 Hz), 4.26 (t, 2H, J = 7.6 Hz), 3.85 (s, 3H), 2.51 (m, 2H), 2.13 (s, 6H), 2.10-1.75 (m, 6H)

**Synthesis of Dye 7**

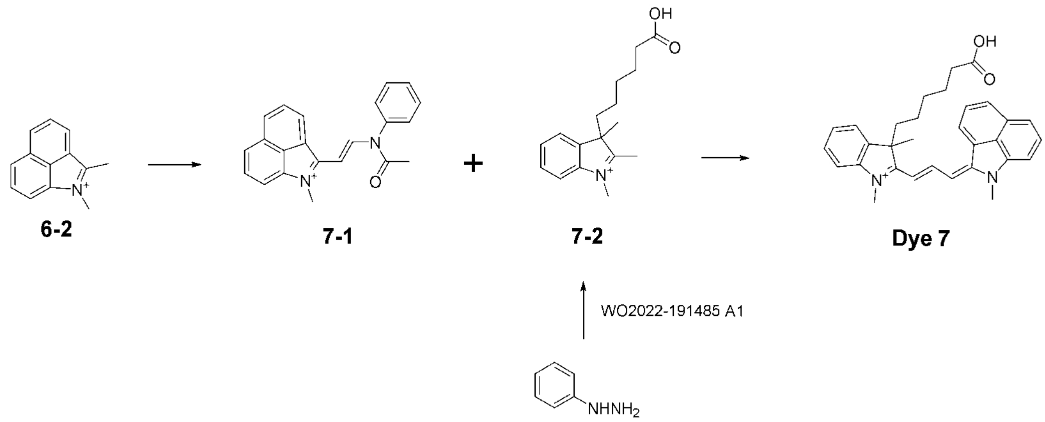


6-2 7-1 7-2 Dye 7

Synthesis of Intermediate 7-1

In a 250 mL single-necked reactor, intermediate 6-2 (5.0 g, 16 mmol), N,N-diphenylformamidine (3.8 g, 21 mmol), and acetic anhydride (40 mL) were added and stirred at 110°C for 1 hour. After cooling, ethyl acetate (100 mL) was added and vigorously stirred. The resulting solid was filtered to synthesize intermediate 7-1.

Synthesis of Intermediate 7-2

Intermediate 7-2 was synthesized with reference to WO2022-191485 A1.

Synthesis of Dye 7

In a 100 mL single-necked reactor, intermediate 7-1 (3.0 g, 6.6 mmol), intermediate 7-2 (2.8 g, 6.6 mmol), and pyridine (30 mL) were added and stirred at 50°C for 1 hour. After concentration, Dye 7 was synthesized by column purification (1.5 g).

$^1$H-NMR of the obtained Dye 7 is as follows:

$^1$H-NMR (400 MHz, DMSO-$d_6$) δ 8.73 (t, 1H, J = 12.8 Hz), 8.36-8.28 (m, 2H), 8.00 (t, 1H, J = 7.6 Hz), 7.80 Hz), 8.21 (d, 1H, J = 8.0 Hz), 7.74-7.37 (m, 6H), 6.86 (d, 2H, J = 13.6 Hz), 4.03 (q, 2H, J = 7.6 Hz), 3.85 (s, 3H), 3.80 (s, 3H), 2.53 (m, 2), 2.10-1.75 (m, 9H) 1.19 (t, 3H,J = 8.0 Hz), 1.02-0.95 (m, 1H), 0.69-0.64 (m, 1H)

**Synthesis of Dye 8**

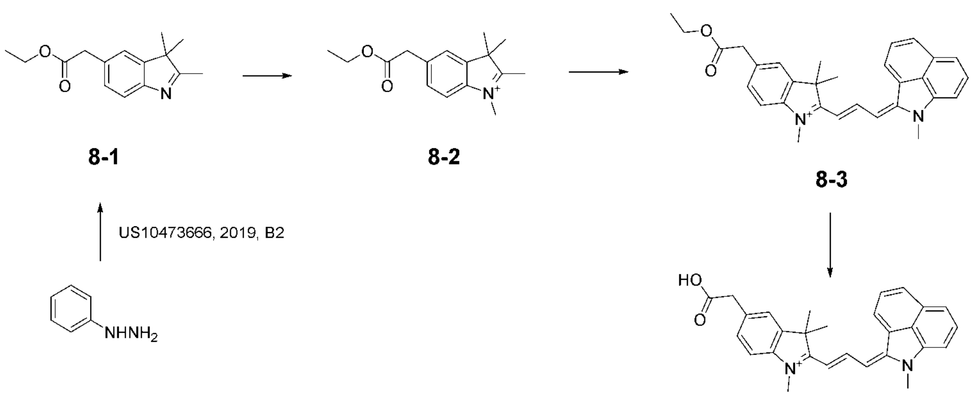


8-1 8-2 8-3 Dye 8

Synthesis of Intermediate 8-1

Intermediate 8-1 was synthesized with reference to US10473666, 2019, B2.

Synthesis of Intermediate 8-2

In a 250 mL single-necked reactor, intermediate 8-1 (10 g, 40.8 mmol), methyl iodide (8.68 g, 61.2 mmol), and acetonitrile (100 mL) were added and stirred under reflux for 24 hours. After cooling, ethyl acetate (100 mL) was added and stirred for 10 minutes. The resulting solid was filtered to synthesize intermediate 8-2.

Synthesis of Intermediate 8-3

In a 100 mL single-necked reactor, intermediate 8-2 (3.0 g, 7.7 mmol), intermediate 7-1 (3.5 g, 7.7 mmol), and pyridine (30 mL) were added and stirred at 50°C for 1 hour. After concentration, intermediate 8-3 was synthesized by column purification.

Synthesis of Dye 8

In a 100 mL single-necked reactor, intermediate 8-3 (2.0 g, 3.5 mmol), 2 N aqueous sodium hydroxide solution (5 mL), and methanol (2 mL) were added and stirred at room temperature for 24 hours. After concentration, 2 M aqueous hydrochloric acid solution (10 mL) and dichloromethane (50 mL) were added and stirred. After separating the organic layer, Dye 8 was synthesized by column purification (0.8 g).

$^1$H-NMR of the obtained Dye 8 is as follows:
$^1$H-NMR (400 MHz, DMSO-$d_6$) δ 8.73 (t, 1H, J = 12.8 Hz), 8.36-8.28 (m, 2H), 8.00 (t, 1H, J = 7.6 Hz), 7.80 (d, 1H, J = 8.0 Hz), 7.74-7.37 (m, 5H), 6.86 (d, 2H, J = 13.6 Hz), 3.96 (s, 2H), 3.85 (s, 3H), 3.80 (s, 3H), 2.12(s, 6H)

**Synthesis of Dye 9**

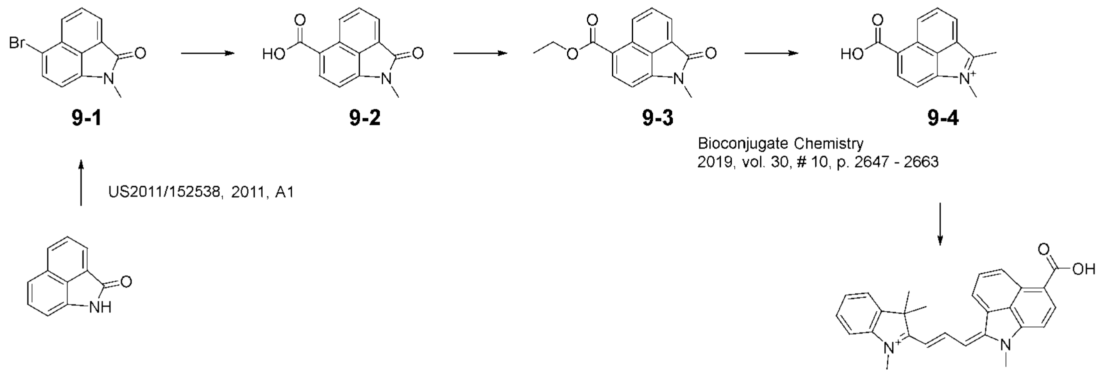


9-1 9-2 9-3 9-4

Dye 9

Synthesis of Intermediate 9-1

Intermediate 9-1 was synthesized with reference to US2011/152538, 2011, A1.

Synthesis of Intermediate 9-2

In a 250 mL three-necked reactor, intermediate 9-1 (10 g, 38 mmol) and tetrahydrofuran (100 mL) were added and stirred under a nitrogen atmosphere at -78°C for 5 minutes. 1.6 M butyllithium (24 mL, 38 mmol) was added and stirred for 1 hour. Dry ice (5 g) was added and stirred at room temperature for 1 hour. After concentration, intermediate 9-2 was synthesized by column purification.

Synthesis of Intermediate 9-3

In a 250 mL single-necked reactor, intermediate 9-2 (6 g, 23 mmol), ethyl iodide (3.9 g, 25 mmol), potassium carbonate (9.0 g, 34 mmol), and N,N-dimethylformamide (60 mL) were added and stirred at room temperature for 24 hours. The solid was filtered off and the mixture was concentrated. Intermediate 9-3 was synthesized by column purification.

Synthesis of Intermediate 9-4

Intermediate 9-4 was synthesized with reference to Bioconjugate Chemistry, 2019, vol. 30, # 10, p. 2647 - 2663.

Synthesis of Dye 9

In a 50 mL single-necked reactor, intermediate 9-4 (1.0 g, 2.6 mmol), intermediate 4-1 (1.2 g, 2.6 mmol), and pyridine (10 mL) were added and stirred at 50°C for 1 hour. After concentration, Dye 9 was synthesized by column purification (0.7 g).

$^1$H-NMR of the obtained Dye 9 is as follows:

$^1$H-NMR (400 MHz, DMSO-$d_6$) δ 8.72 (t, 1H, J = 12.8 Hz), 8.50 (d, 1H, J = 8.0 Hz), 8.34 (d, 1H, J = 8.0 Hz), 8.18 (d, 1H, J = 8.4 Hz), 7.69 (d, 1H, J = 8.4 Hz), 7.60 (t, 1H, J = 8.4 Hz), 7.57-7.50 (m, 2H), 7.45-7.10 (m, 2H), 6.98 (d, 1H, J = 13.6 Hz), 6.86 (d, 1H, J = 12.8 Hz), 3.97 (s, 3H), 3.85 (s, 3H), 2.13 (s, 6H)

**Synthesis of Dye 10**

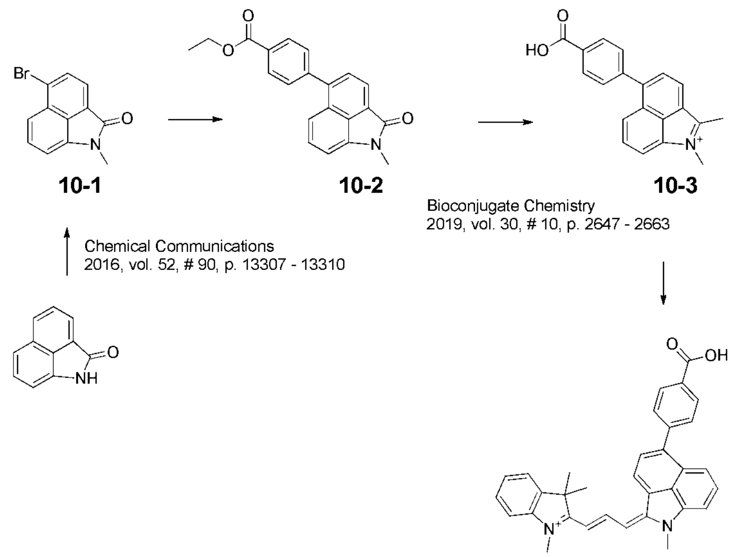


10-1 10-2 10-3

Dye 10

Synthesis of Intermediate 10-1

Intermediate 10-1 was synthesized with reference to Chemical Communications, 2016, vol. 52, # 90, p. 13307 - 13310.

Synthesis of Intermediate 10-2

In a 250 mL three-necked reactor, intermediate 10-1 (4.75 g, 18.2 mmol), 4-(ethoxycarbonyl)phenylboronic acid (5.0 g, 27.8 mmol), potassium carbonate (5.0 g, 36.4 mmol), Pd($PPh_3$)$_4$ (0.1 g, 0.09 mmol), 1,2-dimethoxyethane (30 mL), water (9 mL), and N,N-dimethylformamide (65 mL) were added and stirred at 85°C for 12 hours. After cooling, Celite was layered and the solid was filtered off. The filtrate was concentrated, and intermediate 10-2 was synthesized by column purification.

Synthesis of Intermediate 10-3

Intermediate 10-3 was synthesized with reference to Bioconjugate Chemistry, 2019, vol. 30, no. 10, pp. 2647-2663.

Synthesis of Dye 10

In a 50 mL single-necked reactor, intermediate 10-3 (2.0 g, 4.5 mmol), intermediate 4-1 (2.0 g, 4.5 mmol), and pyridine (20 mL) were added and stirred at 50°C for 1 hour. After concentration, Dye 10 was synthesized by column purification (1.1 g).

$^1$H-NMR of the obtained Dye 10 is as follows:

$^1$H-NMR (400 MHz, DMSO-$d_6$) δ 8.75 (t, 1H, J = 12.8 Hz), 8.49 (d, 1H, J = 8.0 Hz), 8.33 (d, 1H, J = 8.0 Hz), 8.16 (d, 1H, J = 8.4 Hz), 8.14 (d, 2H, J = 8.7 Hz), 7.67 (d, 1H, J = 8.4 Hz), 7.60 (t, 1H, J = 8.4 Hz), 7.60-7.49 (m, 2H), 7.47-7.12 (m, 4H), 7.00 (d, 1H, J = 13.6 Hz), 6.88 (d, 1H, J = 12.8 Hz), 3.96 (s, 3H), 3.84 (s, 3H), 2.11 (s, 6H)

**Synthesis of Dye 11**

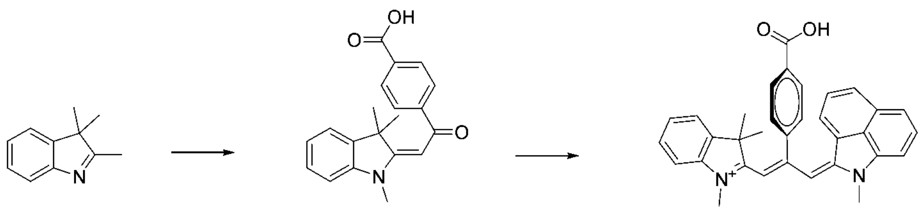

US9150922, 2015, B2

11-1

Dye 11

Synthesis of Intermediate 11-1

Intermediate 11-1 was synthesized with reference to US9150922, 2015, B2.

Synthesis of Dye 11

Dye 11 was synthesized from intermediate 11-1 and intermediate 6-2 by referring to the method described in US9150922, 2015, B2.

$^1$H-NMR of the obtained Dye 11 is as follows:

$^1$H-NMR (400 MHz, DMSO-$d_6$) δ 8.49-8.37 (m, 3H), 8.33 (d, 1H, J = 7.4 Hz), 8.21-8.00 (m, 2H), 7.67 (d, 1H, J = 8.4 Hz), 7.60 (t, 1H, J = 8.4 Hz), 7.53-7.26 (m, 6H), 7.00 (d, 1H, J = 13.6 Hz), 6.85 (m, 2H), 4.22 (s, 3H), 3.85 (s, 3H), 2.11 (s, 6H)

**Synthesis of Dye 12**

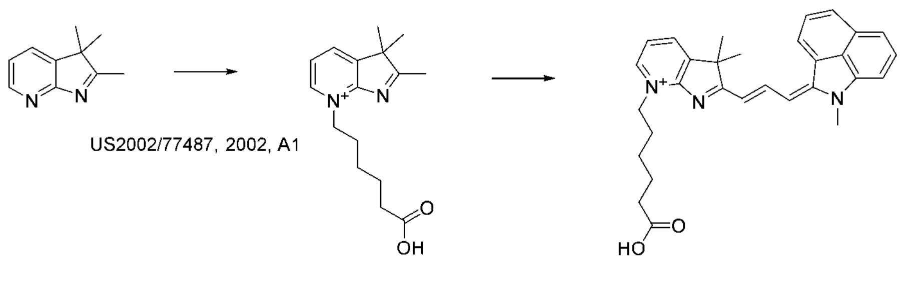


12-1

Dye 12

Synthesis of Intermediate 12-1

Intermediate 12-1 was synthesized with reference to US2002/77487, 2002, A1.

Synthesis of Dye 12

In a 50 mL single-necked reactor, intermediate 12-1 (2.0 g, 5.0 mmol), intermediate 7-1 (2.26 g, 5.0 mmol), and pyridine (20 mL) were added and stirred at 50°C for 1 hour. After concentration, Dye 12 was synthesized by column purification (0.9 g).

$^1$H-NMR of the obtained Dye 12 is as follows:

$^1$H-NMR (400 MHz, DMSO-$d_6$) δ 8.87 (m, 2H), 8.53-8.33 (m, 3H), 8.20 (d, 1H, J = 7.2 Hz), 7.67 (d, 1H, J = 8.4 Hz), 7.60 (t, 1H, J = 8.4 Hz), 7.42-7.26 (m, 2H), 6.85 (m, 2H), 4.52 (q, 2H, J = 7.2 Hz), 3.85 (s, 3H), 2.52 (m, 2H), 2.11 (s, 6H), 2.07-1.71 (m, 6H)

**Synthesis of Dye 13**

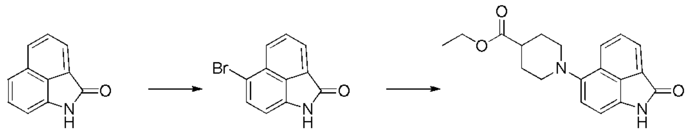

**13-1** **13-2**

Journal of Organic Chemistry
2018, vol. 83, # 8, p. 4389 - 4401

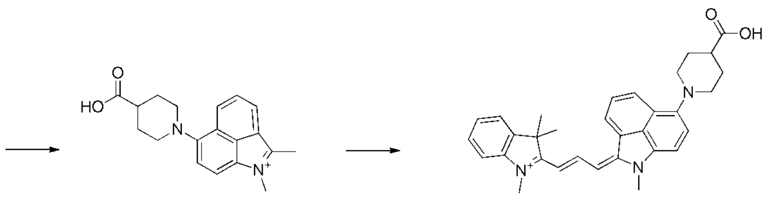

**13-3** **Dye 13**

Bioconjugate Chemistry
2019, vol. 30, # 10, p. 2647 - 2663

Synthesis of Intermediate 13-1

Intermediate 13-1 was synthesized with reference to Journal of Organic Chemistry, 2018, vol. 83, # 8, p. 4389 - 4401.

Synthesis of Intermediate 13-2

In a 1 L four-necked reactor, intermediate 13-1 (50 g, 0.202 mol), ethyl 4-piperidinecarboxylate (38 g, 0.242 mol), $Pd_2(dba)_3$ (7.4 g, 0.008 mol), BINAP (5.0 g, 0.008 mol), sodium tert-butoxide (27 g, 0.282 mol), and toluene (500 mL) were added and stirred under reflux for 12 hours. After cooling, celite was applied and the solid was filtered. The filtrate was concentrated and intermediate 13-2 was synthesized by column purification.

Synthesis of Intermediate 13-3

Intermediate 13-3 was synthesized from intermediate 13-2 by referring to the method described in Bioconjugate Chemistry, 2019, vol. 30, # 10, p. 2647 - 2663.

Synthesis of Dye 13

In a 50 mL single-necked reactor, intermediate 13-3 (0.7 g, 1.6 mmol), intermediate 4-1 (0.72 g, 1.6 mmol), and pyridine (15 mL) were added and stirred at 50°C for 1 hour. After concentration, Dye 13 was synthesized by column purification (0.3 g).

$^1$H-NMR of the obtained Dye 13 is as follows:

$^1$H-NMR (400 MHz, DMSO-$d_6$) δ 8.75 (t, 1H, J = 12.6 Hz), 8.49 (d, 1H, J = 8.0 Hz), 8.31 (d, 1H, J = 8.28 (d, 1H, J = 8.4 Hz), 7.73-7.63 (m, 2H), 7.57-7.52 (m, 2H), 7.45-7.15 (m, 2H), 6.99 (d, 1H, J = 13.6 Hz), 6.88 (d, 1H, J = 12.8 Hz), 3.95 (s, 3H),

3.81 (s, 3H), 3.44 (m, 4H), 2.49-2.44 (m, 1H), 2.10 (s, 6H), 1.88-1.61 (m, 4H)

**Synthesis of Dye 14**

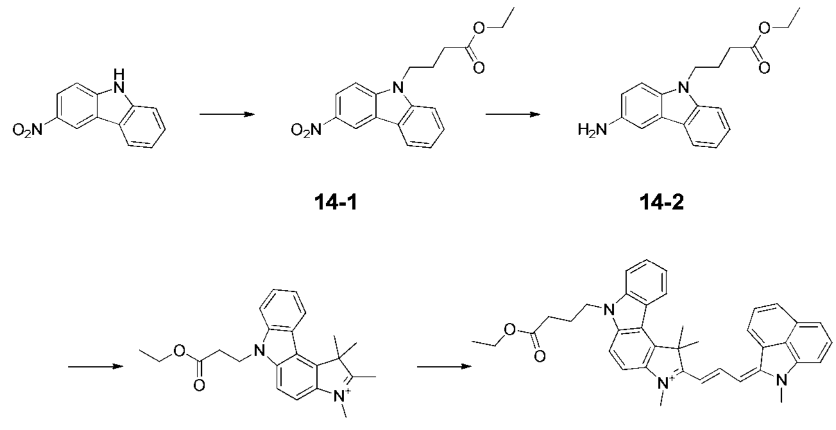

14-1 14-2

KR2020/67733, 2020, A 14-3 Dye 14

Synthesis of Intermediate 14-1

In a 500 mL single-necked reactor, 3-nitro-9H-carbazole (20 g, 94 mmol), ethyl 4-bromobutyrate (22 g, 113 mmol), potassium carbonate (26 g, 190 mmol), and N,N-dimethylformamide (200 mL) were added and stirred at 50°C for 12 hours. After concentration, dichloromethane (200 mL) and water (100 mL) were added, stirred for 5 minutes, and the organic layer was separated. Sodium sulfate anhydrous was added, stirred for 5 minutes, and filtered. The filtrate was concentrated and intermediate 14-1 was synthesized by column purification.

Synthesis of Intermediate 14-2

In a 500 mL single-necked reactor, intermediate 14-1 (12 g, 36 mmol), 5% Pd/C (0.6 g), and methanol (120 mL) were added and stirred under a hydrogen atmosphere for 24 hours. Celite was applied, filtered, and the filtrate was concentrated to synthesize intermediate 14-2.

Synthesis of Intermediate 14-3

Intermediate 14-3 was synthesized from intermediate 14-2 by referring to the method described in KR2020/67733, 2020, A.

Synthesis of Dye 14

In a 50 mL single-necked reactor, intermediate 14-3 (1.6 g, 3.2 mmol), intermediate 7-1 (1.4 g, 3.2 mmol), and pyridine (15 mL) were added and stirred at 50°C for 1 hour. After concentration, Dye 14 was synthesized by column purification (0.7 g).

$^1$H-NMR of the obtained Dye 14 is as follows:

$^1$H-NMR (400 MHz, DMSO-$d_6$) δ 8.72 (t, 1H, J = 12.8 Hz), 8.52 (d, 1H, J = 8.0 Hz), 8.40 (d, 1H, J = 7.8 Hz), 8.22 (d, 1H, J = 8.4 Hz), 8.03 (t, 1H, J = 7.2 Hz), 7.86-7.62 (m, 7H), 7.46-7.40 (m, 2H), 7.00 (d, 1H, J = 13.6 Hz), 6.83 (d, 1H, J = 12.8 Hz), 4.43 (t, 2H, J = 7.6 Hz), 4.12 (q, 2H, J = 7.6 Hz), 3.95 (s, 3H), 3.82 (s, 3H), 2.25 (t, 2H, J = 7.2 Hz), ), 2.12 (s, 6H), 1.91-1.87 (m, 2H), 1.33 (t, 3H, J = 7.6 Hz)

**Synthesis of Dye 15**

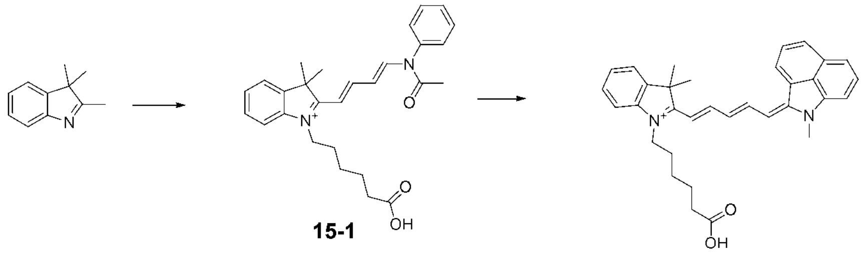

15-1

Journal of the American Chemical Society
2011, vol. 133, # 1, p. 51 - 55

Dye 15

Synthesis of Intermediate 15-1

Intermediate 15-1 was synthesized with reference to Journal of the American Chemical Society, 2011, vol. 133, # 1, p. 51 - 55.

Synthesis of Dye 15

In a 100 mL single-necked reactor, intermediate 15-1 (2.0 g, 3.5 mmol), intermediate 6-2 (1.1 g, 3.5 mmol), and pyridine (20 mL) were added and stirred at 50°C for 1 hour. After concentration, Dye 15 was synthesized by column purification (0.9 g).

$^1$H-NMR of the obtained Dye 15 is as follows:

$^1$H-NMR (400 MHz, DMSO-$d_6$) δ 8.54 (d, 1H, J = 8.0 Hz), 8.49 (t, 2H, J = 15.6 Hz), 8.32 (d, 1H, J = 7.6 Hz), 8.31 (d, 1H, J = 8.2 Hz), 8.00 (t, 1H, J = 7.0 Hz), 7.72 (d, 1H, J = 8.4 Hz), 7.61 (t, 1H, J = 8.4 Hz), 7.60-7.50 (m, 2H), 7.44-7.14 (m, 2H), 6.87 (t, 1H, J = 13.6 Hz), 6.36 (d, 2H, J = 13.6 Hz), 4.23 (t, 2H, J = 7.2 Hz), 3.83 (s, 3H), 2.23 (m, 2H), 2.12 (s, 6H), 2.10-1.76 (m, 6H)

**Synthesis of Dye 16**

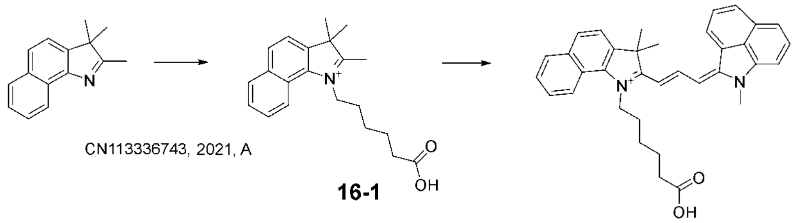


16-1

Dye 16

Synthesis of Intermediate 16-1

Intermediate 16-1 was synthesized with reference to CN113336743, 2021, A.

Synthesis of Dye 16

In a 100 mL single-necked reactor, intermediate 16-1 (3.0 g, 6.6 mmol), intermediate 7-1 (3.0 g, 6.6 mmol), and pyridine (30 mL) were added and stirred at 50°C for 1 hour. After concentration, Dye 16 was synthesized by column purification (2.1 g).

$^1$H-NMR of the obtained Dye 16 is as follows:

$^1$H-NMR (400 MHz, DMSO-$d_6$) δ 8.89 (t, 1H, J = 12.6 Hz), 8.23-8.00 (m, 5H), 7.89 (t, 1H, J = 7.2 Hz), 7.77-7.47 (m, 7H), 7.19 (d, 1H, J = 7.2 Hz), 4.51 (t, 2H, J = 8.4 Hz), 3.97 (s, 3H), 2.54 (t, 2H, J = 6.8 Hz), 2.12 (s, 6H), 2.03-1.77 (m, 6H)

**Synthesis of Dye 17**

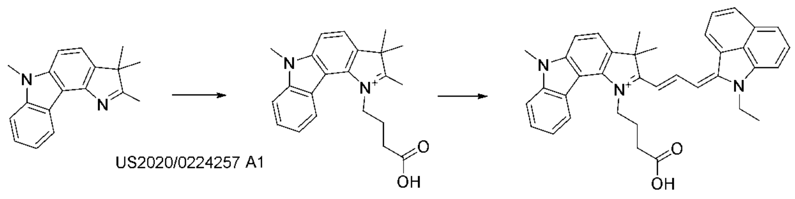


17-1 Dye 17

Synthesis of Intermediate 17-1

Intermediate 17-1 was synthesized with reference to US2020/0224257 A1.

Synthesis of Dye 17

In a 100 mL single-necked reactor, intermediate 17-1 (3.0 g, 5.8 mmol), intermediate 7-1 (2.6 g, 5.8 mmol), and pyridine (30 mL) were added and stirred at 50°C for 1 hour. After concentration, Dye 17 was synthesized by column purification (1.7 g).

$^1$H-NMR of the obtained Dye 17 is as follows:

$^1$H-NMR (400 MHz, DMSO-$d_6$) $\delta$ 8.75 (t, 1H, J = 12.8 Hz), 8.49 (d, 1H, J = 7.8 Hz), 8.44 (d, 1H, J = 7.6 Hz), 8182 (d, 1H, J = 8.2 Hz), 8.01 (t, 1H, J = 7.6 Hz), 7.86-7.40 (m, 8H), 6.68 (m, 2H), 4.58 (q, 2H, J = 6.8 Hz), 4.44 (t, 2H, J = 7.2 Hz), 4.21 (q, 2H, J = 7.8 Hz), 3.52 (s, 3H), 2.25 (t, 2H, J = 7.2 Hz), 2.11 (s, 6H), 1.93 (m, 2H), 1.32 (t, 3H, J = 7.8 Hz)

**Synthesis of Dye 18**

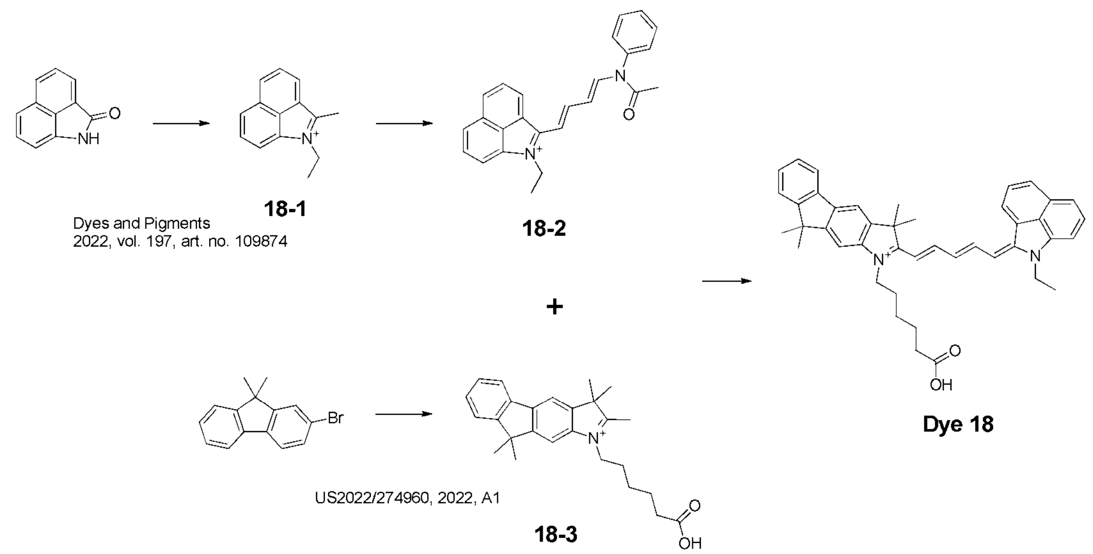


18-1 18-2 + 18-3 Dye 18

Synthesis of Intermediate 18-1

Intermediate 18-1 was synthesized with reference to Dyes and Pigments, 2022, vol. 197, art. no. 109874.

Synthesis of Intermediate 18-2

In a 250 mL single-necked reactor, Intermediate 18-1 (10 g, 3.1 mmol), malonaldehyde dianilide hydrochloride (8.8 g, 34 mmol), and acetic anhydride (100 mL) were added and stirred under reflux for 1 hour. After cooling, ethyl acetate (100 mL) was added to the reactor and stirred for 5 minutes. The resulting solid was filtered to synthesize Intermediate 18-2.

Synthesis of Intermediate 18-3

Intermediate 18-3 was synthesized with reference to US2022/274960, 2022, A1.

Synthesis of Dye 18

In a 100 mL single-necked reactor, intermediate 18-2 (1.9 g, 3.9 mmol), intermediate 18-3 (2.0 g, 3.9 mmol), and pyridine (20 mL) were added and stirred at 50°C for 1 hour. After concentration, Dye 18 was synthesized by column purification (0.7 g).

$^1$H-NMR of the obtained Dye 18 is as follows:
$^1$H-NMR (400 MHz, DMSO-$d_6$) $\delta$ 8.54 (d, 1H, J = 8.0 Hz), 8.49 (m, 2H), 8.32 (d, 1H, J = 8.0 Hz), 8.00 (t, 1H, J = 7.0 Hz), 7.81-7-40 (m, 9H), 6.87 (t, 1H, J = 13.6 Hz), 6.52 (d, 1H, J = 12.8 Hz) 6.36 (d, 1H, J = 13.6 Hz), 4.58 (q, 2H, J = 6.8 Hz), 4.23 (t, 2H, J = 7.2 Hz), 2.25 (t, 2H, J = 7.2 Hz), 1.93-1.75 (s, 9H), 1.56 (s, 6H), 1.35 (t, 3H, J = 7.2 Hz)

**Synthesis of Dye 19**

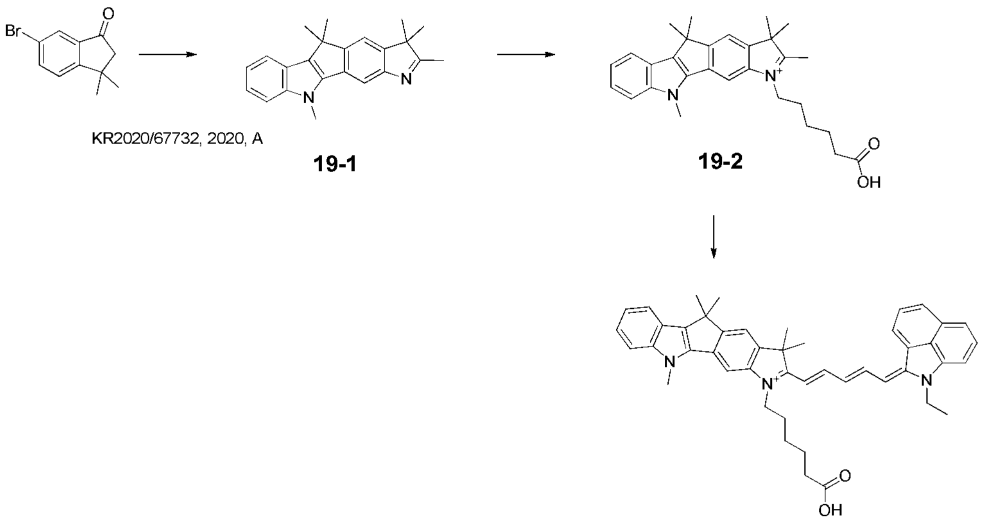


19-1

19-2

Dye 19

Synthesis of Intermediate 19-1

Intermediate 19-1 was synthesized with reference to KR2020/67732, 2020, A.

Synthesis of Intermediate 19-2

In a 250 mL single-necked reactor, Intermediate 19-1 (10 g, 30 mmol), 6-bromohexanoic acid (8.9 g, 46 mmol), and 1,2-dichlorobenzene (100 mL) were added and stirred at 150°C for 2 hours. After cooling, acetone (100 mL) was added to the reactor and stirred for 5 minutes. The resulting solid was filtered to synthesize Intermediate 19-2.

Synthesis of Dye 19

In a 100 mL single-necked reactor, Intermediate 19-2 (2.0 g, 3.8 mmol), Intermediate 18-2 (1.9 g, 3.8 mmol), and pyridine (20 mL) were added and stirred at 50°C for 1 hour. After concentration, Dye 19 was synthesized by column purification (0.3 g).

$^1$H-NMR of the obtained Dye 19 is as follows:
$^1$H-NMR (400 MHz, DMSO-$d_6$) $\delta$ 8.48-8.51 (m, 3H), 8.40 (d, 1H, J = 7.8 Hz), 8.22 (d, 1H, J = 8.4 Hz), 8.03 (t, 1H, J = 7.6 Hz), 7.86-7.40 (m, 6H), 7.19 (t, 1H, J = 8.0 Hz), 7.09 (t, 1H, 8.4 Hz), 6.75-6.53 (m, 3H), 4.41 (t, 2H, J = 7.2 Hz), 4.23 (q, 2H, J = 6.8 Hz), 3.91 (s, 3H), 2.53 (t, 2H, J = 7.2 Hz), 2.13 (s, 6H), 1.90-1.50 (m, 9H), 1.33 (t, 3H, J = 7.8 Hz)

**Synthesis of Dye 20**

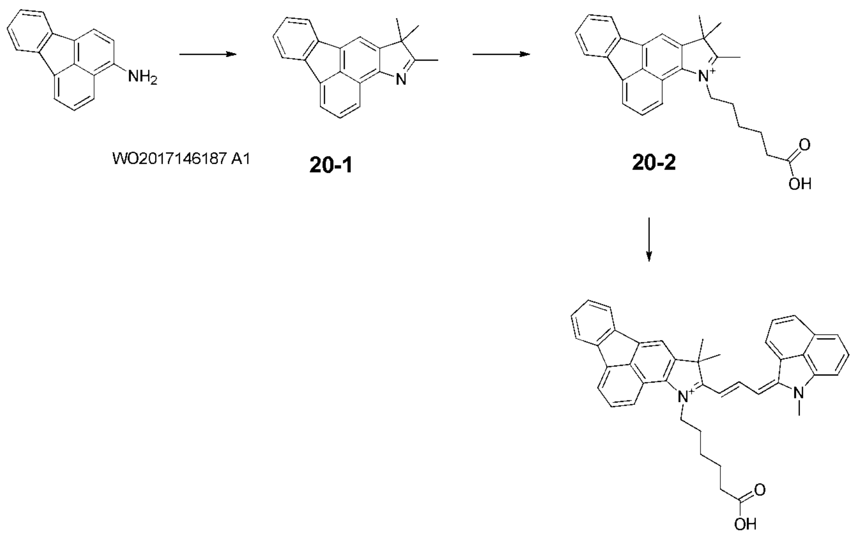

WO2017146187 A1

**20-1**

**20-2**

**Dye 20**

Synthesis of Intermediate 20-1

Intermediate 20-1 was synthesized with reference to WO2017146187 A1.

Synthesis of Intermediate 20-2

In a 250 mL single-necked reactor, Intermediate 20-1 (10 g, 35 mmol), 6-bromohexanoic acid (10.3 g, 53 mmol), and 1,2-dichlorobenzene (100 mL) were added and stirred at 150°C for 2 hours. After cooling, acetone (100 mL) was added to the reactor and stirred for 5 minutes. The resulting solid was filtered to synthesize Intermediate 20-2.

Synthesis of Dye 20

In a 100 mL single-necked reactor, Intermediate 20-2 (3.0 g, 6.3 mmol), Intermediate 7-1 (2.9 g, 6.3 mmol), and pyridine (30 mL) were added and stirred at 50°C for 1 hour. After concentration, Dye 20 was synthesized by column purification (2.3 g).

$^1$H-NMR of the obtained Dye 20 is as follows:

$^1$H-NMR (400 MHz, DMSO-$d_6$) $\delta$ 8.59-8.52 (m, 4H), 8.38 (d, 1H, J = 8.0 Hz), 8.30 (d, 1H), 8.22 (d, 1H, J = 8.2 Hz), 8.14-8.10 (m, 2H), 8.03 (t, 1H, J = 7.2 Hz), 7.90-7.65 (m, 3H), 7.53-7.42 (m, 2H), 6.83 (m, 2H), 4.28 (t, 2H, J = 6.8 Hz), 3.83 (s, 3H), 2.52 (t, 2H, J = 7.6 Hz), 2.10 (s, 6H), 1.98-1.53 (m, 6H), 1.30 (t, 3H, J = 7.6 Hz)

**Synthesis of Dye 21**

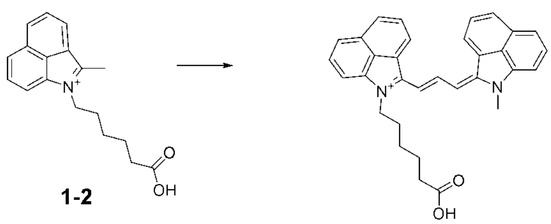

**1-2**

**Dye 21**

Synthesis of Dye 21

In a 100 mL single-necked reactor, Intermediate 1-2 (2.0 g, 4.9 mmol), Intermediate 7-1 (2.2 g, 4.9 mmol), and pyridine (10 mL) were added and stirred at 50°C for 1 hour. After concentration, Dye 21 was synthesized by column purification (1.7 g).

$^1$H-NMR of the obtained Dye 21 is as follows:
$^1$H-NMR (400 MHz, DMSO-$d_6$) δ 9.14 (t, 1H, J = 12.8 Hz), 8.64 (d, 2H, J = 7.2 Hz), 8.33 (d, 2H, J = 7.6 Hz), 8.06-8.01 (m, 2H), 7.85 (d, 2H, J = 7.0 Hz), 7.70-7.67 (m, 4H), 7.17 (d, 2H, J = 13.2 Hz), 4.33 (t, 2H, J =7.2 Hz), 3.92 (s, 3H), 2.51 (t, 2H, J = 7.6 Hz), 1.92-1.43 (m, 6H)

**Synthesis of Dye** 22

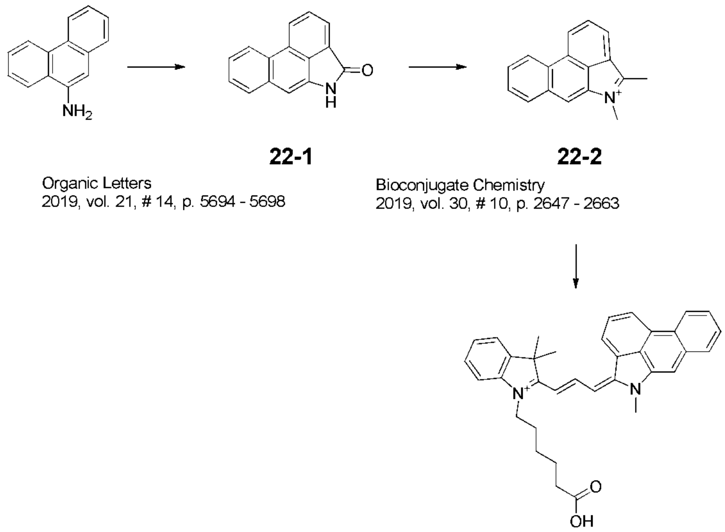


**Dye 22**

Synthesis of Intermediate 22-1

Intermediate 22-1 was synthesized with reference to Organic Letters, 2019, vol. 21, # 14, p. 5694 - 5698.

Synthesis of Intermediate 22-2

Intermediate 22-2 was synthesized from intermediate 22-1 by referring to the method described in Bioconjugate Chemistry, 2019, vol. 30, # 10, p. 2647 - 2663.

Synthesis of Dye 22

In a 100 mL single-necked reactor, Intermediate 22-2 (1.3 g, 3.7 mmol), Intermediate 6-1 (2.0 g, 3.7 mmol), and pyridine (13 mL) were added and stirred at 50°C for 1 hour. After concentration, Dye 22 was synthesized by column purification (0.6 g).

$^1$H-NMR of the obtained Dye 22 is as follows:
$^1$H-NMR (400 MHz, DMSO-$d_6$) δ 8.54 (t, 1H, J = 12.8 Hz), 8.37 (d, 1H, J = 7.8 Hz) 8.30 (d, 1H, J = 8.2 Hz), 8.04 (t, 1H, J = 7.2 Hz), 7.70-7.50 (m, 5H), 7.45-7.10 (m, 4H), 7.00 (d, 1H, J = 13.6 Hz), 6.88 (d, 1H, J = 12.8 Hz), 4.28 (t, 2H, J = 7.2 Hz), 3.84 (s, 3H), 2.51 (m, 2H), 2.13 (s, 6H), 2.09-1.75 (m, 6H)

**Synthesis of Dye 23**

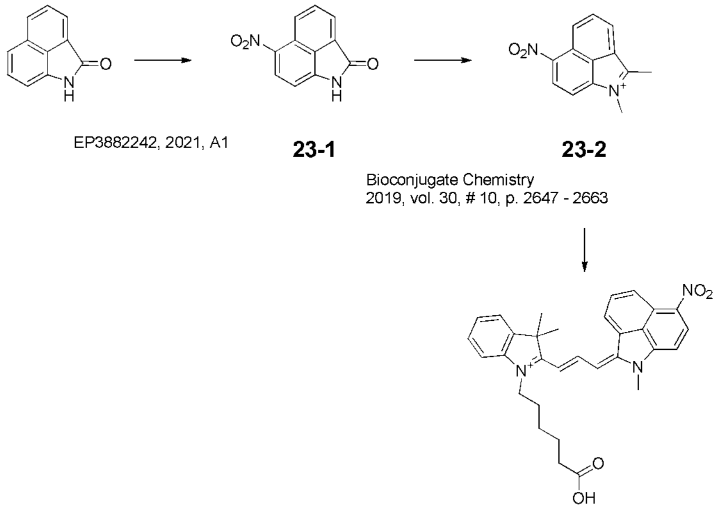


**23-1** **23-2**

**Dye 23**

Synthesis of Intermediate 23-1

Intermediate 23-1 was synthesized with reference to EP3882242, 2021, A1.

Synthesis of Intermediate 23-2

Intermediate 23-2 was synthesized from intermediate 23-1 by referring to the method described in Bioconjugate Chemistry, 2019, vol. 30, # 10, p. 2647 - 2663.

Synthesis of Dye 23

In a 100 mL single-necked reactor, Intermediate 23-2 (3.2 g, 9.0 mmol), Intermediate 6-1 (4.9 g, 9.0 mmol), and pyridine (30 mL) were added and stirred at 50°C for 1 hour. After concentration, Dye 23 was synthesized by column purification (1.9 g).

$^1$H-NMR of the obtained Dye 23 is as follows:
$^1$H-NMR (400 MHz, DMSO-$d_6$) δ 9.07 (d, 1H, J = 8.4 Hz), 8.72 (t, 1H, J = 12.8 Hz), 8.62 (d, 1H, J = 8.0 Hz), 8.41 (d, 1H, J = 7.8 Hz), 8.18 (d, 1H, J = 8.4 Hz), 7.62 (t, 1H, J = 8.4 Hz), 7.64-7.50 (m, 2H), 7.48-7.28 (m, 2H), 6.98 (d, 1H, J = 12.8 Hz), 6.84 (d, 1H, J = 12.8 Hz), 4.24 (t, 2H, J = 7.6 Hz), 3.97 (s, 3H), 2.52 (m, 2H), 2.10 (s, 6H), 2.11-1.73 (m, 6H)

**Synthesis of Dye 24**

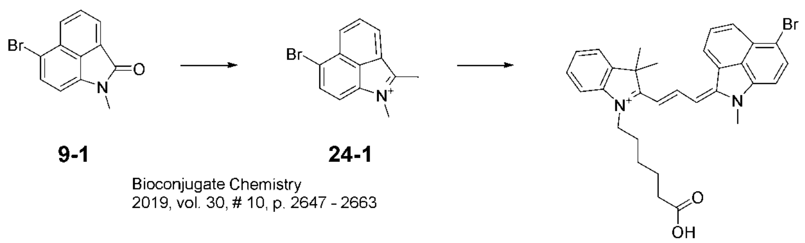


**9-1** **24-1**

**Dye 24**

Synthesis of Intermediate 24-1

Intermediate 24-1 was synthesized with reference to Bioconjugate Chemistry, 2019, vol. 30, no. 10, pp. 2647-2663.

Synthesis of Dye 24

In a 100 mL single-necked reactor, Intermediate 24-1 (2.6 g, 6.7 mmol), Intermediate 6-1 (3.7 g, 6.7 mmol), and pyridine (30 mL) were added and stirred at 50°C for 1 hour. After concentration, Dye 24 was synthesized by column purification (1.9 g).

$^1$H-NMR of the obtained Dye 24 is as follows:

$^1$H-NMR (400 MHz, DMSO-$d_6$) δ 8.70 (t, 1H, J = 12.8 Hz), 8.58 (d, 1H, J = 8.0 Hz), 8.39 (d, 1H, J = 7.2 Hz), 8.24 (d, 1H, J = 8.0 Hz), 7.89 (d, 1H, J = 8.4 Hz), 7.62 (t, 1H, J = 8.4 Hz), 7.59-7.28 (m, 4H), 6.82 (d, 1H, J = 12.8 Hz), 6.51 (d, 1H, J = 12.6 Hz), 4.22 (t, 2H, J = 7.8 Hz), 3.83 (s, 3H), 2.51 (m, 2H), 2.13 (s, 6H), 2.10-1.59 (m, 6H)

**Synthesis of Dye 25**

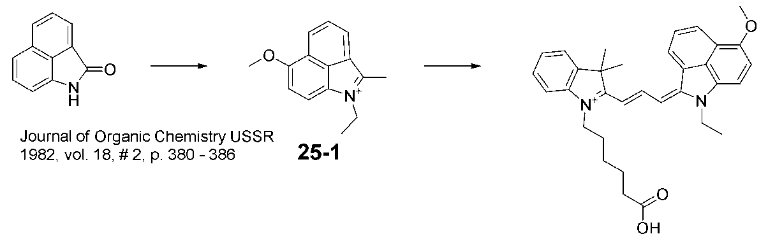


**25-1**

**Dye 25**

Synthesis of Intermediate 25-1

Intermediate 25-1 was synthesized with reference to Journal of Organic Chemistry USSR, 1982, vol. 18, no. 2, pp. 380-386.

Synthesis of Dye 25

In a 100 mL single-necked reactor, Intermediate 25-1 (2.0 g, 5.7 mmol), Intermediate 6-1 (3.1 g, 5.7 mmol), and pyridine (20 mL) were added and stirred at 50°C for 1 hour. After concentration, Dye 25 was synthesized by column purification (0.8 g).

$^1$H-NMR of the obtained Dye 25 is as follows:

$^1$H-NMR (400 MHz, DMSO-$d_6$) δ 8.64 (t, 1H, J = 13.6 Hz), 8.55 (d, 1H, J = 8.0 Hz), 8.34 (d, 1H, J = 7.6 Hz), 8.22 (d, 1H, J = 7.8 Hz), 7.86 (d, 1H, J = 8.4 Hz), 7.60 (t, 1H, J = 8.4 Hz), 7.53-7.22 (m, 4H), 6.80 (d, 1H, J = 12.8 Hz), 6.50 (d, 1H, J = 12.6 Hz), 4.22 (t, 2H, J = 7.6 Hz), 4.15 (q, 2H, J = 7.8 Hz), 3.90 (s, 3H), 2.51 (m, 2H), 2.11 (s, 6H), 2.11-1.50 (m, 6H), 1.32 (t, 3H, J = 7.2 Hz)

**Synthesis of Dye 26**

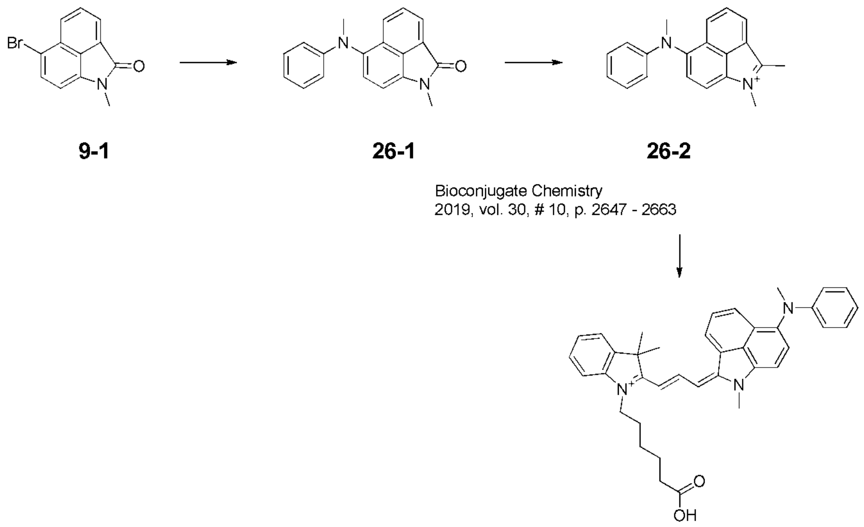


9-1 26-1 26-2

**Dye 26**

Synthesis of Intermediate 26-1

In a 500 mL four-necked reactor, Intermediate 9-1 (20 g, 0.077 mol), N-methylaniline (9.8 g, 0.092 mol), $Pd_2(dba)_3$ (2.8 g, 0.003 mol), BINAP (1.9 g, 0.003 mol), sodium tert-butoxide (10.3 g, 0.107 mol), and toluene (200 mL) were added and stirred under reflux for 12 hours. After cooling, celite filtration was performed to remove solids. The filtrate was concentrated, and intermediate 26-1 was synthesized by column purification.

Synthesis of Intermediate 26-2

Intermediate 26-2 was synthesized from intermediate 26-1 by referring to the method described in Bioconjugate Chemistry, 2019, vol. 30, no. 10, pp. 2647-2663.

Synthesis of Dye 26

In a 100 mL single-necked reactor, Intermediate 26-2 (3.0 g, 7.2 mmol), Intermediate 6-1 (4.0 g, 7.2 mmol), and pyridine (30 mL) were added and stirred at 50°C for 1 hour. After concentration, Dye 26 was synthesized by column purification (1.9 g).

$^1$H-NMR of the obtained Dye 26 is as follows:

$^1$H-NMR (400 MHz, DMSO-$d_6$) δ 8.63 (t, 1H, J = 13.6 Hz), 8.52 (d, 1H, J = 7.8 Hz), 8.32 (d, 1H, J = 7.8 Hz), 8.21 (d, 1H, J = 7.8 Hz), 7.84 (d, 1H, J = 8.2 Hz), 7.67 (t, 1H, J = 8.2 Hz), 7.58-7.22 (m, 9H), 6.96 (d, 1H, J = 12.6 Hz), 6.69 (d, 1H, J = 13.2 Hz), 4.22 (t, 2H, J = 7.6 Hz), 3.84 (s, 3H), 3.42 (s, 3H), 2.53 (m, 2H), 2.12 (s, 6H), 2.10-1.47 (m, 6H)

**Synthesis of Dye 27**

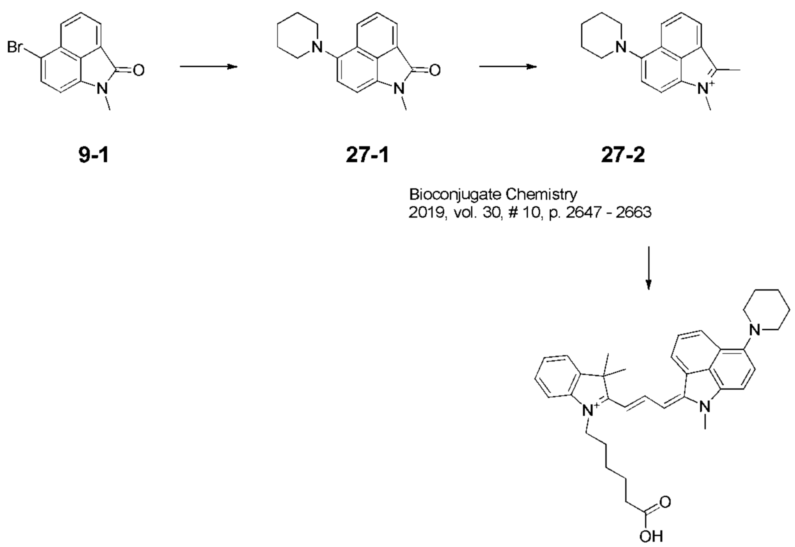


9-1 27-1 27-2

Dye 27

Synthesis of Intermediate 27-1

In a 500 mL four-necked reactor, Intermediate 9-1 (20 g, 0.077 mol), piperidine (7.8 g, 0.092 mol), $Pd_2(dba)_3$ (2.8 g, 0.003 mol), BINAP (1.9 g, 0.003 mol), sodium tert-butoxide (10.3 g, 0.107 mol), and toluene (200 mL) were added and stirred under reflux for 12 hours. After cooling, celite filtration was performed to remove solids. The filtrate was concentrated, and Intermediate 27-1 was synthesized by column purification.

Synthesis of Intermediate 27-2

Intermediate 27-2 was synthesized from intermediate 27-1 by referring to the method described in Bioconjugate Chemistry, 2019, vol. 30, no. 10, pp. 2647-2663.

Synthesis of Dye 27

In a 100 mL single-necked reactor, Intermediate 27-2 (3.0 g, 7.6 mmol), Intermediate 6-1 (4.2 g, 7.2 mmol), and pyridine (30 mL) were added and stirred at 50°C for 1 hour. After concentration, Dye 27 was synthesized by column purification (1.6 g).

$^1$H-NMR of the obtained Dye 27 is as follows:

$^1$H-NMR (400 MHz, DMSO-$d_6$) $\delta$ 8.67 (t, 1H, J = 13.6 Hz), 8.56 (d, 1H, J = 7.6 Hz), 8.37 (d, 1H, J = 8.0 Hz), 8.24 (d, 1H, J = 7.6 Hz), 7.84 (d, 1H, J = 8.0 Hz), 7.67 (m, 1H), 7.55-7.20 (m, 4H), 6.87 (d, 1H, J = 12.6 Hz), 6.70 (d, 1H, J = 12.6 Hz), 4.24 (t, 2H, J = 7.6 Hz), 3.84 (s, 3H), 3.40-3.34 (m, 4H), 2.53 (m, 2H), 2.12 (s, 6H), 2.10-1.47 (m, 12H)

**Synthesis of Dye 28**

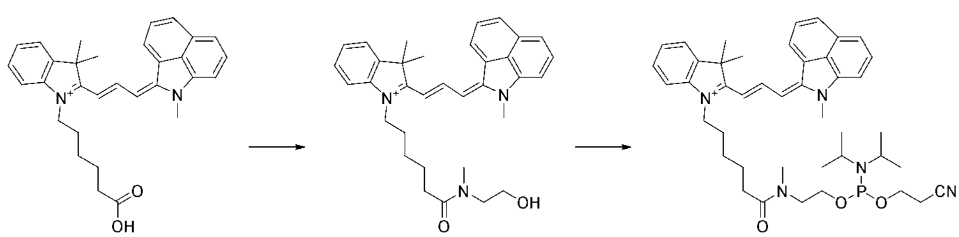

Dye 6 28-1 Dye 28

Synthesis of Intermediate 28-1

In a 100 mL single-necked reactor, Dye 6 (2 g, 3.4 mmol), 2-(methylamino)ethanol (0.3 g, 4.1 mmol), HATU (1.9 g, 5.1 mmol), triethylamine (1.4 mL, 10.1 mmol), and dimethylformamide (20 mL) were added and stirred at room temperature for 1 hour. After concentration, water was added to the reactor, and the mixture was vigorously stirred and extracted with dichloromethane. Sodium sulfate was added, stirred for 5 minutes, and filtered. The filtrate was concentrated, and Intermediate 28-1 was synthesized by column purification.

Synthesis of Dye 28

In a 25 mL single-necked reactor, Intermediate 28-1 (1.8 g, 2.8 mmol), 2-cyanoethyl N,N'-diisopropylchlorophosphoramidite (0.85 g, 3.6 mmol), triethylamine (0.77 mL, 5.5 mmol), and dichloromethane (20 mL) were added and stirred at room temperature for 1 hour. Water was added to the reactor, and the mixture was vigorously stirred and extracted with dichloromethane. Sodium sulfate was added, stirred for 5 minutes, and filtered. The filtrate was concentrated, and Dye 28 was synthesized by column purification (1.5 g).

$^1$H-NMR of the obtained Dye 28 is as follows:

$^1$H-NMR (400 MHz, DMSO-$d_6$) δ 8.75 (t, 1H, J = 12.6 Hz), 8.50 (d, 1H, J = 8.0 Hz), 8.35 (d, 1H, J = 8.0 Hz), 8.21 (d, 1H, J = 8.2 Hz), 8.03 (t, 1H, J = 7.2 Hz), 7.70 (d, 1H, J = 8.4 Hz), 7.60 (t, 1H, J = 8.2 Hz), 7.60-7.50 (m, 2H), 7.44-7.12 (m, 2H), 6.97 (d, 1H, J = 13.6 Hz), 6.84 (d, 1H, J = 12.8 Hz), 4.25 (t, 2H, J = 7.4 Hz), 3.83 (s, 3H), 3.50-3.42 (m, 11H), 2.69 (t, 2H, J = 5.6 Hz), 2.48 (m, 2H), 2.13 (s, 6H), 2.10-1.75 (m, 6H), 1.20-1.18 (m, 12H)

**Synthesis of Dye 29**

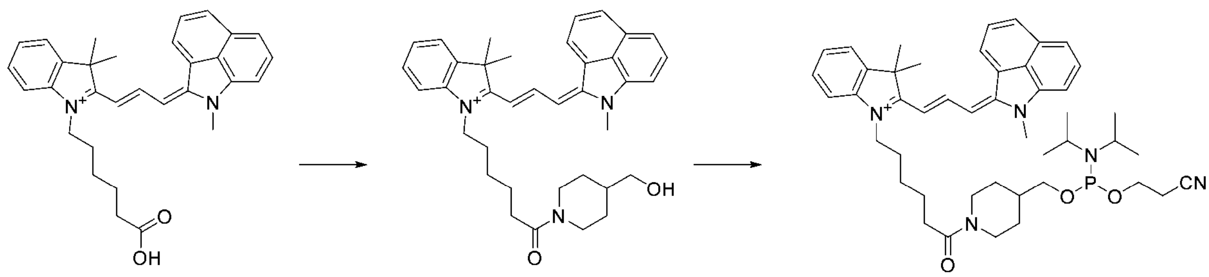

Dye 6 29-1 Dye 29

Synthesis of Intermediate 29-1

In a 100 mL single-necked reactor, Dye 6 (3.0 g, 5.1 mmol), 4-piperidinemethanol (0.70 g, 6.1 mmol), HATU (2.9 g, 7.6 mmol), triethylamine (2.1 mL, 15.2 mmol), and dimethylformamide (30 mL) were added and stirred at room temperature for 1 hour. After concentration, water was added to the reactor, and the mixture was vigorously stirred and extracted with dichloromethane. Sodium sulfate was added, stirred for 5 minutes, and filtered. The filtrate was concentrated, and Intermediate 29-1 was synthesized by column purification.

Synthesis of Dye 29

In a 25 mL single-necked reactor, Intermediate 29-1 (2.6 g, 3.8 mmol), 2-cyanoethyl N,N'-diisopropylchlorophosphoramidite (1.2 g, 4.9 mmol), triethylamine (1 mL, 7.5 mmol), and dichloromethane (25 mL) were added and stirred at room temperature for 1 hour. Water was added to the reactor, and the mixture was vigorously stirred and extracted with dichloromethane. Sodium sulfate was added, stirred for 5 minutes, and filtered. The filtrate was concentrated, and Dye 29 was synthesized by column purification (2.2 g).

$^1$H-NMR of the obtained Dye 29 is as follows:

$^1$H-NMR (400 MHz, DMSO-$d_6$) δ 8.77 (t, 1H, J = 12.8 Hz), 8.50 (d, 1H, J = 7.8 Hz), 8.33 (d, 1H, J = 7.8 Hz), 8.27 (d, 1H, J = 7.3 Hz), 8.03 (t, 1H, J = 7.8 Hz), 7.70 (d, 1H, J = 7.8 Hz), 7.63 (t, 1H, J = 8.2 Hz), 7.61-7.45 (m, 2H), 7.45-7.10 (m, 2H), 6.94 (d, 1H, J = 12.8 Hz), 6.84 (d, 1H, J = 12.8 Hz), 4.25 (t, 2H, J = 7.4 Hz), 3.83 (s, 3H), 3.70-3.48 (m, 10H), 2.78 (t, 2H, J = 5.8 Hz), 2.50 (m, 2H), 2.11 (s, 6H), 1.89-1.44 (m, 11H), 1.21-1.11 (m, 12H)

**Synthesis of Dye 30**

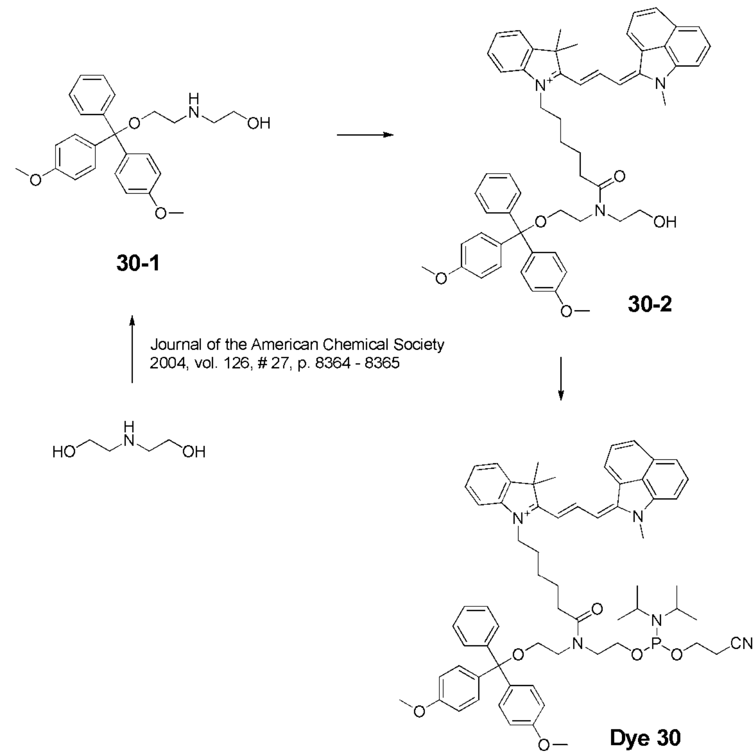


30-1

30-2

Dye 30

Synthesis of Intermediate 30-1

Intermediate 30-1 was synthesized with reference to Journal of the American Chemical Society, 2004, vol. 126, no. 27, pp. 8364-8365.

Synthesis of Intermediate 30-2

In a 100 mL single-necked reactor, Dye 6 (3.0 g, 5.1 mmol), Intermediate 30-1 (2.47 g, 6.1 mmol), HATU (2.9 g, 7.6 mmol), triethylamine (2.1 mL, 15.2 mmol), and dimethylformamide (30 mL) were added and stirred at room temperature for 1 hour. After concentration, water was added to the reactor, and the mixture was vigorously stirred and extracted with dichloromethane. Sodium sulfate was added, stirred for 5 minutes, and filtered. The filtrate was concentrated, and Intermediate 30-2 was synthesized by column purification.

Synthesis of Dye 30

In a 25 mL single-necked reactor, Intermediate 30-2 (2.4 g, 2.0 mmol), 2-cyanoethylN,N'-diisopropylchlorophosphoramidite (0.62 g, 4.9 mmol), triethylamine (0.6 mL, 4.1 mmol), and dichloromethane (25 mL) were added and stirred at room temperature for 1 hour. Water was added to the reactor, and the mixture was vigorously stirred and extracted with dichloromethane. Sodium sulfate was added, stirred for 5 minutes, and filtered. The filtrate was concentrated, and Dye 30 was synthesized by column purification (1.1 g).

$^1$H-NMR of the obtained Dye 30 is as follows:

$^1$H-NMR (400 MHz, DMSO-$d_6$) δ 8.76 (t, 1H, J = 12.8 Hz), 8.52 (d, 1H, J = 7.8 Hz), 8.34 (d, 1H, J = 7.8 Hz), 8.28 (d, 1H, J = 7.4 Hz), 8.01 (t, 1H, J = 7.2 Hz), 7.71-7.63 (m, 2H), 7.61-7.45 (m, 2H), 7.45-7.10 (m, 11H), 6.94 (d, 1H, J = 12.8 Hz), 6.85-6.82 (m, 5H), 4.24 (t, 2H, J = 7.4 Hz), 3.84 (s, 3H), 3.77 (s, 6H), 3.59-3.41 (m, 12H), 2.76 (t, 2H, J = 6.2 Hz), 2.51 (m, 2H), 2.11 (s, 6H), 1.86-1.44 (m, 6H), 1.23-1.14 (m, 12H)

**Synthesis of Dye 31**

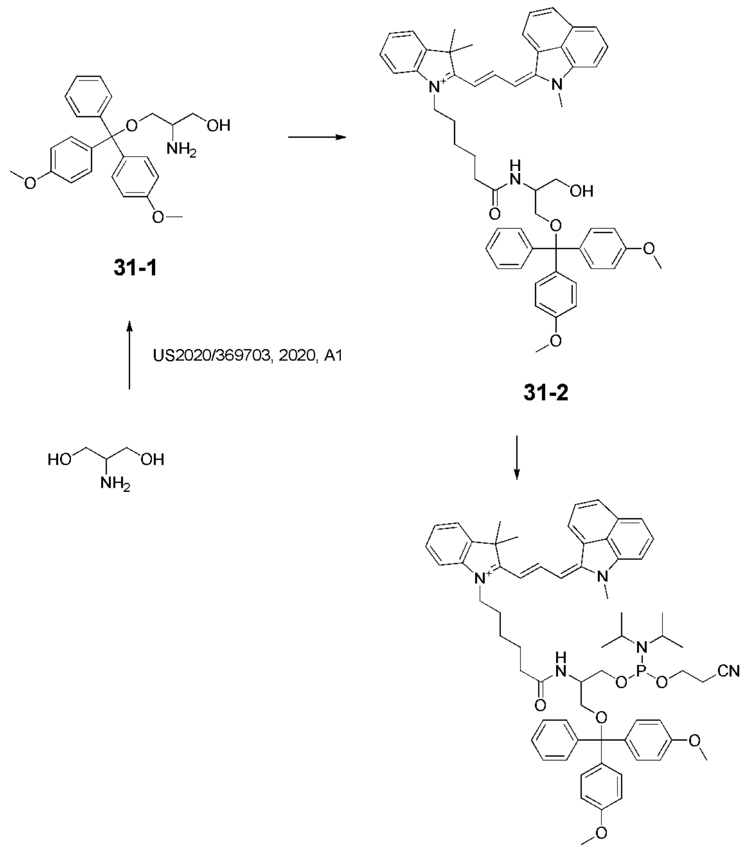


31-1

31-2

Dye 31

Synthesis of Intermediate 31-1

Intermediate 31-1 was synthesized with reference to US2020/369703, 2020, A1.

Synthesis of Intermediate 31-2

In a 100 mL single-necked reactor, Dye 6 (4.0 g, 6.8 mmol), Intermediate 31-1 (3.2 g, 8.1 mmol), HATU (3.9 g, 10.1 mmol), triethylamine (2.8 mL, 20.3 mmol), and dimethylformamide (40 mL) were added and stirred at room temperature for 1 hour. After concentration, water was added to the reactor, and the mixture was vigorously stirred and extracted with dichloromethane. Sodium sulfate was added, stirred for 5 minutes, and filtered. The filtrate was concentrated, and Intermediate 31-2 was synthesized by column purification.

Synthesis of Dye 31

In a 25 mL single-necked reactor, Intermediate 31-2 (2.5 g, 2.6 mmol), 2-cyanoethyl N,N'-diisopropylchlorophosphoramidite (0.8 g, 3.4 mmol), triethylamine (0.7 mL, 5.2 mmol), and dichloromethane (25 mL) were added and stirred at room temperature for 1 hour. Water was added to the reactor, and the mixture was vigorously stirred and extracted with dichloromethane. Sodium sulfate was added, stirred for 5 minutes, and filtered. The filtrate was concentrated, and Dye 31 was synthesized by column purification (1.3 g).

$^1$H-NMR of the obtained Dye 31 is as follows:
$^1$H-NMR (400 MHz, DMSO-$d_6$) $\delta$ 8.77 (t, 1H, J = 12.8 Hz), 8.50 (d, 1H, J = 7.6 Hz), 8.36 (d, 1H, J = 8.0 Hz), 8.25 (d, 1H, J = 7.2 Hz), 8.00 (t, 1H, J = 7.2 Hz), 7.72-7.61 (m, 2H), 7.59-7.45 (m, 2H), 7.44-7.10 (m, 11H), 6.97 (d, 1H, J = 13.2 Hz), 6.87-6.80 (m, 5H), 4.22 (t, 2H, J = 7.4 Hz), 3.83 (s, 3H), 3.75 (s, 6H), 3.55-3.40 (m, 8H), 2.76 (t, 2H, J = 6.0 Hz), 2.50 (m, 2H), 2.13 (s, 6H), 1.90-1.40 (m, 6H), 1.21-1.11 (m, 12H)

**Synthesis of Dye 32**

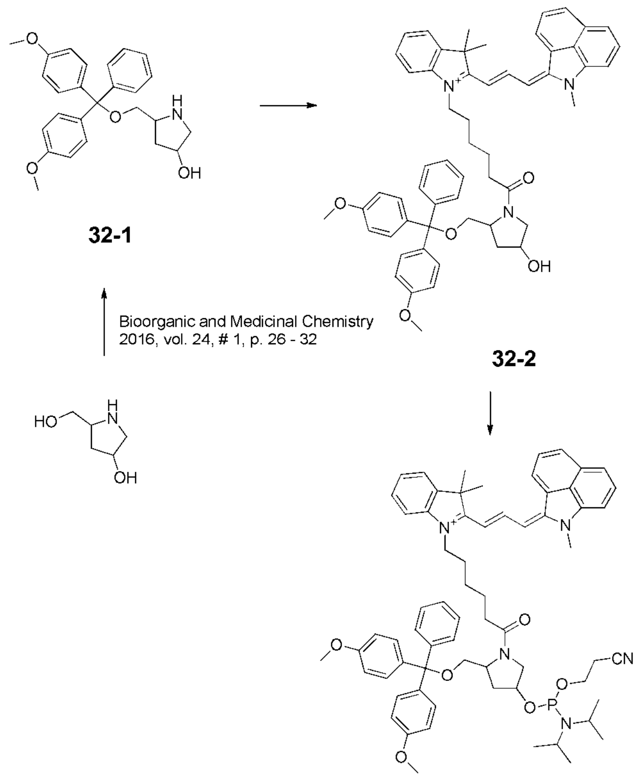


32-1

32-2

Dye 32

Synthesis of Intermediate 32-1

Intermediate 32-1 was synthesized with reference to Bioorganic and Medicinal Chemistry, 2016, vol. 24, no. 1, pp. 26-32.

Synthesis of Intermediate 32-2

In a 100 mL single-necked reactor, Dye 6 (4.0 g, 6.8 mmol), Intermediate 32-1 (3.4 g, 8.1 mmol), HATU (3.9 g, 10.1 mmol), triethylamine (2.8 mL, 20.3 mmol), and dimethylformamide (40 mL) were added and stirred at room temperature for 1 hour. After concentration, water was added to the reactor, and the mixture was vigorously stirred and extracted with dichloromethane. Sodium sulfate was added, stirred for 5 minutes, and filtered. The filtrate was concentrated, and Intermediate 32-2 was synthesized by column purification.

Synthesis of Dye 32

In a 25 mL single-necked reactor, Intermediate 32-2 (2.7 g, 2.7 mmol), 2-cyanoethyl N,N'-diisopropylchlorophosphoramidite (0.84 g, 3.5 mmol), triethylamine (0.8 mL, 5.4 mmol), and dichloromethane (30 mL) were added and stirred at room temperature for 1 hour. Water was added to the reactor, and the mixture was vigorously stirred and extracted with dichloromethane. Sodium sulfate was added, stirred for 5 minutes, and filtered. The filtrate was concentrated, and Dye 32 was synthesized by column purification (1.7 g).

$^{1}$H-NMR of the obtained Dye 32 is as follows:

$^{1}$H-NMR (400 MHz, DMSO-$d_6$) δ 8.77 (m, 1H), 8.49 (d, 1H, J = 7.6 Hz), 8.35 (d, 1H, J = 7.8 Hz), 8.27 (d, 1H, J = 7.6 Hz), 7.98 (t, 1H, J = 7.6 Hz), 7.70-7.45 (m, 4H), 7.44-7.09 (m, 11H), 6.95 (d, 1H, J = 13.2 Hz), 6.89-6.80 (m, 5H), 4.56 (m,1H), 4.22 (t, 2H, J = 7.4 Hz), 3.85 (s, 3H), 3.74 (s, 6H), 3.55-3.32 (m, 10H), 2.76 (t, 2H, J = 6.0 Hz), 2.50 (m, 2H), 2.13 (s, 6H), 1.90-1.40 (m, 8H), 1.21-1.11 (m, 12H)

**Synthesis of Dye 33**

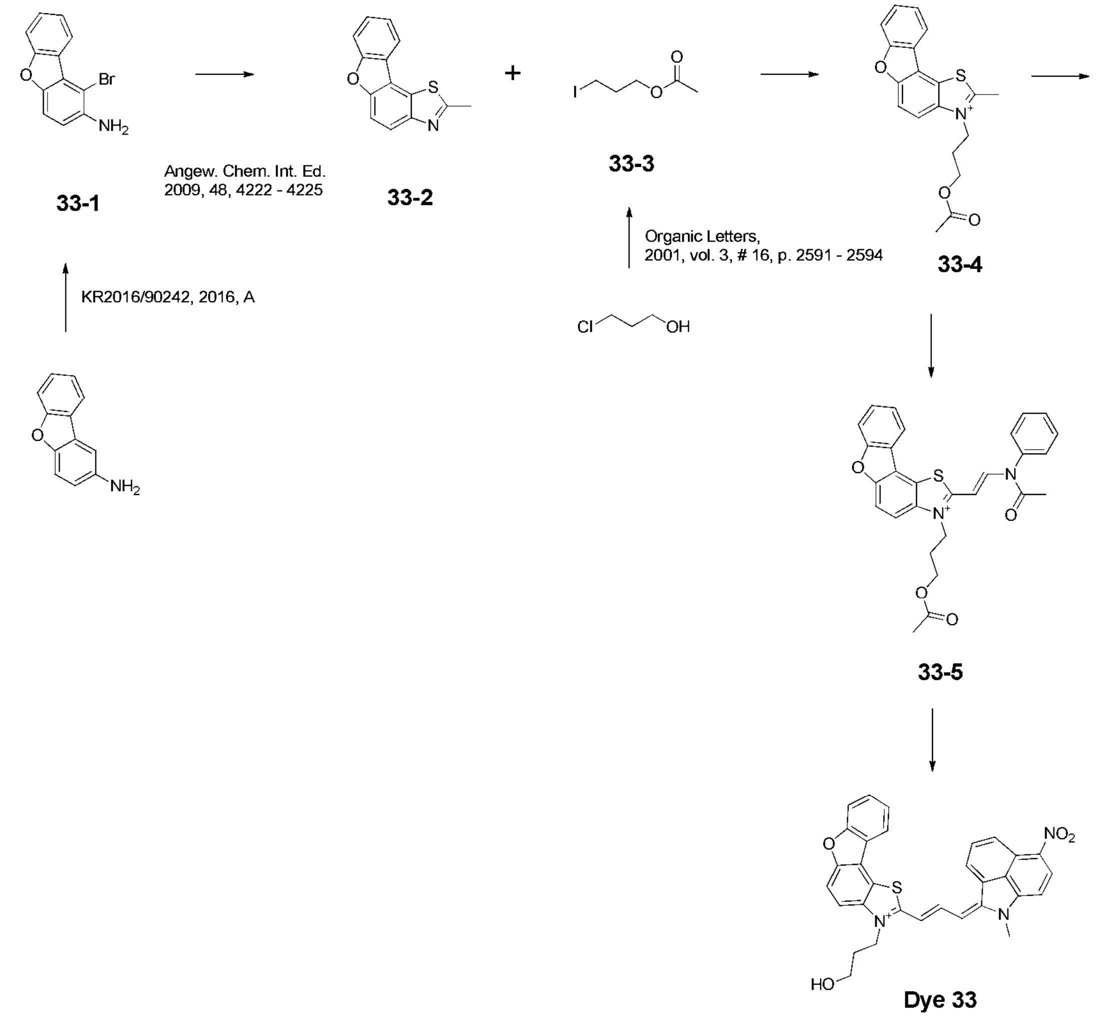


Synthesis of Intermediate 33-1

Intermediate 33-1 was synthesized with reference to KR2016/90242, 2016, A.

Synthesis of Intermediate 33-2

Intermediate 33-2 was synthesized from Intermediate 33-1 by referring to the method described in Angewandte Chemie International Edition, 2009, vol. 48, pp. 4222-4225.

Synthesis of Intermediate 33-3

Intermediate 33-3 was synthesized with reference to Organic Letters, 2001, vol. 3, no. 16, pp. 2591-2594.

Synthesis of Intermediate 33-4

In a 250 mL single-necked reactor, Intermediate 33-2 (12 g, 50.2 mmol), Intermediate 33-3 (17.2 g, 75.3 mmol), and acetonitrile (120 mL) were added and refluxed at room temperature for 12 hours. After concentration, Intermediate 33-4 was synthesized by column purification.

Synthesis of Intermediate 33-5

In a 500 mL single-necked reactor, Intermediate 33-4 (18.0 g, 39 mmol), N,N-diphenylformamidine (8.3 g, 42 mmol), and acetic anhydride (200 mL) were added and stirred at 110°C for 1 hour. After cooling, the reaction solution was poured into ethyl acetate (500 mL) and stirred vigorously. The resulting solid was filtered to synthesize Intermediate 33-5.

Synthesis of Dye 33

In a 100 mL single-necked reactor, Intermediate 33-5 (3.0 g, 4.9 mmol), Intermediate 23-2 (1.7 g, 4.9 mmol), and pyridine (30 mL) were added and stirred at 50°C for 1 hour. After concentration, 6 N hydrochloric acid aqueous solution (20 mL) was added, and the mixture was stirred at room temperature for 12 hours. After concentration, Dye 33 was synthesized by column purification (1.6 g).

$^1$H-NMR of the obtained Dye 33 is as follows:

$^1$H-NMR (400 MHz, DMSO-$d_6$) δ 9.08 (d, 1H, J = 8.2 Hz), 8.71 (t, 1H, J = 12.6 Hz), 8.61 (d, 1H, J = 8.0 Hz), 8.40 (d, 1H, J = 7.8 Hz), 8.18 (d, 1H, J = 8.4 Hz), 7.88-7.40 (m, 7H), 6.97 (d, 1H, J = 12.6 Hz), 6.83 (d, 1H, J = 12.6 Hz), 4.25 (t, 2H, J = 7.6 Hz), 3.86 (m, 2H), 3.97 (s, 3H), 2.09 (m, 2H)

**Synthesis of Dye 34**

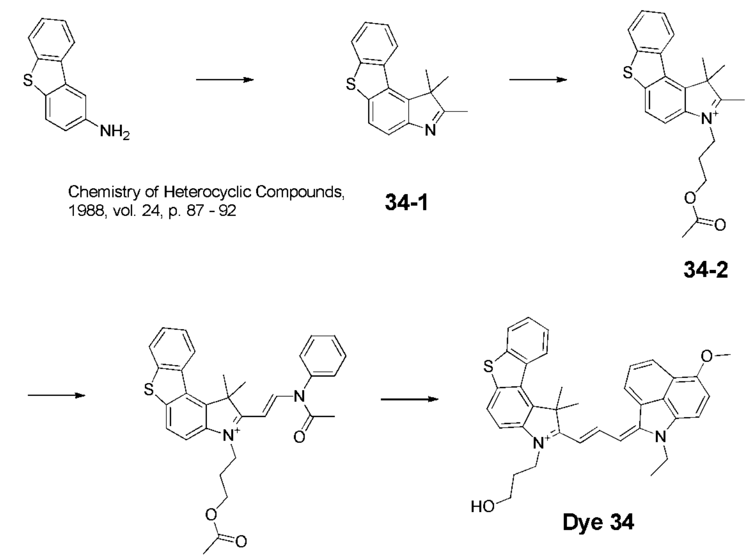


34-1

34-2

34-3

Dye 34

Synthesis of Intermediate 34-1

Intermediate 34-1 was synthesized with reference to Chemistry of Heterocyclic Compounds, 1988, vol. 24, pp. 87-92.

Synthesis of Intermediate 34-2

In a 250 mL single-necked reactor, Intermediate 34-1 (10 g, 37.7 mmol), Intermediate 33-3 (12.9 g, 56.6 mmol), and acetonitrile (100 mL) were added and refluxed at room temperature for 12 hours. After concentration, Intermediate 34-2 was synthesized by column purification.

Synthesis of Intermediate 34-3

In a 250 mL single-necked reactor, Intermediate 34-2 (15.0 g, 30 mmol), N,N-diphenylformamidine (6.6 g, 33 mmol), and acetic anhydride (150 mL) were added and stirred at 110°C for 1 hour. After cooling, the reaction solution was poured into ethyl acetate (500 mL) and stirred vigorously. The resulting solid was filtered to synthesize Intermediate 34-3.

Synthesis of Dye 34

In a 100 mL single-necked reactor, Intermediate 34-3 (3.0 g, 4.7 mmol), Intermediate 25-1 (1.7 g, 4.7 mmol), and pyridine (30 mL) were added and stirred at 50°C for 1 hour. After concentration, 6 N hydrochloric acid aqueous solution (20 mL) was added, and the mixture was stirred at room temperature for 12 hours. After concentration, Dye 34 was

synthesized by column purification (1.8 g).

$^1$H-NMR of the obtained Dye 34 is as follows:

$^1$H-NMR (400 MHz, DMSO-$d_6$) δ 8.62 (t, 1H, J = 12.8 Hz), 8.60 (d, 1H, J = 8.0 Hz), 8.40 (d, 1H, J = 7.8 Hz), 8.23-8.01 (m, 2H) 7.93-7.40 (m, 8H), 6.67 (m, 2H), 4.25 (t, 2H, J = 7.6 Hz), 4.22 (q, 2H, J = 7.2 Hz), 3.86 (m, 2H), 2.09 (m, 2H), 1.18 (t, 2H, J = 7.2 Hz)

**Synthesis of Dye 36**

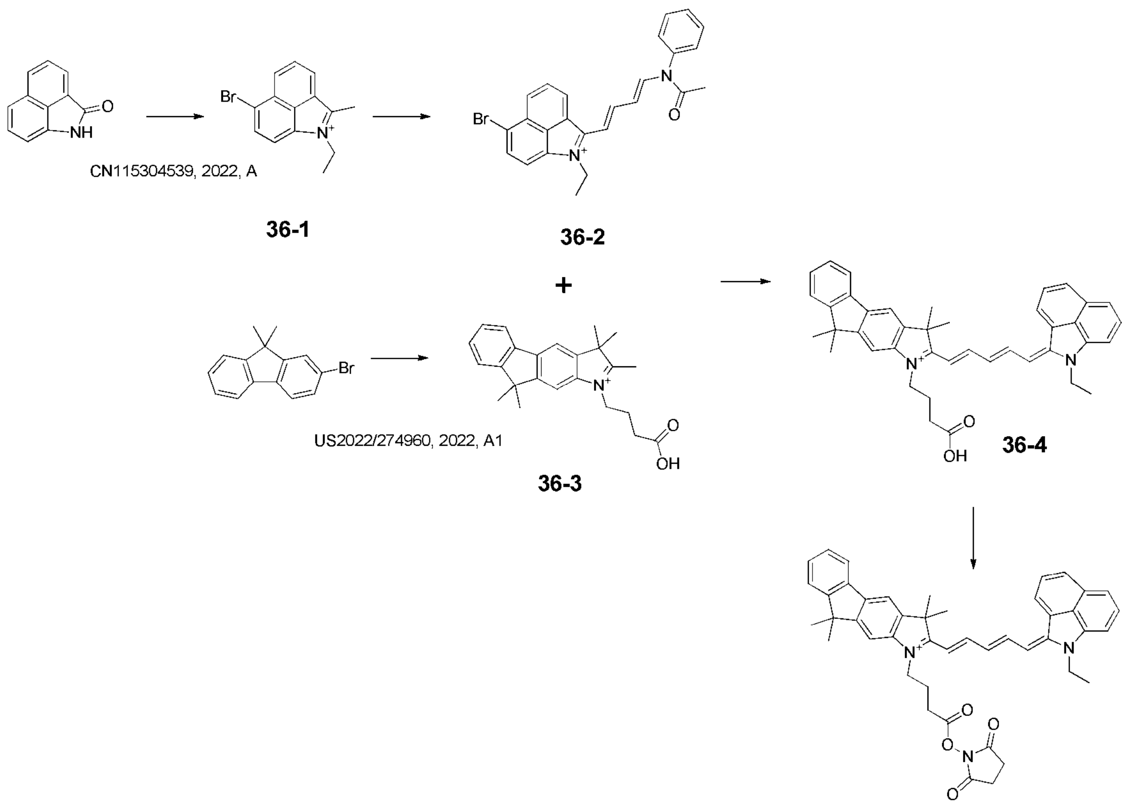


36-1

36-2

+

36-3

36-4

Dye 36

Synthesis of Intermediate 36-1

Intermediate 36-1 was synthesized with reference to CN115304539, 2022, A.

Synthesis of Intermediate 36-2

In a 250 mL single-necked reactor, Intermediate 36-1 (10 g, 25 mmol), malonaldehyde dianilide hydrochloride (7.1 g, 27 mmol), and acetic anhydride (100 mL) were added and refluxed for 1 hour. After cooling, ethyl acetate (500 mL) was added to the reactor and stirred for 5 minutes. The resulting solid was filtered to synthesize Intermediate 36-2.

Synthesis of Intermediate 36-3

4-Bromobutyric acid was used to synthesize Intermediate 36-3 with reference to US2022/274960, 2022, A1.

Synthesis of Intermediate 36-4

In a 100 mL single-necked reactor, Intermediate 36-2 (4.0 g, 7.0 mmol), Intermediate 36-3 (3.1 g, 7.0 mmol), and pyridine (40 mL) were added and stirred at 50°C for 1 hour. After concentration, Intermediate 36-4 was synthesized by column purification.

Synthesis of Dye 36

In a 100 mL single-necked reactor, Intermediate 36-4 (2.0 g, 2.8 mmol), HSTU (O-(N-Succinimidyl)-N,N,N',N'-tetramethyluronium hexafluorophosphate) (1.6 g, 3.6 mmol), triethylamine (1.2 mL, 8.3 mmol), and dimethylformamide (20 mL) were added and stirred at room temperature for 30 minutes. After concentration, Dye 36 was synthesized by column purification (1.7 g).

$^1$H-NMR of the obtained Dye 36 is as follows:

$^1$H-NMR (400 MHz, DMSO-$d_6$) $\delta$ 8.70 (t, 1H, J = 12.8 Hz), 8.54 (d, 1H, J = 8.0 Hz), 8.38 (d, 1H, J = 7.8 Hz), 8.23-7.40 (m, 10H), 6.76 (m, 2H), 4.25 (t, 2H, J = 7.2 Hz), 4.22 (q, 2H, J = 7.2 Hz), 2.85 (s, 4H), 2.50 (t, 2H, J = 7.6 Hz), 2.09 (m, 2H), 1.76 (s, 6H), 1.73 (s, 6H) 1.21 (t, 2H, J = 7.2 Hz)

**Synthesis of Dye 38**

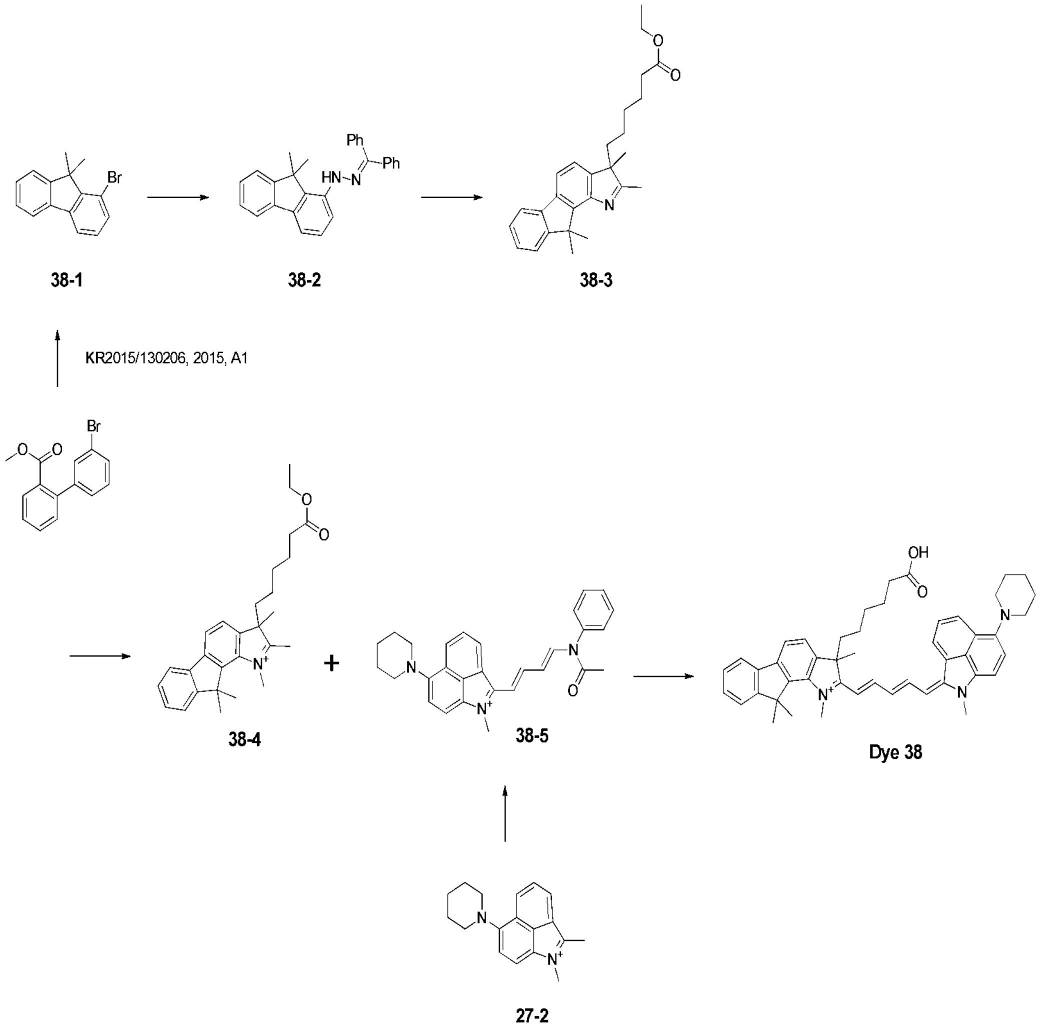


38-1 38-2 38-3 38-4 38-5 Dye 38 27-2

Synthesis of Intermediate 38-1

Intermediate 38-1 was synthesized with reference to KR2015/130206, 2015, Al.

Synthesis of Intermediate 38-2

In a 2 L four-necked reactor, Intermediate 38-1 (70 g, 0.256 mol), benzophenone hydrazone (60 g, 0.229 mol), $Pd_2(dba)_3$ (9.4 g, 0.010 mol), BINAP (6.4 g, 0.010 mol), sodium tert-butoxide (35 g, 0.359 mol), and toluene (700 mL) were added and stirred at 100°C for 12 hours. Celite filtration was performed while hot to remove solids. The filtrate was

concentrated, and Intermediate 38-2 was synthesized by column purification.

Synthesis of Intermediate 38-3

In a 1 L single-necked reactor, Intermediate 38-2 (80 g, 0.206 mol), 7-methyl-8-oxo-nonanoic acid (57.6 g, 0.309 mol), concentrated hydrochloric acid (160 mL), and ethanol (640 mL) were added and refluxed for 12 hours. After concentration, water (500 mL) was added to the reactor, followed by extraction with ethyl acetate (500 mL × 2). Anhydrous magnesium sulfate was added to the organic layer and stirred for 5 minutes, and the solids were filtered off. The filtrate was concentrated, and Intermediate 38-3 was synthesized by column purification.

Synthesis of Intermediate 38-4

In a 250 mL single-necked reactor, Intermediate 38-3 (10 g, 24.8 mmol), methyl iodide (5.3 g, 37.2 mmol), and acetonitrile (100 mL) were added and refluxed for 24 hours. After cooling, ethyl acetate (200 mL) was added and stirred for 10 minutes. The resulting solid was filtered to synthesize Intermediate 38-4.

Synthesis of Intermediate 38-5

In a 250 mL single-necked reactor, Intermediate 27-2 (8 g, 20 mmol), malonaldehyde dianilide hydrochloride (5.8 g, 22 mmol), and acetic anhydride (80 mL) were added and refluxed for 1 hour. After cooling, ethyl acetate (300 mL) was added to the reactor and stirred for 5 minutes. The resulting solid was filtered to synthesize Intermediate 38-5.

Synthesis of Dye 38

In a 100 mL single-necked reactor, Intermediate 38-4 (2.0 g, 3.7 mmol), Intermediate 38-5 (2.1 g, 3.7 mmol), and pyridine (20 mL) were added and stirred at 50°C for 1 hour. After concentration, Dye 38 was synthesized by column purification (0.7 g).

$^1$H-NMR of the obtained Dye 38 is as follows:

$^1$H-NMR (400 MHz, DMSO-$d_6$) $\delta$ 8.52-8.50 (m, 3H), 8.38 (d, 1H, J = 7.8 Hz), 8.20-7.22 (m, 9H), 6.87 (t, 1H, J = 12.6 Hz), 6.35 (d, 2H, J = 13.6 Hz), 3.98 (s, 3H), 3.80 (s, 3H), 3.44-3.40 (m, 4H), 2.54 (t, 2H, J = 7.6 Hz), 2.10-1.75 (m, 21H), 1.02-0.95 (m, 1H), 0.69-0.64 (m, 1H)

**Synthesis of Dye 41**

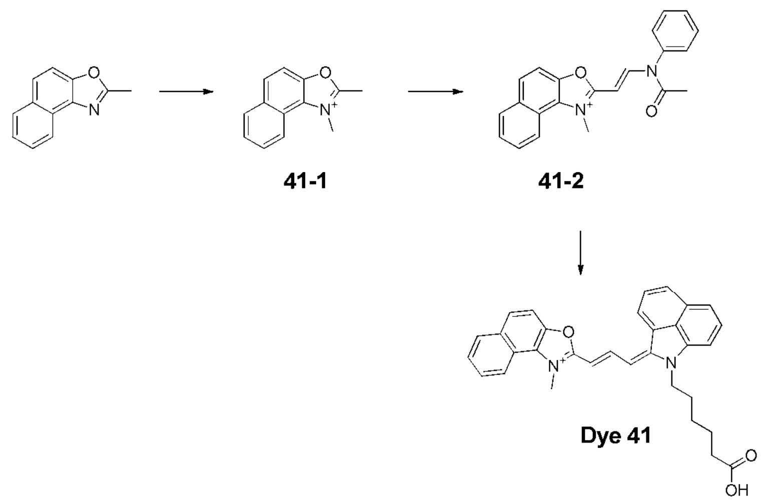

41-1

41-2

Dye 41

Synthesis of Intermediate 41-1

In a 250 mL single-necked reactor, 2-methylnaphtho[1,2-d]oxazole (10 g, 54.6 mmol), methyl iodide (11.6 g, 81.9 mmol), and acetonitrile (100 mL) were added and refluxed at room temperature for 12 hours. After concentration, Intermediate 41-1 was synthesized by column purification.

Synthesis of Intermediate 41-2

In a 500 mL single-necked reactor, Intermediate 41-1 (12.0 g, 37 mmol), N,N-diphenylformamidine (8.0 g, 41 mmol), and acetic anhydride (120 mL) were added and stirred at 110°C for 1 hour. After cooling, the reaction mixture was poured into ethyl acetate (600 mL) and stirred vigorously. The resulting solid was filtered to synthesize Intermediate 41-2.

Synthesis of Dye 41

In a 100 mL single-necked reactor, Intermediate 41-2 (4.0 g, 8.5 mmol), Intermediate 1-2 (3.5 g, 8.5 mmol), and pyridine (40 mL) were added and stirred at 50°C for 1 hour. After concentration, Dye 41 was synthesized by column purification (1.4 g).

$^1$H-NMR of the obtained Dye 41 is as follows:

$^1$H-NMR (400 MHz, DMSO-$d_6$) δ 8.60 (t, 1H, J = 12.6 Hz), 8.51 (d, 1H, J = 8.0 Hz), 8.40 (d, 1H, J = 7.8 Hz), 8.20-8.18 (m, 3H), 7.93-7.90 (m, 3H), 7.62-7.34 (m, 4H), 6.97 (d, 1H, J = 12.6 Hz), 6.83 (d, 1H, J = 12.6 Hz), 4.25 (t, 2H, J = 7.6 Hz), 3.87 (s, 3H), 2.54 (m, 2H), 2.08-1.73 (m, 6H)

**Synthesis of Dye 42**

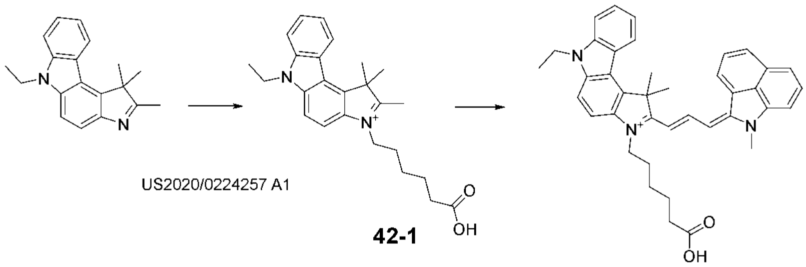


42-1

Dye 42

Synthesis of Intermediate 42-1

Intermediate 42-1 was synthesized with reference to US2020/0224257 A1.

Synthesis of Dye 42

In a 100 mL single-necked reactor, Intermediate 42-1 (3.0 g, 5.8 mmol), Intermediate 7-1 (2.6 g, 5.8 mmol), and pyridine (30 mL) were added and stirred at 50°C for 1 hour. After concentration, Dye 42 was synthesized by column purification (1.7 g).

$^1$H-NMR of the obtained Dye 42 is as follows:

$^1$H-NMR (400 MHz, DMSO-$d_6$) δ 8.70 (t, 1H, J = 13.2 Hz), 8.52 (d, 1H, J = 8.0 Hz), 8.40 (d, 1H, J = 7.8 Hz), 8.22 (d, 1H, J = 8.4 Hz), 8.03 (t, 1H, J = 7.6 Hz), 7.86-7.40 (m, 8H), 7.00 (d, 1H, J = 14.0 Hz), 6.81 (d, 1H, J = 12.8 Hz), 4.58 (q, 2H, J = 6.8 Hz), 4.44 (t, 2H, J = 7.2 Hz), 3.76 (s, 3H), 2.25 (t, 2H, J = 7..2 Hz), 2.11 (s, 6H), 1.93-1.50 (m, 6H), 1.35 (t, 3H, J = 7.2 Hz)

**Synthesis of Dye 43**

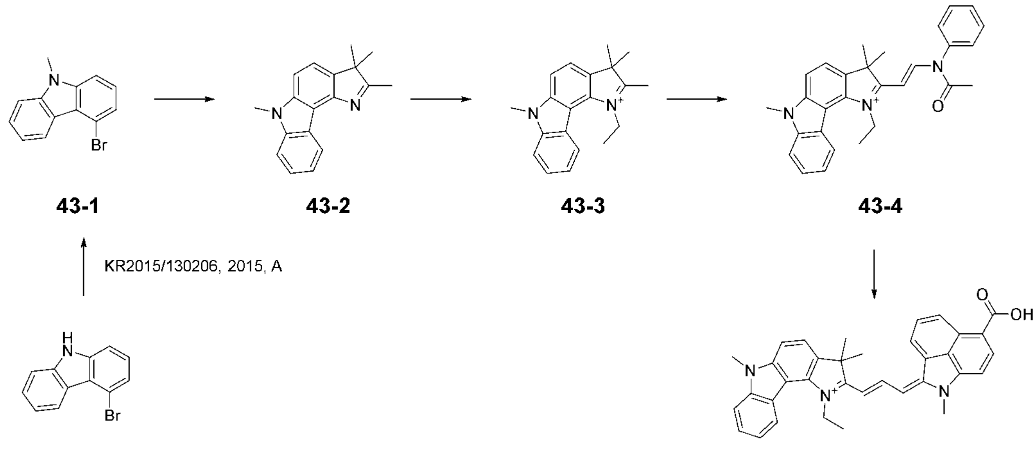


43-1 43-2 43-3 43-4

Dye 43

Synthesis of Intermediate 43-1

Intermediate 43-1 was synthesized with reference to KR2015/130206 A1.

Synthesis of Intermediate 43-2

Intermediate 43-2 was synthesized with reference to the synthesis methods of Intermediate 38-2 and Intermediate 38-3.

Synthesis of Intermediate 43-3

In a 250 mL single-necked reactor, Intermediate 43-2 (10 g, 38.1 mmol), iodoethane (8.9 g, 57.2 mmol), and acetonitrile (100 mL) were added and stirred under reflux for 24 hours. After cooling, ethyl acetate (200 mL) was added and stirred for 10 minutes. The resulting solid was filtered to synthesize Intermediate 43-3.

Synthesis of Intermediate 43-4

In a 250 mL single-necked reactor, Intermediate 43-3 (6.0 g, 14 mmol), N,N-diphenylformamidine (3.1 g, 16 mmol), and acetic anhydride (60 mL) were added and stirred at 110°C for 1 hour. After cooling, ethyl acetate (200 mL) was added and vigorously stirred. The resulting solid was filtered to synthesize Intermediate 43-4.

Synthesis of Dye 43

In a 100 mL single-necked reactor, Intermediate 43-4 (3.0 g, 5.3 mmol), Intermediate 9-4 (1.9 g, 5.3 mmol), and pyridine (30 mL) were added and stirred at 50°C for 1 hour. After concentration, Dye 43 was synthesized by column purification (0.4 g).

$^1$H-NMR of the obtained Dye 43 is as follows:
$^1$H-NMR (400 MHz, DMSO-$d_6$) δ 9.03 (d, 1H, J = 8.0 Hz), 8.70 (t, 1H, J = 12.6 Hz), 8.51 (d, 1H, J = 8.0 Hz), 8.22 (d, 1H, J = 8.4 Hz), 7.98 (m, 1H), 7.86-7.40 (m, 7H), 7.00 (d, 1H, J = 14.0 Hz), 6.81 (d, 1H, J = 12.6 Hz), 4.55 (q, 2H, J = 7.2 Hz), 3.87 (s,3H), 3.76 (s, 3H), 2.10 (s, 6H), 1.35 (t, 3H, J = 7.2 Hz)

**Synthesis of Dye 47**

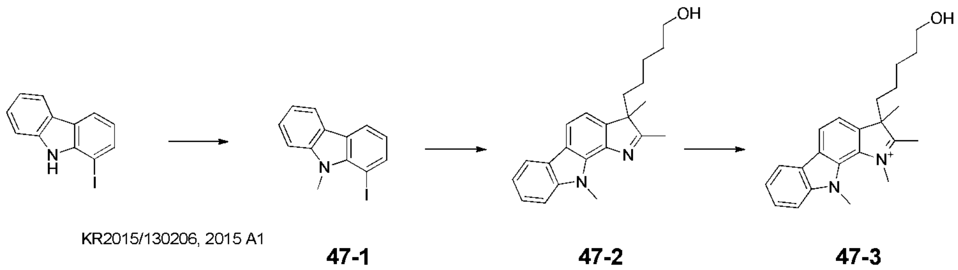


47-1 47-2 47-3

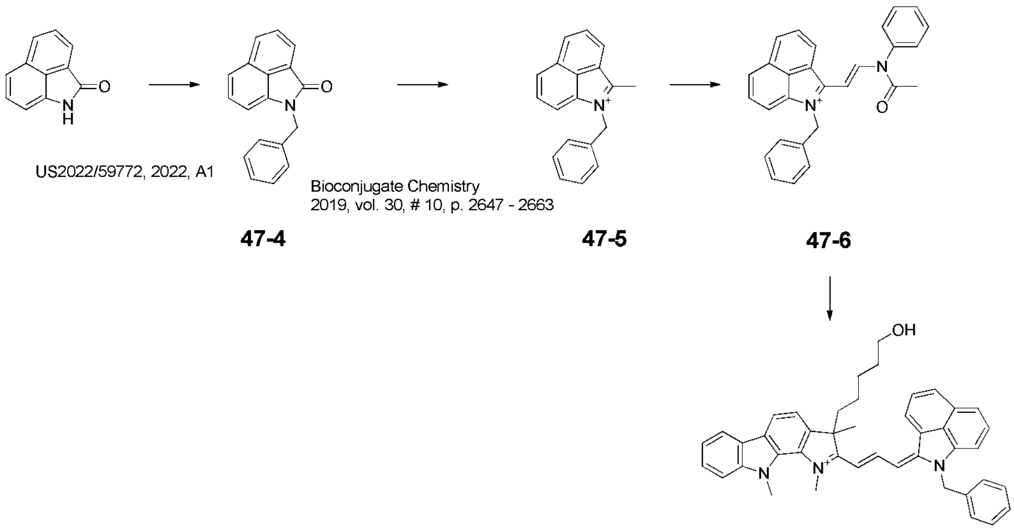


47-4 47-5 47-6

Dye 47

Synthesis of Intermediate 47-1

Intermediate 47-1 was synthesized with reference to KR2015/130206 A1.

Synthesis of Intermediate 47-2

Intermediate 47-2 was synthesized with reference to the synthesis methods of Intermediate 38-2 and Intermediate 38-3.

Synthesis of Intermediate 47-3

In a 250 mL single-necked reactor, Intermediate 47-2 (10 g, 30 mmol), iodomethane (6.4 g, 45 mmol), and acetonitrile (100 mL) were added and stirred under reflux for 24 hours. After cooling, ethyl acetate (100 mL) was added and stirred for 10 minutes. The resulting solid was filtered to synthesize Intermediate 47-3.

Synthesis of Intermediate 47-4

Intermediate 47-4 was synthesized with reference to US2022/59772 A1.

Synthesis of Intermediate 47-5

Intermediate 47-5 was synthesized from Intermediate 47-4 by referring to the method described in Bioconjugate Chemistry, 2019, vol. 30, no. 10, pp. 2647-2663.

Synthesis of Intermediate 47-6

In a 250 mL single-necked reactor, Intermediate 47-5 (5.0 g, 13 mmol), N,N-diphenylformamidine (2.8 g, 14

mmol), and acetic anhydride (50 mL) were added and stirred at 110°C for 1 hour. After cooling, ethyl acetate (100 mL) was added and vigorously stirred. The resulting solid was filtered to synthesize Intermediate 47-6.

Synthesis of Dye 47

In a 100 mL single-necked reactor, Intermediate 47-3 (2.7 g, 5.7 mmol), Intermediate 47-6 (3.0 g, 5.7 mmol), and pyridine (30 mL) were added and stirred at 50°C for 1 hour. After concentration, Dye 47 was synthesized by column purification (1.1 g).

$^1$H-NMR of the obtained Dye 47 is as follows:

$^1$H-NMR (400 MHz, DMSO-$d_6$) δ 8.54 (t, 1H, J = 12.6 Hz), 8.49 (d, 1H, J = 8.0 Hz), 8.38 (d, 1H, J = 7.8 Hz), 8.22 (d, 1H, J = 8.0 Hz), 8.02 (d, 1H, J = 7.8 Hz), 7.84-7.38 (m, 13H), 6.81 (m, 2H), 5.87 (s, 2H), 3.97 (s, 3H), 3.86 (m, 2H), 3.80 (s, 3H), 2.10-1.75 (m, 11H), 1.02-0.95 (m, 1H), 0.69-0.64 (m, 1H)

**Synthesis of Dye 48**

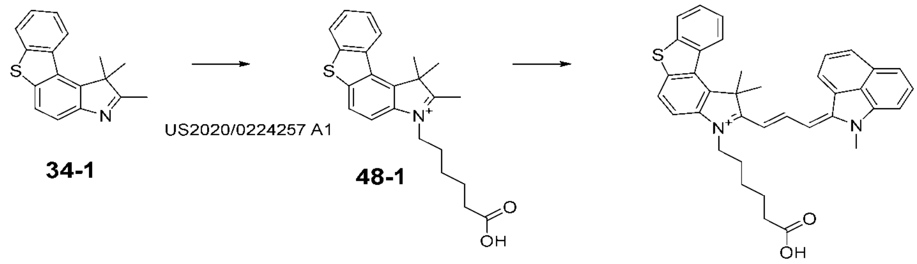


**34-1** **48-1** **Dye 48**

Synthesis of Intermediate 48-1

Intermediate 48-1 was synthesized from Intermediate 34-1 by referring to the method described in US2020/0224257 A1.

Synthesis of Dye 48

In a 100 mL single-necked reactor, Intermediate 48-1 (2.0 g, 3.9 mmol), Intermediate 7-1 (1.8 g, 3.9 mmol), and pyridine (20 mL) were added and stirred at 50°C for 1 hour. After concentration, Dye 48 was synthesized by column purification (0.3 g).

$^1$H-NMR of the obtained Dye 48 is as follows:

$^1$H-NMR (400 MHz, DMSO-$d_6$) δ 8.62 (t, 1H, J = 12.8 Hz), 8.60 (d, 1H, J = 8.0 Hz), 8.40 (d, 1H, J = 7.8 Hz), 8.23-8.01 (m, 2H) 7.93-7.40 (m, 9H), 6.67 (m, 2H), 4.20 (t, 2H, J = 7.6 Hz), 3.77 (s, 3H), 2.66 (t, 2H, J = 7.6 Hz), 2.11 (s, 6H), 1.92-1.67 (m, 6H)

**Synthesis of Dye 55**

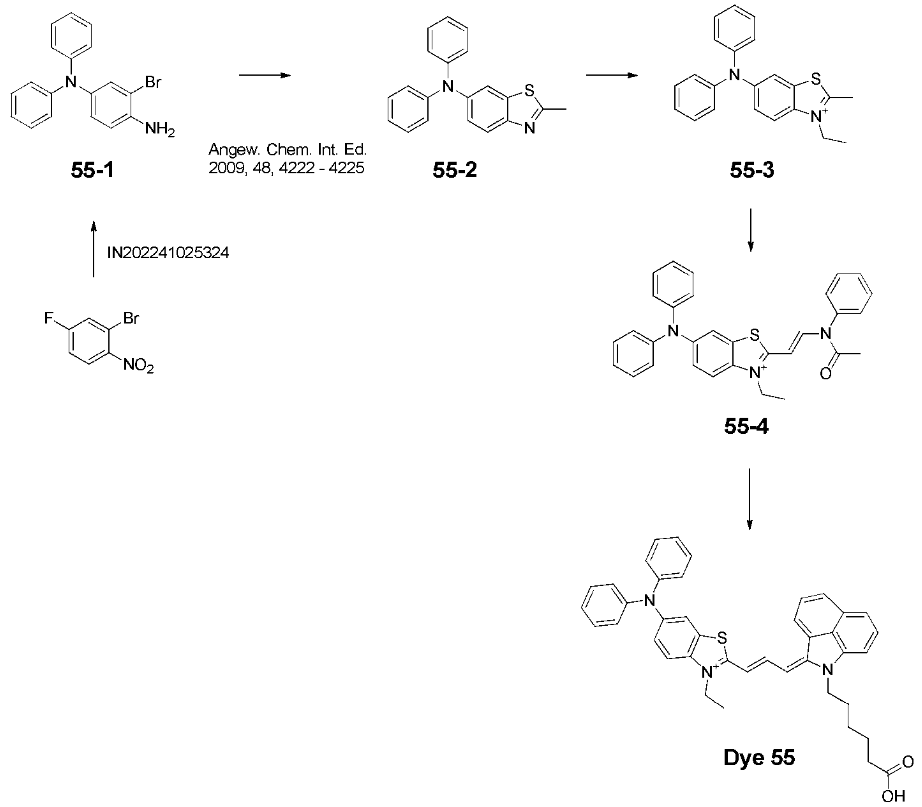


55-1 55-2 55-3 55-4 Dye 55

Synthesis of Intermediate 55-1

Intermediate 55-1 was synthesized with reference to IN202241025324.

Synthesis of Intermediate 55-2

Intermediate 55-2 was synthesized from Intermediate 55-1 by referring to the method described in Angew. Chem. Int. Ed. 2009, 48, 4222-4225.

Synthesis of Intermediate 55-3

In a 250 mL single-necked reactor, Intermediate 55-2 (10 g, 32 mmol), iodoethane (7.4 g, 48 mmol), and acetonitrile (100 mL) were added and stirred under reflux for 24 hours. After cooling, ethyl acetate (100 mL) was added to the reactor and stirred for 10 minutes. The resulting solid was filtered to synthesize Intermediate 55-3.

Synthesis of Intermediate 55-4

In a 250 mL single-necked reactor, Intermediate 55-3 (5.0 g, 11 mmol), N,N-diphenylformamidine (2.3 g, 12 mmol), and acetic anhydride (50 mL) were added and stirred at 110°C for 1 hour. After cooling, ethyl acetate (100 mL) was added to the reactor and stirred vigorously. The resulting solid was filtered to synthesize Intermediate 55-4.

Synthesis of Dye 55

In a 100 mL single-necked reactor, Intermediate 55-4 (2.0 g, 3.2 mmol), Intermediate 1-2 (1.3 g, 3.2 mmol), and pyridine (20 mL) were added and stirred at 50°C for 1 hour. After concentration, Dye 55 was synthesized by column purification (0.2 g).

$^1$H-NMR of the obtained Dye 55 is as follows:
$^1$H-NMR (400 MHz, DMSO-$d_6$) δ 8.64 (t, 1H, J = 12.8 Hz), 8.54 (d, 1H, J = 8.0 Hz), 8.45-8.40 (m, 2H), 8.23-8.01 (m, 2H), 7.85-7.68 (m, 4H), 7.28-6.96 (m, 10H), 6.67 (m, 2H), 4.28 (q, 2H, J = 7.2 Hz), 4.20 (t, 2H, J = 7.6 Hz), 2.57 (t, 2H, J = 7.2 Hz), 1.98-1.69 (m, 6H), 1.49 (t, 3H, J = 7.2 Hz)

**Synthesis of Dye 57**

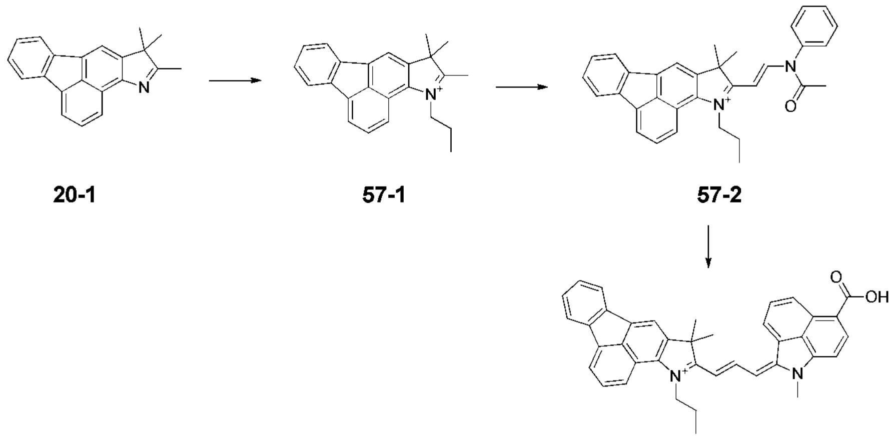

20-1 57-1 57-2

Dye 57

Synthesis of Intermediate 57-1

In a 250 mL single-necked reactor, Intermediate 20-1 (8.0 g, 28 mmol), iodopropane (7.2 g, 42 mmol), and acetonitrile (80 mL) were added and stirred under reflux for 24 hours. After cooling, ethyl acetate (100 mL) was added to the reactor and stirred for 10 minutes. The resulting solid was filtered to synthesize Intermediate 57-1.

Synthesis of Intermediate 57-2

In a 250 mL single-necked reactor, Intermediate 57-1 (6.0 g, 13 mmol), N,N-diphenylformamidine (2.9 g, 15 mmol), and acetic anhydride (50 mL) were added and stirred at 110°C for 1 hour. After cooling, ethyl acetate (100 mL) was added to the reactor and stirred vigorously. The resulting solid was filtered to synthesize Intermediate 57-2.

Synthesis of Dye 57

In a 100 mL single-necked reactor, Intermediate 57-2 (3.0 g, 5.0 mmol), Intermediate 9-4 (1.8 g, 5.0 mmol), and pyridine (30 mL) were added and stirred at 50°C for 1 hour. After concentration, Dye 57 was synthesized by column purification (1.4 g).

$^1$H-NMR of the obtained Dye 57 is as follows:

$^1$H-NMR (400 MHz, DMSO-$d_6$) $\delta$ 9.03 (d, 1H, J = 8.0 Hz), 8.60-8.52 (m, 3H), 8.38 (d, 1H, J = 8.0 Hz), 8.31 (d, 1H), 8.22 (d, 1H, J = 8.0 Hz), 8.15-8.10 (m, 2H), 8.03 (t, 1H, J = 7.2 Hz), 7.90-7.62 (m, 2H), 7.54-7.40 (m, 2H), 6.83 (m, 2H), 4.28 (t, 2H, J = 7.2 Hz), 3.85 (s, 3H), 1.96 (m, 2H), 2.10 (s, 6H), 1.35 (t, 3H, J = 7.6 Hz)

**Synthesis of Dye 59**

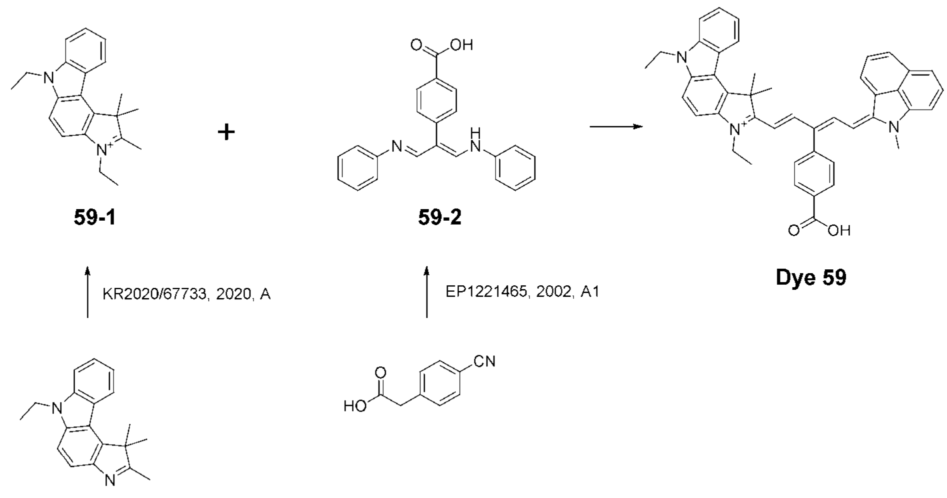


59-1

59-2

Dye 59

Synthesis of Intermediate 59-1

Intermediate 59-1 was synthesized with reference to KR2020/67733, 2020, A.

Synthesis of Intermediate 59-2

Intermediate 59-2 was synthesized with reference to EP1221465, 2002, A1.

Synthesis of Dye 59

In a 100 mL single-necked reactor, Intermediate 59-1 (4.3 g, 10.0 mmol), Intermediate 6-2 (3.0 g, 10.0 mmol), Intermediate 59-2 (1.9 g, 5.0 mmol), and pyridine (30 mL) were added and stirred at 50°C for 1 hour. After concentration, Dye 59 was synthesized by column purification (0.4 g).

$^1$H-NMR of the obtained Dye 59 is as follows:

$^1$H-NMR (400 MHz, DMSO-$d_6$) δ 8.44 (d, 2H, J = 12.8 Hz), 8.49 (d, 1H, J = 8.0 Hz), 8.38 (d, 1H, J = 7.8 Hz), 8.22 (d, 1H, J = 8.0 Hz), 8.02 (d, 1H, J = 7.8 Hz), 7.95-7.38 (m, 12H), 6.34 (d, 2H, J = 13.2 Hz), 4.26 (q, 2H, J = 7.8 Hz), 4.02 (q, 2H, J = 7.6 Hz), 3.80 (s, 3H), 2.10 (s, 6H), 1.37 (t, 3H, J = 7.6 Hz), 1.09 (t, 3H, J = 7.8 Hz)

**Synthesis of Dye 60**

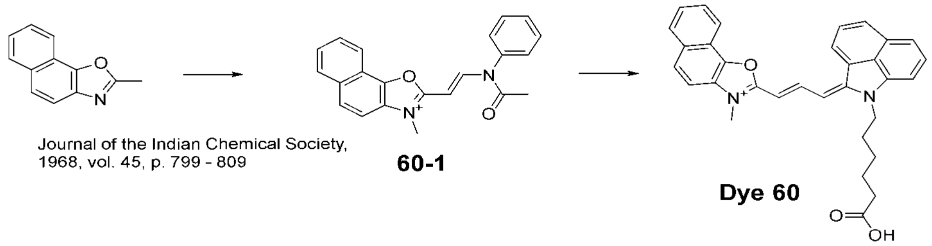


60-1

Dye 60

Synthesis of Intermediate 60-1

Intermediate 60-1 was synthesized with reference to Journal of the Indian Chemical Society, 1968, vol. 45, pp. 799-809.

Synthesis of Dye 60

In a 100 mL single-necked reactor, Intermediate 60-1 (2.0 g, 4.3 mmol), Intermediate 1-2 (1.8 g, 4.3 mmol), and pyridine (20 mL) were added and stirred at 50°C for 1 hour. After concentration, Dye 60 was synthesized by column purification (0.1 g).

$^1$H-NMR of the obtained Dye 60 is as follows:
$^1$H-NMR (400 MHz, DMSO-$d_6$) δ 8.69 (t, 1H, J = 12.8 Hz), 8.50 (d, 1H, J = 7.2 Hz), 8.40 (d, 1H, J = 8.0 Hz), 8.20 (d, 1H, J = 8.2 Hz), 7.88 (t, 1H, J = 7.2 Hz), 7.75-7.53 (m, 4H), 7.47-7.25 (m, 3H), 7.00 (d, 1H, J = 7.0 Hz), 6.88 (d, 1H, J = 13.4 Hz), 6.52 (d, 1H, J = 13.2 Hz), 4.46 (q, 2H, J = 7.6 Hz), 3.97 (s, 3), 2.55 (m, 2H), 2.08-1.72 (m, 6H)

**Synthesis of Dye 61**

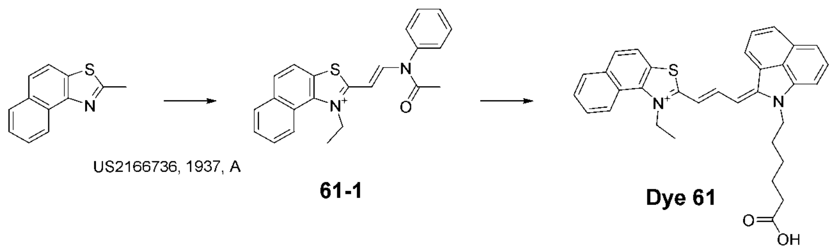


**61-1** **Dye 61**

Synthesis of Intermediate 61-1

Intermediate 61-1 was synthesized with reference to US2166736, 1937, A.

Synthesis of Dye 61

In a 100 mL single-necked reactor, Intermediate 61-1 (4.0 g, 8.0 mmol), Intermediate 1-2 (3.3 g, 8.0 mmol), and pyridine (40 mL) were added and stirred at 50°C for 1 hour. After concentration, Dye 61 was synthesized by column purification (2.3 g).
$^1$H-NMR of the obtained Dye 61 is as follows:
$^1$H-NMR (400 MHz, DMSO-$d_6$) δ 8.73 (t, 1H, J = 13.4 Hz), 8.52 (d, 1H, J = 7.8 Hz), 8.42 (d, 1H, J = 8.2 Hz), 8.22 (d, 1H, J = 8.4 Hz), 8.00 (t, 1H, J = 7.2 Hz), 7.76-7.51 (m, 4H), 7.49-7.25 (m, 3H), 7.01 (d, 1H, J = 7.2 Hz), 6.88 (d, 1H, J = 12.8 Hz), 6.52 (d, 1H, J = 13.2 Hz), 4.51 (q, 2H, J = 7.6 Hz), 4.22 (t, 2H, J = 7.8 Hz), 2.57 (m, 2H), 2.10-1.74 (m, 6H), 1.33 (t, 3H, J = 7.8 Hz)

**Synthesis of Dye 62**

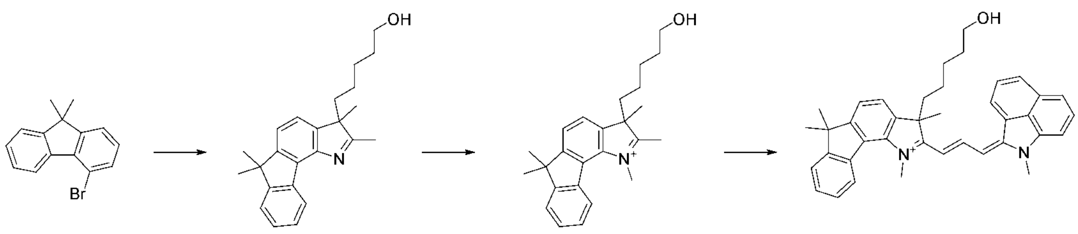

**62-1** **62-2** **Dye 62**

Synthesis of Intermediate 62-1

Intermediate 62-1 was synthesized with reference to the synthesis methods of Intermediate 38-2 and Intermediate 38-3.

Synthesis of Intermediate 62-2

In a 250 mL single-necked reactor, Intermediate 62-1 (6 g, 17 mmol), methyl iodide (3.7 g, 26 mmol), and acetonitrile (60 mL) were added and stirred under reflux for 24 hours. After cooling, ethyl acetate (100 mL) was added and stirred for 10 minutes. The resulting solid was filtered to synthesize Intermediate 62-2.

Synthesis of Dye 62

In a 100 mL single-necked reactor, Intermediate 62-2 (2.0 g, 4.0 mmol), Intermediate 7-1 (1.9 g, 4.0 mmol), and pyridine (20 mL) were added and stirred at 50°C for 1 hour. After concentration, Dye 62 was synthesized by column purification (1.1 g).

$^1$H-NMR of the obtained Dye 62 is as follows:

$^1$H-NMR (400 MHz, DMSO-$d_6$) $\delta$ 8.55 (t, 1H, J = 13.4 Hz), 8.43 (d, 1H, J = 7.80 Hz), 8.32 (d, 1H, J = 7.8 Hz), 8.20 (d, 1H, J = 8.0 Hz), 8.00 (d, 1H, J = 7.2 Hz), 7.86-7.39 (m, 8H), 6.82 (m, 2H), 5.87 (s, 2H), 4.16 (s, 3H), 3.86 (s, 3H), 3.82 (m 2H), 2.10-1.75 (m, 15H), 1.02-0.95 (m, 1H), 0.69-0.64 (m, 1H)

**Synthesis of Dye 63**

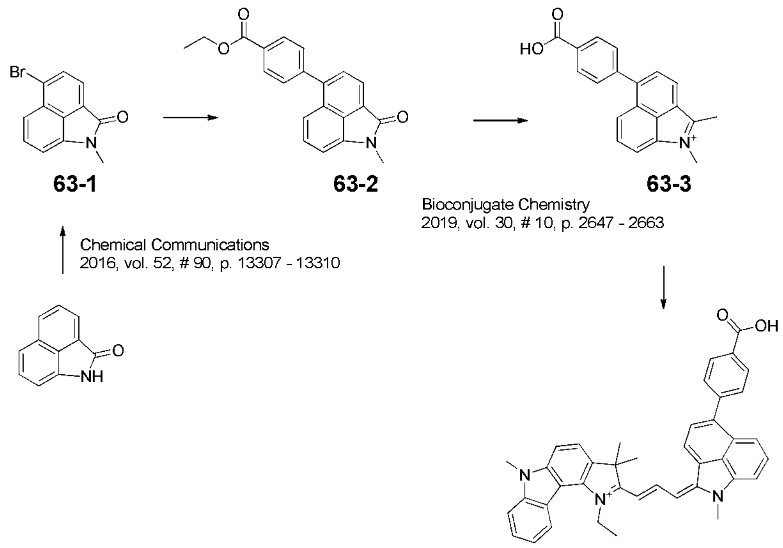


Dye 63

Synthesis of Intermediate 63-1

Intermediate 63-1 was synthesized with reference to Chemical Communications, 2016, vol. 52, no. 90, pp. 13307-13310.

Synthesis of Intermediate 63-2

In a 250 mL three-necked reactor, Intermediate 63-1 (4.75 g, 18.2 mmol), 4-(ethoxycarbonyl)phenylboronic acid (5.0 g, 27.8 mmol), potassium carbonate (5.0 g, 36.4 mmol), Pd($PPh_3$)$_4$ (0.1 g, 0.09 mmol), 1,2-dimethoxyethane (30 mL), water (9 mL), and N,N-dimethylformamide (65 mL) were added and stirred at 85°C for 12 hours. After cooling, celite filtration was performed to remove solids. The filtrate was concentrated, and Intermediate 63-2 was synthesized by column purification.

Synthesis of Intermediate 63-3

Intermediate 63-3 was synthesized with reference to Bioconjugate Chemistry, 2019, vol. 30, no. 10, pp. 2647-2663.

Synthesis of Dye 63

In a 50 mL single-necked reactor, Intermediate 63-3 (3.0 g, 7.1 mmol), Intermediate 43-4 (4.0 g, 7.1 mmol), and pyridine (20 mL) were added and stirred at 50°C for 1 hour. After concentration, Dye 63 was synthesized by column purification (1.3 g).

$^1$H-NMR of the obtained Dye 63 is as follows:

$^1$H-NMR (400 MHz, DMSO-$d_6$) δ 8.63 (t, 1H, J = 12.8 Hz), 8.43 (d, 1H, J = 8.0 Hz), 8.37 (d, 1H, J = 7.8 Hz), 8.16 (d, 1H, J = 8.0 Hz), 8.14 (d, 2H, J = 8.0 Hz), 7.78-7.49 (m, 6H), 7.45-7.12 (m, 4H), 6.88 (d, 1H, J = 12.8 Hz), 6.54 (d, 1H, J = 13.4 Hz), 4.52 (q, 2H, J = 7.8 Hz), 3.83 (s, 3H), 3.76 (s, 3H), 2.11 (s, 6H), 1.35 (t, 3H, J = 7.8 Hz)

## Synthesis of Dye 66

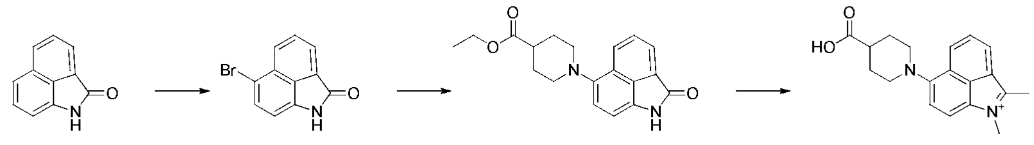

66-1 66-2 66-3

Journal of Organic Chemistry 2018, vol. 83, # 8, p. 4389 - 4401

Bioconjugate Chemistry 2019, vol. 30, # 10, p. 2647 - 2663

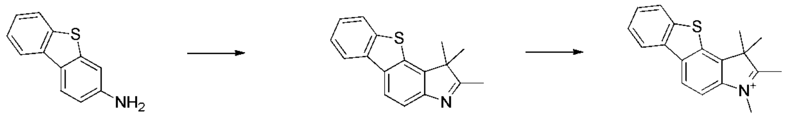

66-4 66-5

Chemistry of Heterocyclic Compounds, 1988, vol. 24, p. 87 - 92

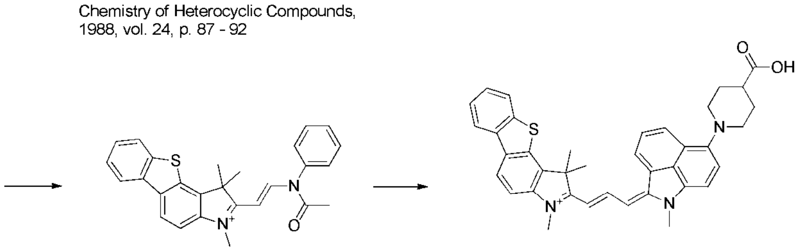

66-6 Dye 66

Synthesis of Intermediate 66-1

Intermediate 66-1 was synthesized with reference to Journal of Organic Chemistry, 2018, vol. 83, no. 8, pp. 4389-4401.

Synthesis of Intermediate 66-2

In a 1 L four-necked reactor, Intermediate 66-1 (50 g, 0.202 mol), ethyl 4-piperidinecarboxylate (38 g, 0.242 mol), $Pd_2(dba)_3$ (7.4 g, 0.008 mol), BINAP (5.0 g, 0.008 mol), sodium tert-butoxide (27 g, 0.282 mol), and toluene (500 mL) were added and stirred under reflux for 12 hours. After cooling, celite filtration was performed to remove solids. The filtrate was concentrated, and Intermediate 66-2 was synthesized by column purification.

Synthesis of Intermediate 66-3

Intermediate 66-3 was synthesized from Intermediate 66-2 by referring to the method described in Bioconjugate Chemistry, 2019, vol. 30, no. 10, pp. 2647-2663.

Synthesis of Intermediate 66-4

Intermediate 66-4 was synthesized with reference to Chemistry of Heterocyclic Compounds, 1988, vol. 24, pp. 87-92.

Synthesis of Intermediate 66-5

In a 250 mL single-necked reactor, Intermediate 66-4 (10.0 g, 38 mmol), iodomethane (8.0 g, 57 mmol), and acetonitrile (100 mL) were added and stirred under reflux for 24 hours. After cooling, ethyl acetate (100 mL) was added and

stirred for 10 minutes. The resulting solid was filtered to synthesize Intermediate 66-5.

Synthesis of Intermediate 66-6

In a 250 mL single-necked reactor, Intermediate 66-5 (5.0 g, 12 mmol), N,N-diphenylformamidine (2.6 g, 14 mmol), and acetic anhydride (50 mL) were added and stirred at 110°C for 1 hour. After cooling, ethyl acetate (100 mL) was added and stirred vigorously. The resulting solid was filtered to synthesize Intermediate 66-6.

Synthesis of Dye 66

In a 50 mL single-necked reactor, Intermediate 66-3 (3.0 g, 6.9 mmol), Intermediate 66-6 (3.8 g, 6.9 mmol), and pyridine (30 mL) were added and stirred at 50°C for 1 hour. After concentration, Dye 66 was synthesized by column purification (0.9 g).

$^1$H-NMR of the obtained Dye 66 is as follows:

$^1$H-NMR (400 MHz, DMSO-$d_6$) δ 8.54 (t, 1H, J = 12.6 Hz), 8.30 (d, 1H, J = 8.2 Hz), 8.03 (d, 1H, J = 8.4 Hz), 7.73-7.63 (m, 3H), 7.57-7.15 (m, 6H), 6.67 (d, 1H, J = 13.6 Hz), 6.51 (d, 1H, J = 12.8 Hz), 3.95 (s, 3H), 3.81 (s, 3H), 3.44 (m, 4H), 2.49-2.44 (m, 1H), 2.10 (s, 6H), 1.88-1.61 (m, 4H)

**Synthesis of Dye 77**

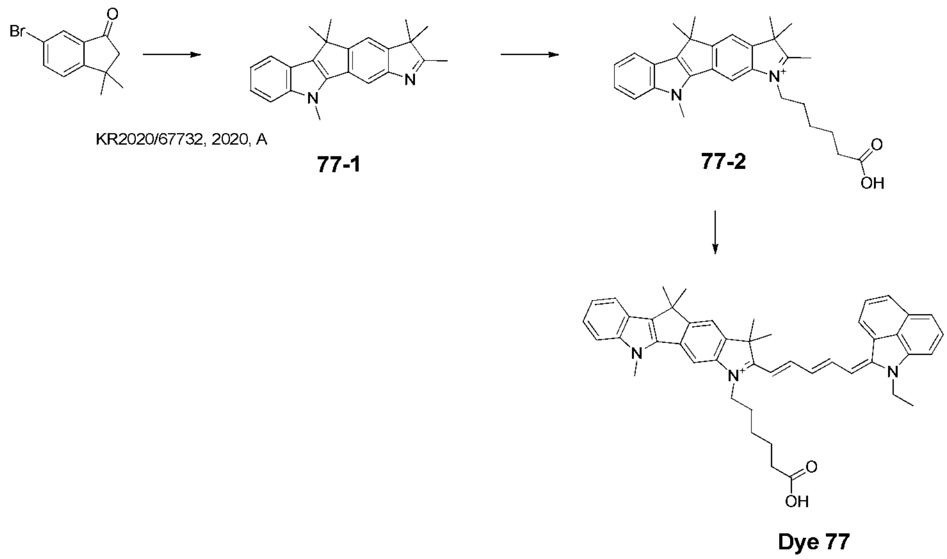


77-1

77-2

Dye 77

Synthesis of Intermediate 77-1

Intermediate 77-1 was synthesized with reference to KR2020/67732, 2020, A.

Synthesis of Intermediate 77-2

In a 250 mL single-necked reactor, Intermediate 77-1 (10 g, 30 mmol), 6-bromohexanoic acid (8.9 g, 46 mmol), and 1,2-dichlorobenzene (100 mL) were added and stirred at 150°C for 2 hours. After cooling, acetone (100 mL) was added to the reactor and stirred for 5 minutes. The resulting solid was filtered to synthesize Intermediate 77-2.

Synthesis of Dye 77

In a 100 mL single-necked reactor, Intermediate 77-2 (2.0 g, 3.8 mmol), Intermediate 18-2 (1.9 g, 3.8 mmol), and pyridine (20 mL) were added and stirred at 50°C for 1 hour. After concentration, Dye 77 was synthesized by column purification (0.7 g).

$^1$H-NMR of the obtained Dye 77 is as follows:

$^1$H-NMR (400 MHz, DMSO-$d_6$) δ 8.48-8.51 (m, 3H), 8.40 (d, 1H, J = 7.8 Hz), 8.22 (d, 1H, J = 8.4 Hz), 8.03 (t, 1H, J = 7.6 Hz),

7.86-7.40 (m, 4H), 7.19 (t, 1H, J = 8.0 Hz), 7.09 (t, 1H, 8.4 Hz), 6.75-6.53 (m, 3H), 4.41 (t, 2H, J = 7.2 Hz), 4.23 (q, 2H, J = 6.8 Hz), 3.61 (s, 3H), 2.53 (t, 2H, J = 7.2 Hz), 2.13 (s, 6H), 1.90-1.50 (m, 12H), 1.33 (t, 3H, J = 7.8 Hz)

**Synthesis of Dye 82**

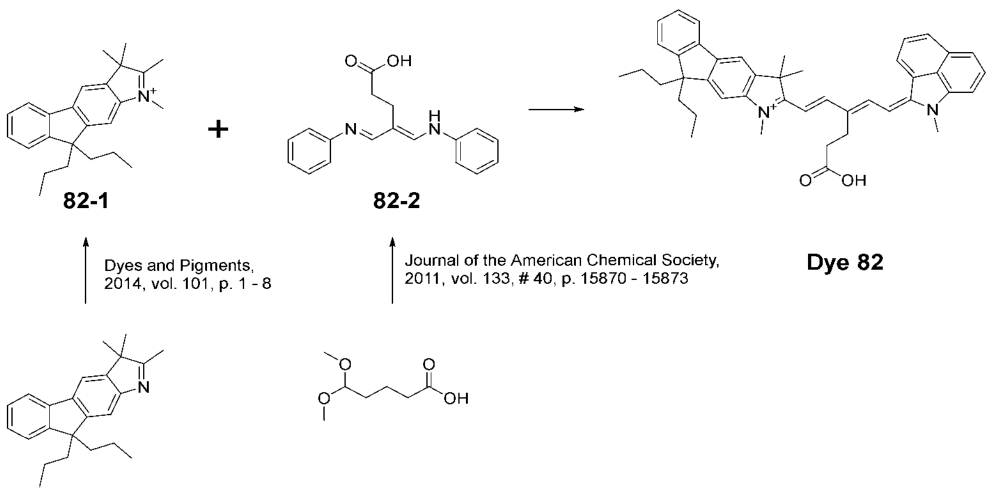


82-1 82-2 Dye 82

Synthesis of Intermediate 82-1

Intermediate 82-1 was synthesized with reference to Dyes and Pigments, 2014, vol. 101, p. 1 - 8.

Synthesis of Intermediate 82-2

Intermediate 82-1 was synthesized with reference to Journal of the American Chemical Society, 2011, vol. 133, # 40, p. 15870 - 15873.

Synthesis of Dye 82

In a 100 mL single-necked reactor, Intermediate 82-1 (4.7 g, 10.0 mmol), Intermediate 6-2 (3.0 g, 10.0 mmol), Intermediate 82-2 (1.6 g, 5.0 mmol), and pyridine (30 mL) were added and stirred at 50°C for 1 hour. After concentration, Dye 82 was synthesized by column purification (0.3g).

$^1$H-NMR of the obtained Dye 82 is as follows:

$^1$H-NMR (400 MHz, DMSO-$d_6$) δ 8.54 (d, 2H, J = 13.4 Hz), 8.49 (m, 2H), 8.32 (d, 1H, J = 8.0 Hz), 8.02 (t, 1H, J = 7.2 Hz), 7.79-7.38 (m, 8H), 6.58 (d, 2H, J = 12.8 Hz), 4.39 (s, 3H), 3.76 (s, 3H), 2.82 (t, 2H, J = 7.8 Hz), 2.17 (t, 2H, J = 7.8 Hz), 2.07-1.99 (m, 4H), 1.73 (s, 6H), 0.89-0.67 (m, 10H)

The photophysical properties measured in DMSO solvent for the non-fluorescent compounds synthesized according to the above synthesis examples are shown in Table 1 below.

[Table 1]

| Classification | $\lambda_{abs}$(nm) | $\varepsilon_{max}$ | Quantum Yield(Φ) |
|---|---|---|---|
| Dye 2 | 664 | 28,000 | 0.006 |
| Dye 6 | 672 | 75,000 | 0.005 |
| Dye 12 | 676 | 53,000 | 0.002 |
| Dye 16 | 692 | 68,000 | 0.001 |
| Dye 17 | 672 | 37,000 | 0.002 |
| Dye 18 | 680 | 74,000 | 0.005 |
| Dye 48 | 690 | 65,000 | 0.001 |

(continued)

| Classification | $\lambda_{abs}$(nm) | $\varepsilon_{max}$ | Quantum Yield($\Phi$) |
|---|---|---|---|
| Dye 60 | 600 | 42,000 | 0.007 |
| Dye 61 | 630 | 24,000 | 0.002 |

Referring to Table 1, it can be expected that the non-fluorescent compounds defined herein exhibit a quenching effect on the luminescence properties of fluorescent compounds via the FRET mechanism in a wavelength range of about 550 to 900 nm.

**Experimental Example 1. Evaluation of Spectrum Changes According to pH of Conjugates (Oligonucleotides) Singly Labeled with a Non-Fluorescent Compound at the 3'-Terminal Position**

Conjugates were prepared by labeling Dye 2, Dye 16, and Dye 17, which are non-fluorescent compounds synthesized according to the above synthesis examples, and commercially available quenchers BHQ1, BHQ2, and BHQ3 at the 3'-terminal of oligonucleotides, respectively, and spectrum changes according to pH of the conjugates were examined.

[Table 2]

| Classification | 5' → 3' |
|---|---|
| Ex 1-1 | 5'-SEQ 1-Dye 2-3' |
| Ex 1-2 | 5'-SEQ 1-Dve 16-3' |
| Ex 1-3 | 5'-SEQ 1-Dye 17-3' |
| Ex 1-4 | 5'-SEQ 1-BHQ1-3' |
| Ex 1-5 | 5'-SEQ 1-BHQ2-3' |
| Ex 1-6 | 5'-SEQ 1-BHQ3-3' |
| *SEQ 1: TTTTTTTTTT | |

Specifically, the conjugates were synthesized such that 10 thymine residues were sequentially attached starting from the 3' position using a Controlled Pore Glass (CPG) support substituted with the non-fluorescent compound on a MerMade™ 48X DNA synthesizer (see Table 2).

Subsequently, the T10 oligonucleotides labeled with each non-fluorescent compound were cleaved from the solid support under standard conditions ($NH_4OH$, 30 v/v% in H2O) and purified to separate only the Full-Length Product (FLP) by RP-HPLC, followed by drying.

The dried conjugates were titrated to pH 2, 5, 6, 8, and 10 using a pH meter, and all samples were diluted to 3 $\mu$M. Spectrum changes of all solutions were measured at room temperature using a UV spectrophotometer.

[Table 3]

| Classification | $\lambda_{max}$(nm) shift depending on pH | | | | |
|---|---|---|---|---|---|
| | pH 2 | pH 5 | pH 6 | pH 8 | pH 10 |
| Ex 1-1 | 664 | 664 | 664 | 664 | 664 |
| Ex 1-2 | 692 | 692 | 692 | 692 | 691 |
| Ex 1-3 | 672 | 672 | 672 | 672 | 671 |
| Ex 1-4 | 580 | 548 | 540 | 540 | 541 |
| Ex 1-5 | 610 | 588 | 582 | 583 | 583 |
| Ex 1-6 | 664 | 671 | 675 | 681 | 677 |

Referring to Table 3, Ex 1-4 to Ex 1-6 exhibited a greater change in λ_max as the pH decreased, and their standard deviation over the pH range of 2 to 10 was approximately 6-17 nm, indicating a large variation. In contrast, Ex 1-1

to Ex 1-3 all maintained consistent spectra over the same pH range of 2 to 10, showing minimal effect from pH, and their standard deviation was approximately 0-0.4, confirming that the properties of the non-fluorescent compounds were maintained over a wide pH range.

**Experimental Example 2. Evaluation of Quenching Efficiency According to the Distance Between Quencher and Fluorophore**

Forward probes (hereinafter referred to as "fp") and reverse probes (hereinafter referred to as "rp") were synthesized as shown in Table 4 below. The reverse probes shown in Table 4 have sequences that are complementary to all or part of the forward probes.

Specifically, rp 1-1, rp 2-1, and rp 3-1 were each hybridized with fp1 and fp2 to form a blunt ended hybrid; rp 1-2, rp 2-2, and rp 3-2 were each hybridized with fp1 and fp2 to form a 5-mer staggered hybrid; and rp 1-3, rp 2-3, and rp 3-3 were each hybridized with fp1 and fp2 to form a 10-mer staggered hybrid. Schematic diagrams of the blunt ended hybrid, 5-mer staggered hybrid, and 10-mer staggered hybrid are shown in Figure 1.

Herein, the blunt ended hybrid is a hybrid for confirming static quenching, that is, quenching caused by the formation of a ground state complex between the quencher and the fluorophore, and the staggered hybrids are hybrids for confirming dynamic quenching, particularly quenching by FRET.

The forward probes and the reverse probes were synthesized by the same method as described in Experimental Example 1.

[Table 4]

| Classification | 5' → 3' |
|---|---|
| fp 1 | 5'-6-FAM-SEQ 2-3' |
| fp 2 | 5'-Cy5-SEQ 2-3' |
| rp 1-1 | 5'-SEQ 3-Dye 2-3' |
| rp 1-2 | 5'-SEQ 4-Dye 2-3' |
| rp 1-3 | 5'-SEQ 5-Dye 2-3' |
| rp 2-1 | 5'-SEQ 3-Dye 16-3' |
| rp 2-2 | 5'-SEQ 4-Dye 16-3' |
| rp 2-3 | 5'-SEQ 5-Dye 16-3' |
| rp 3-1 | 5'-SEQ 3-Dye 17-3' |
| rp 3-2 | 5'-SEQ 4-Dye 17-3' |
| rp 3-3 | 5'-SEQ 5-Dye 17-3' |
| *SEQ 2: TTTTTTTTTTTAGCTTGCAGATGTCATAGT<br>*SEQ 3: ACTATGACATCTGCAAGCTAAAAAAAAAAA<br>*SEQ 4: ACTATGACATCTGCAAGCTAAAAAA<br>*SEQ 5: ACTATGACATCTGCAAGCTA | |

In order to evaluate the quenching efficiency according to the distance between the quencher and the fluorophore, the fluorescence intensity of fp1 and fp2, which are conjugates labeled only with a fluorophore at the 5' end, was first measured, and subsequently, the forward probes and the reverse probes were hybridized in the combinations shown in Table 5 below, the fluorescence intensity was measured, and the quenching efficiency was evaluated by substituting the two measurement results into Equation 1 below.

[Equation 1]

$$Quenching\ efficiency = 100 - \left(\frac{fluorescence\ intensity\ of\ hybrid}{fluorescence\ intensity\ of\ the\ fluorophore-labeled\ oligo} \times 100\right.$$

The fluorophore-labeled oligonucleotide was diluted to a concentration of 0.3 μM using a hybridization buffer (final concentration: NaCl 50 mM, $MgCl_2$ 5 mM, Tris-HCl 10 mM), and the fluorescence intensity was measured using a Varioskan LUX Multimode Microplate Reader (Thermo Fisher Scientific) (6-FAM: $\lambda_{ex}$ 495 nm/$\lambda_{em}$ 520 nm, Cy5: $\lambda_{ex}$ 650 nm/$\lambda_{em}$ 665 nm). Then, the fluorophore-labeled oligonucleotide and quencher-labeled oligonucleotide were diluted with the hybridization buffer to concentrations of 0.3 μM and 0.6 μM, respectively, hybridized, and the fluorescence intensity according to the distance between the fluorophore and quencher was measured using the Varioskan LUX Multimode Microplate Reader.

[Table 5]

| Classification | Probe combination | | Quenching efficiency (%) |
|---|---|---|---|
| | Forward probe | Reverse probe | |
| Ex 2-1 | fp 1 | rp 1-1 | 92 |
| | | rp 1-2 | 87 |
| | | rp 1-3 | 77 |
| Ex 2-2 | | rp 2-1 | 92 |
| | | rp 2-2 | 88 |
| | | rp 2-3 | 72 |
| Ex 2-3 | | rp 3-1 | 92 |
| | | rp 3-2 | 88 |
| | | rp 3-3 | 75 |
| Ex 2-4 | fp 2 | rp 1-1 | 95 |
| | | rp 1-2 | 95 |
| | | rp 1-3 | 94 |
| Ex 2-5 | | rp 2-1 | 90 |
| | | rp 2-2 | 90 |
| | | rp 2-3 | 87 |
| Ex 2-6 | | rp 3-1 | 95 |
| | | rp 3-2 | 95 |
| | | rp 3-3 | 94 |

The commercially available fluorescent compound Cy5 is a representative fluorescent dye in the RED region, exhibiting maximum emission at 650 nm, while Dye 2, Dye 16, and Dye 17 exhibit maximum absorption at wavelengths above 650 nm. Therefore, quenching through the FRET mechanism can occur due to the overlap between the emission spectrum of the fluorescent dye and the absorption spectrum of the quencher.

Referring to the results in Table 5, the difference in quenching efficiency according to the distance between the fluorescent dye and the quencher is only about 1-3%. Thus, it can be confirmed that the non-fluorescent compounds defined herein are capable of exhibiting quenching not only through dynamic quenching, represented by the FRET mechanism, but also through static quenching.

The commercially available fluorescent compound 6-FAM is a representative fluorescent dye in the short-wavelength region, exhibiting maximum emission at 520 nm, whereas Dye 2, Dye 16, and Dye 17 exhibit maximum absorption at wavelengths above 650 nm, resulting in minimal overlap between the emission spectrum of the fluorescent dye and the absorption spectrum of the quencher.

Referring to the results in Table 5, quenching efficiency decreases by approximately 15-20% as the distance between the fluorescent dye and the quencher increases. This suggests that when the fluorescent dye and the quencher are in close proximity, interactions between them lead to the formation of a dimer, thereby causing quenching through static quenching.

**Experimental Example 3. Evaluation of Binding Stability Depending on Fluorescent Dye-Quencher Combinations**

Forward probes (hereinafter referred to as "fp") and reverse probes (hereinafter referred to as "rp") were synthesized as shown in Table 6 below. The forward and reverse probes were synthesized in the same manner as described in Example 1.

[Table 6]

| Classification | 5' → 3' |
|---|---|
| fp 4 | 5'-Cy5-SEQ 2-3' |
| rp 4-1 | 5'-SEQ 3-3' |
| rp 4-2 | 5'-SEQ 3-Dye 2-3' |
| rp 4-3 | 5'-SEQ 3-Dye 16-3' |
| rp 4-4 | 5'-SEQ 3-Dye 17-3' |

To evaluate the binding stability according to the combinations of fluorescent dyes and quenchers, the forward probes and the reverse probes were hybridized in the combinations shown in Table 7 below, and the melting temperature (Tm) was measured from the melt curve while increasing the temperature from 37 °C to 95 °C in 0.5 °C increments.

Hybridization of the forward probes and the reverse probes was carried out by diluting them to a concentration of 0.3 μM in hybridization buffer (final concentration: NaCl 50 mM, $MgCl_2$ 5 mM, Tris-HCl 10 mM) and adjusting SYBR Green to 1X. The Tm was then measured using a CFX96 PCR machine (Bio-Rad).

[Table 7]

| Classification | Probe combination | | Tm (°C) |
|---|---|---|---|
| | Forward probe | Reverse probe | |
| Ex 3-1 | fp 4 | rp 4-1 | 70.4 |
| Ex 3-1 | fp 4 | rp 4-2 | 72.5 |
| Ex 3-2 | fp 4 | rp 4-3 | 72.8 |
| Ex 3-3 | fp 4 | rp 4-4 | 73.3 |

Referring to the results of Table 7, it was confirmed that the Tm of the hybrid of the oligonucleotides labeled with the quencher increased by about 3 °C compared to that of the hybrid of the oligonucleotides not labeled with the quencher (Ex 3-1 and 3-2). This result is presumed to be because, by using the non-fluorescent compound defined herein as a quencher, the binding strength of the double-stranded oligonucleotide formed by the affinity between the fluorescent dye and the quencher became more stable.

**Experimental Example 4. Evaluation of the signal-to-noise ratio (S/N ratio) of PCR using double-labeled oligonucleotides**

With reference to the above Experimental Examples, double-labeled oligonucleotides were prepared according to the combinations shown in Table 8 below, in which a commercial fluorescent compound selected from 6-FAM, HEX, TAMRA, Cy5, or Cy5.5 was labeled at the 5'-end, and a commercial quencher selected from BHQ-1, BHQ-2, or BHQ-3 or a non-fluorescent compound defined herein selected from Dye 2, Dye 16, or Dye 17 was labeled at the 3'-end. PCR analysis (see Table 9) using the above double-labeled oligonucleotides was performed to evaluate the PCR characteristics and the signal-to-noise ratio (S/N ratio). Real-Time PCR for BQCV (black queen cell virus) plasmid DNA was performed in duplicate at a template concentration of 10 fg/μL using a CFX-96™ Touch (Bio-Rad). The evaluation results are shown in Figures 2 and 3.

[Table 8]

| Classification | 5' → 3' |
|---|---|
| Ex 4-1 | 5'-6-FAM-SEQ 6-BHQ-1-3' |

(continued)

| Classification | 5' → 3' |
|---|---|
| Ex 4-2 | 5'-6-FAM-SEQ 6-Dye 2-1-3' |
| Ex 4-3 | 5'-6-FAM-SEQ 6 -Dye 16-1-3' |
| Ex 4-4 | 5'-6-FAM-SEQ 6-Dye 17-1-3' |
| Ex 4-5 | 5'-HEX-SEQ 6-BHQ-1-3' |
| Ex 4-6 | 5'-HEX-SEQ 6-Dye 2-1-3' |
| Ex 4-7 | 5'-HEX-SEQ 6-Dye 16-1-3' |
| Ex 4-8 | 5'-HEX-SEQ 6-Dye 17-1-3' |
| Ex 4-9 | 5'-TAMRA-SEQ 6-BHQ-2-3' |
| Ex 4-10 | 5'-TAMRA-SEQ 6-Dye 2-1-3' |
| Ex 4-11 | 5'-TAMRA-SEQ 6-Dye 16-1-3' |
| Ex 4-12 | 5'-TAMRA-SEQ 6-Dye 17-1-3' |
| Ex 4-13 | 5'-Cy5-SEQ 6-BHQ-2-3' |
| Ex 4-14 | 5'-Cy5-SEQ 6-Dye 2-1-3' |
| Ex 4-15 | 5'-Cy5-SEQ 6-Dye 16-1-3' |
| Ex 4-16 | 5'-Cy5-SEQ 6-Dye 17-1-3' |
| Ex 4-17 | 5'-Cy5.5-SEQ 6-BHQ-3-3' |
| Ex 4-18 | 5'-Cy5.5-SEQ 6-Dye 2-1-3' |
| Ex 4-19 | 5'-Cy5.5-SEQ 6-Dye 16-1-3' |
| Ex 4-20 | 5'-Cy5.5-SEQ 6-Dye 17-1-3' |
| *SEQ 6: Black queen cell virus complement (23 mer; CCATCTTTATCGGTACGCCGCCC) | |

[Table 9]

| Classification | Content (μl) |
|---|---|
| (Bioline) SensiFAST™ Probe No-ROX Mix (2X) | 10 |
| BQCV plasmid DNA (10 fg/ul) | 3 |
| BQCV F/R primer mix (5 pmole/ul) | 1 |
| BQCV Dual-labeled probe (5 pmole/ul) | 1 |
| DEPC Water | 5 |
| * PCR Protocol : 95°C, 3min - [95°C, 10s - 60°C, 30s] x 45cycles | |

Referring to Figures 2 and 3, it was confirmed that, for the commercial fluorescent dyes 6-FAM, HEX, TAMRA, Cy5, and Cy5.5, when the non-fluorescent compound defined herein was used as a quencher, lower Ct values and higher S/N ratios were obtained compared to when the commercial quenchers BHQ-1, BHQ-2, or BHQ-3 were used.

Based on these results, it was confirmed that the non-fluorescent compound defined herein can be used as a quencher for various fluorescent dyes having different emission spectra even as a single compound. Furthermore, considering molecular diagnostic applications, it can be expected that the non-fluorescent compound defined herein will provide improved detection sensitivity and accuracy compared to commercial quenchers even in cases where the target gene is present in a relatively low concentration in a sample. In particular, it was confirmed that the non-fluorescent compound defined herein can function as a molecular diagnostic probe via static quenching even in cases where there is minimal spectral overlap with 6-FAM.

**Experimental Example 5. Evaluation of sequence dependency of double-labeled oligonucleotides**

With reference to the above Experimental Examples, double-labeled oligonucleotides were prepared according to the combinations shown in Table 10 below, in which a commercial fluorescent dye Cy5 was labeled at the 5'-end, and a commercial quencher BHQ-2 or a non-fluorescent compound defined herein Dye 6, Dye 18, or Dye 60 was labeled at the 3'-end. PCR analysis (see Table 9) using the above double-labeled oligonucleotides was performed to evaluate PCR characteristics and the signal-to-noise ratio (S/N ratio). The evaluation results are shown in Table 11 and Figures 4 to 6.

[Table 10]

| Classification | 5' → 3' |
|---|---|
| Ex 5-1 | 5'-Cy5-SEQ 7-Dye 6-3' |
| Ex 5-2 | 5'-Cy5-SEQ 7-BHQ-2-3' |
| Ex 5-3 | 5'-Cy5-SEQ 8-Dye 18-3' |
| Ex 5-4 | 5'-Cy5-SEQ 8-BHQ-2-3' |
| Ex 5-5 | 5'-Cy5-SEQ 9-Dye 60-3' |
| Ex 5-6 | 5'-Cy5-SEQ 9-BHQ-2-3' |
| *SEQ 7: Neisseria gonorrhoeae complement (23 mer; CAGTCGAGAACAGCAAAGCAACG)<br>*SEQ 8: Mycoplasma genitalium complement (28 mer; AGCCTTTCTAACCGCTGCACTTACCCTT)<br>*SEQ 9: Chlamydia trachomatis complement (27 mer; TTGTCCATATCTTTGATACGACGCCGC) | |

[Table 11]

| Classification | DNA (fg/ul) | Ct | Background | S/N Ratio | Norm. S/N Ratio |
|---|---|---|---|---|---|
| Ex 5-1 | 1000 | 20.13 | 4138 | 5.27 | 1.55 |
| | 100 | 23.61 | | | |
| | 10 | 26.83 | | | |
| | 1 | 30.20 | | | |
| Ex 5-2 | 1000 | 20.10 | 6241 | 3.39 | 1.00 |
| | 100 | 23.60 | | | |
| | 10 | 26.96 | | | |
| | 1 | 30.32 | | | |
| Ex 5-3 | 1000 | 23.88 | 2626 | 5.65 | 1.45 |
| | 100 | 27.02 | | | |
| | 10 | 30.37 | | | |
| | 1 | 33.36 | | | |
| Ex 5-4 | 1000 | 24.15 | 4067 | 3.90 | 1.00 |
| | 100 | 27.14 | | | |
| | 10 | 30.49 | | | |
| | 1 | 33.59 | | | |
| Ex 5-5 | 1000 | 23.67 | 4756 | 3.63 | 1.24 |
| | 100 | 26.86 | | | |
| | 10 | 30.14 | | | |
| | 1 | 33.47 | | | |

(continued)

| Classification | DNA (fg/ul) | Ct | Background | S/N Ratio | Norm. S/N Ratio |
|---|---|---|---|---|---|
| Ex 5-6 | 1000 | 23.95 | 6095 | 2.94 | 1.00 |
| | 100 | 27.12 | | | |
| | 10 | 30.34 | | | |
| | 1 | 33.88 | | | |

Referring to Table 11 and Figures 4 to 9, the Ct value range of the non-fluorescent compound defined herein was -0.41 < ΔCt < 0.03, indicating that it has sensitivity equivalent to or higher than that of the commercial quencher BHQ-2, and it was confirmed that it exhibits lower background and higher S/N ratio compared to the commercial quencher.

**Experimental Example 6. Evaluation of reducing agent resistance of double-labeled oligonucleotides**

With reference to the above Experimental Examples, double-labeled oligonucleotides were prepared according to the combinations shown in Table 12 below, in which a commercial fluorescent dye 6-FAM, Cy5, or Cy5.5 was labeled at the 5'-end, and a commercial quencher BHQ-1, BHQ-2, or BHQ-3 or a non-fluorescent compound defined herein Dye 6, Dye 48, or Dye 60 was labeled at the 3'-end. The change in HPLC purity was measured after addition of DTT (1,4-dithiothreitol) as a reducing agent to a concentration of 100 mM and overnight reaction for 24 hours. Specifically, the oligonucleotides corresponding to Ex 6-1 to Ex 6-6 were each quantified to a concentration of 100 μM using a 100 mM DTT solution, and their purity was measured immediately. After storage under room temperature conditions (21°C) for 24 hours, purity was measured again using the same method. The evaluation results are shown in Figures 7 and 8.

[Table 12]

| Classification | 5' → 3' |
|---|---|
| Ex 6-1 | 5'-6-FAM-SEQ 10-Dye 6-3' |
| Ex 6-2 | 5'-Cy5-SEQ 10-Dye 48-3' |
| Ex 6-3 | 5'-Cy5.5-SEQ 10-Dye 60-3' |
| Ex 6-4 | 5'-6-FAM-SEQ 10-BHQ 1-3' |
| Ex 6-5 | 5'-Cy5-SEQ 10-BHQ 2-3' |
| Ex 6-6 | 5'-Cy5.5-SEQ 10-BHQ 3-3' |
| *SEQ 10: TTTTTTTTTTTTTTTTTTTT | |

Referring to Figures 7 and 8, it was confirmed that the oligonucleotides using the non-fluorescent compound defined herein as a quencher exhibited only a slight change in purity, ranging from a minimum of about 0.4% to a maximum of about 1.6%, under reducing conditions, whereas the oligonucleotides using commercial quenchers BHQ-1, BHQ-2, or BHQ-3 as quenchers exhibited a drastic change in purity, ranging from a minimum of about 69.7% to a maximum of about 86.7%, under reducing conditions. That is, the non-fluorescent compound defined herein can be stably maintained even in the presence of a reducing agent due to its high durability, and thus is expected to be usable in a wider range of molecular diagnostic fields compared to commercial quenchers.

**Experimental Example 7. Comparison of efficiency of double-labeled oligonucleotides depending on the presence of MGB**

With reference to the above Experimental Examples, double-labeled oligonucleotides were prepared according to the combinations shown in Table 13 below, in which a commercial fluorescent dye 6-FAM, HEX, TAMRA, or Cy5 was labeled at the 5'-end, a non-fluorescent compound defined herein Dye 12, Dye 48, or Dye 61 was labeled at the 3'-end, and an MGB (Minor Groove Binder) was included. PCR characteristics (see Experimental Example 4) and Tm change (see Experimental Example 3) depending on the presence of the MGB in the double-labeled oligonucleotides were measured. The measurement results are shown in Table 14 and Figures 9 to 12.

Specifically, the double-labeled oligonucleotides containing MGB were synthesized by attaching, in sequence from the 3'-position, each target-complementary sequence (14 mer) as a probe using MGB-CPG substituted with Dye 12,

Dye 48, or Dye 61 as the support, on a MerMade™ 48X DNA synthesizer, wherein only the oxidation step in the standard procedure, which may introduce side reactions to the MGB, was replaced with 0.5M CSO [(1S)-(+)-(10-camphorsulfonyl)-oxaziridine)]. Subsequently, 6-FAM, HEX, TAMRA, or Cy5 was attached at the 5'-end, and the fluorescent dye-labeled probe was separated from the solid support using a standard method ($NH_4OH$, 30 v/v% in $H_2O$), followed by RP-HPLC purification to isolate only the FLP (Full-Length Product), and dried.

[Table 13]

| Classification | 5' → 3' |
|---|---|
| Ex 7-1 | 5'-6-FAM-SEQ 11-MGB-Dye 61-3' |
| Ex 7-2 | 5'-HEX-SEQ 11-MGB-Dye 61-3' |
| Ex 7-3 | 5'-TAMRA-SEQ 11-MGB-Dye 12-3' |
| Ex 7-4 | 5'-Cy5-SEQ 11-MGB-Dye 48-3' |
| Ex 7-5 | 5'-6-FAM-SEQ 11-Dye 61-3' |
| Ex 7-6 | 5'-HEX-SEQ 11-Dye 61-3' |
| Ex 7-7 | 5'-TAMRA-SEQ 11-Dye 12-3' |
| Ex 7-8 | 5'-Cy5-SEQ 11-Dye 48-3' |
| *SEQ 11: Black queen cell virus complement (14 mer; TTATCGGTACGCCG) | |

[Table 14]

| Classification | 6-FAM | HEX | TAMRA | Cy5 |
|---|---|---|---|---|
| Ex 7-1 ~ Ex 7-4 | 64.4°C | 61.1°C | 60.5°C | 63.7°C |
| Ex 7-5 ~ Ex 7-8 | 58.2°C | 55.3°C | 55.0°C | 57.6°C |

Referring to Table 14, it was confirmed that the average Tm values of Ex 7-1 to Ex 7-4 were about 6°C higher than those of Ex 7-5 to Ex 7-8. In addition, referring to Figures 9 to 12, it was confirmed that ΔRFU increased and Ct values decreased in Ex 7-1 to Ex 7-4 compared to Ex 7-5 to Ex 7-8.

## Experimental Example 8. Evaluation of PCR characteristics of oligonucleotides containing multiple quenchers

With reference to the above Experimental Examples, oligonucleotides were prepared according to the combinations shown in Table 15 below, in which a commercial fluorescent dye 6-FAM was labeled at the 5'-end, a commercial quencher IBFQ (Iowa Black FQ) or a non-fluorescent compound defined herein Dye 6, Dye 18, or Dye 60 was labeled at the 3'-end, and a commercial quencher Eclipse® (Elitech group) or ZEN was additionally introduced between the oligonucleotide sequences. PCR characteristics of the oligonucleotides (see Experimental Example 4) were evaluated. Ex 8-5 used a triple-labeled oligonucleotide commercially available from Integrated DNA Technologies, Inc. The evaluation results are shown in Figure 13.

[Table 15]

| Classification | Dual-labeled probe/triple-labeled probe (probe sequence: M.musculus complement) |
|---|---|
| Ex 8-1 | 5'-6-FAM-SEQ 12-Dye 6 -3' |
| Ex 8-2 | 5'-6-FAM-SEQ 12-Dye 18 -3' |
| Ex 8-3 | 5'-6-FAM-SEQ 12-Dye 60 -3' |
| Ex 8-4 | 5'-6-FAM-SEQ 13-Dye 6-3' |

(continued)

| Classification | Dual-labeled probe/triple-labeled probe (probe sequence: M.musculus complement) |
|---|---|
| Ex 8-5 | 5'-6-FAM-SEQ 14-IBFQ-3' |
| *SEQ 12: AAGAGTAGT-Eclipse-AGCCTAAGAGTGTCAGTTGTACATCA<br>*SEQ 13: AAGAGTAGTAGCCTAAGAGTGTCAGTTGTACATCA<br>*SEQ 14: AAGAGTAGT-ZEN-AGCCTAAGAGTGTCAGTTGTACATCA | |

Referring to Figure 13, it was confirmed that, compared to Ex 8-4, which is a dual-labeled oligonucleotide containing a single quencher, Ex 8-1 to Ex 8-3, which are triple-labeled oligonucleotides containing two quenchers, exhibited a 65% reduction in background, a 42% increase in ΔRFU, and lower Ct values. That is, it was confirmed that the PCR amplification performance of triple-labeled oligonucleotides containing two quenchers was superior to that of dual-labeled oligonucleotides containing a single quencher.

In addition, in the case of Ex 8-1 to Ex 8-3, which include a non-fluorescent compound defined herein as one of the two quenchers, it was confirmed that they exhibited PCR amplification performance comparable to that of Ex 8-5, which is a commercially available triple-labeled oligonucleotide.

**Experimental Example 9. Evaluation of solubility of non-fluorescent compounds**

With reference to the synthesis method of Dye 6 described in the Synthesis Examples, a non-fluorescent compound in which $R_4$ and $R_{11}$ in the structure of Dye 6 were hydroxyl groups (-OH) and a non-fluorescent compound in which $R_4$ and $R_{11}$ in the structure of Dye 6 were sulfonate ($-SO_3^-$) groups were additionally synthesized, and the solubility of the non-fluorescent compounds in each of the solvents shown in Table 16 below was measured. Specifically, an amount corresponding to 2 mg of the non-fluorescent compound was placed in 2 mL of each solvent, allowed to stand at 25°C for 30 minutes, and it was checked whether the non-fluorescent compound was completely dissolved.

[Table 16]

| Classification | Ex 9-1 (Dye 6) | Ex 9-2 (-OH modification) | Ex 9-2 ($-SO_3^-$ modification) |
|---|---|---|---|
| Dichloromethane | O | O | X |
| Acetonitrile | O | O | X |
| Methanol | O | O | X |
| Purified water | X | X | O |

Referring to Table 16, it was confirmed that the non-fluorescent compounds according to Ex 9-1 and Ex 9-2 exhibited lipophilicity, whereas the non-fluorescent compound according to Ex 9-3 exhibited hydrophilicity.

Although several embodiments of the present invention have been described above, those skilled in the art can modify and change the present invention in various ways by adding, changing, or deleting components without departing from the spirit of the present invention as set forth in the claims, and such modifications are also included within the scope of the rights of the present invention.

## Claims

**1.** A non-fluorescent compound represented by Chemical Formula 1 below,

wherein the non-fluorescent compound is capable of reducing fluorescence through at least one mechanism selected from static quenching and dynamic quenching,

[Chemical Formula 1]

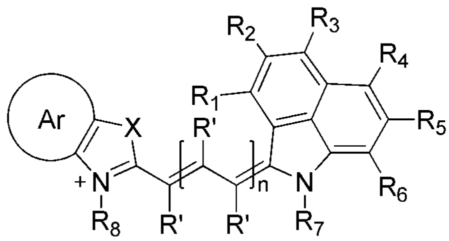

wherein,

Ar is a C5-C50 aryl, a C2-C50 heteroaryl, or a C5-C50 aliphatic-aromatic mixed ring, and is optionally substituted with at least one R",

$R_1$ to $R_6$, R' and R" are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido ($-O^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$,

$R_x$ is a reactive group, or a group comprising a backbone including a hydrocarbon having 1 to 40 carbon atoms to which at least one reactive group is bound,

wherein the reactive group is selected from carboxyl, carboxyl derivatives, carboxylate ($-CO_2^-$), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkane, haloacetamide, imido ester, glycol, halotriazine, hydrazine, acyl halide, alkyl halide and aryl halide,

$R_s$ is a carrier molecule, or a group comprising a backbone including a hydrocarbon having 1 to 40 carbon atoms to which at least one carrier molecule is bound,

$R_7$ and $R_8$ are each independently hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, optionally substituted C5-C50 aryl, optionally substituted C2-C50 heteroaryl, optionally substituted C5-C50 aralkyl, $R_x$ and $R_s$,

X is $CR_{20}R_{21}$, $NR_{22}$, O or S,

$R_{20}$ to $R_{22}$ are each independently hydrogen, optionally substituted C1-C40 alkyl, optionally substituted C1-C40 heteroalkyl, optionally substituted C3-C30 cycloalkyl, optionally substituted C3-C30 heterocycloalkyl, optionally substituted aryl, optionally substituted heteroaryl, $R_x$ and $R_s$, or are bonded to each other to form an optionally substituted 5- to 7-membered ring,

n is an integer of 0 to 5,

optionally, any two adjacent groups among $R_1$ to $R_6$ are bonded to each other to form an optionally substituted 5- to 7-membered ring, and

at least one $R_x$ or at least one $R_s$ is present in the non-fluorescent compound represented by Chemical Formula 1.

**2.** The non-fluorescent compound of claim 1,

wherein Ar is selected from [Ar-1] to [Ar-6] below,

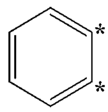

[Ar-1]

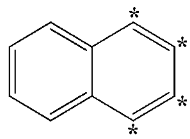

[Ar-2]

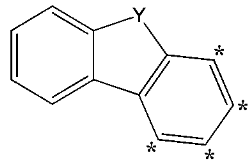

[Ar-3]

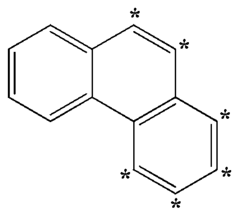

[Ar-4]

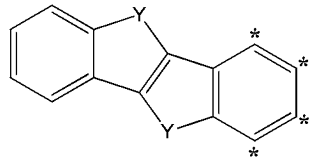

[Ar-5]

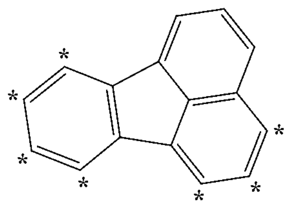

[Ar-6]

wherein [Ar-1] to [Ar-6] are each optionally substituted with at least one R",
* denotes a carbon position capable of being bonded to the nitrogen-containing five-membered ring of Chemical Formula 1,
Y is each independently $CR_{23}R_{24}$, $NR_{25}$, O or S, and
$R_{23}$ to $R_{25}$ are each independently optionally substituted C1-C40 alkyl, optionally substituted C1-C40 heteroalkyl, optionally substituted C3-C30 cycloalkyl, optionally substituted C3-C30 heterocycloalkyl, optionally substituted aryl, optionally substituted heteroaryl, Rx and Rs, or are bonded to each other to form an optionally substituted 5- to 7-membered ring.

**3.** A non-fluorescent compound represented by Chemical Formula 6 below,

wherein the non-fluorescent compound is capable of reducing fluorescence through at least one mechanism selected from static quenching and dynamic quenching,

[Chemical Formula 6]

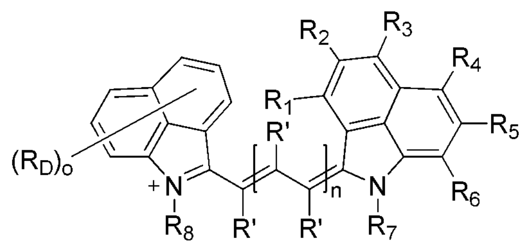

wherein,
$R_1$ to $R_6$, R' and $R_D$ are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido ($-O^-$), optionally substituted C1-C40 alkoxy, optionally substituted C3-C40 cycloalkoxy, optionally substituted C5-C40 aryloxy, optionally substituted C2-C40 heteroaryloxy,

optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$,

$R_x$ is a reactive group, or a group comprising a backbone including a hydrocarbon having 1 to 40 carbon atoms to which at least one reactive group is bound,

wherein the reactive group is selected from carboxyl, carboxyl derivatives, carboxylate ($-CO_2^-$), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkane, haloacetamide, imido ester, glycol, halotriazine, hydrazine, acyl halide, alkyl halide and aryl halide,

$R_s$ is a carrier molecule, or a group comprising a backbone including a hydrocarbon having 1 to 40 carbon atoms to which at least one carrier molecule is bound,

$R_7$ and $R_8$ are each independently hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, optionally substituted C5-C50 aryl, optionally substituted C2-C50 heteroaryl, optionally substituted C5-C50 aralkyl, $R_x$ and $R_s$,

n is an integer of 0 to 5,

o is an integer of 0 to 6,

optionally, any two adjacent groups among $R_1$ to $R_6$ are bonded to each other to form an optionally substituted 5- to 7-membered ring, and

at least one $R_x$ or at least one $R_s$ is present in the non-fluorescent compound represented by Chemical Formula 6.

**4.** A non-fluorescent compound represented by Chemical Formula 7 below,

wherein the non-fluorescent compound is capable of reducing fluorescence through at least one mechanism selected from static quenching and dynamic quenching,

[Chemical Formula 7]

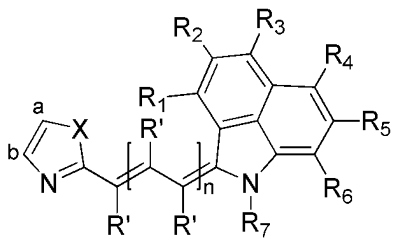

wherein,

$R_1$ to $R_6$ and R' are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido ($-O^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate,

thiocarbonylamino, isonitrile, cyanate, imide, ether, thioether, $R_x$ and $R_s$,
$R_x$ is a reactive group, or a group comprising a backbone including a hydrocarbon having 1 to 40 carbon atoms to which at least one reactive group is bound,
wherein the reactive group is selected from carboxyl, carboxyl derivatives, carboxylate ($-CO2^-$), carboxylate salts, hydroxyl, dienophile, aldehyde, substituted ketone, sulfonyl halide, thiol, unsubstituted amino, primary amino, alkene, alkyne, halogen, hydrazide, azide, imide, ketene, isocyanate, thiocyanate, isothiocyanate, epoxide, maleimide, 1,2,4,5-tetrazine derivatives, cycloalkyne derivatives, cycloalkene, triphosphate, phosphoramidite, substituted thioketone, haloformyl, formyl, acyl, acylamide, acyl azide, organic acid anhydride, aniline, aziridine, boronate, carbodiimide, diazoalkane, haloacetamide, imido ester, glycol, halotriazine, hydrazine, acyl halide, alkyl halide and aryl halide,
$R_s$ is a carrier molecule, or a group comprising a backbone including a hydrocarbon having 1 to 40 carbon atoms to which at least one carrier molecule is bound,
$R_7$ is selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, optionally substituted C5-C50 aryl, optionally substituted C2-C50 heteroaryl, optionally substituted C5-C50 aralkyl, $R_x$ and $R_s$,
X is $CR_{20}R_{21}$, $NR_{22}$, O or S,
$R_{20}$ to $R_{22}$ are each independently hydrogen, optionally substituted C1-C40 alkyl, optionally substituted C1-C40 heteroalkyl, optionally substituted C3-C30 cycloalkyl, optionally substituted C3-C30 heterocycloalkyl, optionally substituted aryl, optionally substituted heteroaryl, $R_x$ and $R_s$, or are bonded to each other to form an optionally substituted 5- to 7-membered ring,
n is an integer of 0 to 5,
a and b in Chemical Formula 7 are bonded to two adjacent substituents selected from $R_{31}$ to $R_{35}$ of Chemical Formula 8 below,

[Chemical Formula 8]

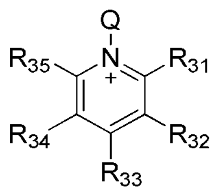

substituents among $R_{31}$ to $R_{35}$ of Chemical Formula 8 that are not bonded to a and b of Chemical Formula 7 are each independently selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, hydroxyl, oxido ($-O^-$), optionally substituted C1-C40 alkoxy, optionally substituted C3-C40 cycloalkoxy, optionally substituted C5-C40 aryloxy, optionally substituted C2-C40 heteroaryloxy, optionally substituted C5-C50 aryl, optionally substituted C2-C50 heteroaryl, optionally substituted C5-C50 aralkyl, optionally substituted C1-C40 alkylthio, optionally substituted C5-C40 arylthio, optionally substituted C3-C40 cycloalkylthio, optionally substituted C2-C40 heteroarylthio, optionally substituted acylamino, acyloxy, substituted sulfonate ester, optionally substituted sulfonamide, substituted ester, nitroso (-N=O), halogen, optionally substituted silyl, optionally substituted amide, optionally substituted carbamate, nitrile, acetal, ketal, optionally substituted amino, thioamide, aminocarbonyl-oxy, aminosulfonyl-oxy, amidino group, sulfonyl-oxy, sulfonate, thiocarbonylamino, isonitrile, cyanate, imide, ether, thioether, $R_x$ and $R_s$,
optionally, two adjacent substituents among $R_{31}$ to $R_{35}$ of Chemical Formula 8 that are not bonded to a and b of Chemical Formula 7 are bonded to each other to form an optionally substituted 5- to 7-membered ring,
Q is selected from hydrogen, optionally substituted C1-C40 alkyl, optionally substituted C1-C40 heteroalkyl, optionally substituted C2-C40 alkenyl, optionally substituted C2-C40 alkynyl, optionally substituted C3-C20 cycloalkyl, optionally substituted C3-C20 cycloalkenyl, optionally substituted C2-C20 heterocycloalkyl, optionally substituted C5-C50 aryl, optionally substituted C2-C50 heteroaryl, optionally substituted C5-C50 aralkyl, $R_x$ and $R_s$,
and at least one $R_x$ or at least one $R_s$ is present in the non-fluorescent compound represented by Chemical Formula 7.

5. The non-fluorescent compound of any one of claims 1 to 4, wherein, when at least one $R_x$ or at least one $R_s$ is present in the non-fluorescent compound, the at least one $R_x$ or the at least one $R_s$ present in the non-fluorescent compound is protected by a protecting group.

6. The non-fluorescent compound of any one of claims 1 to 4, wherein the non-fluorescent compound is capable of reducing fluorescence in a wavelength range of 550-900 nm through dynamic quenching or reducing fluorescence in a wavelength range of 300-900 nm through static quenching.

7. The non-fluorescent compound of any one of claims 1 to 4, wherein the non-fluorescent compound is capable of reducing fluorescence in a wavelength range of 550-900 nm through fluorescence resonance energy transfer (FRET) or reducing fluorescence in a wavelength range of 300-900 nm through ground state quenching.

8. The non-fluorescent compound of any one of claims 1 to 4, wherein the non-fluorescent compound is a compound for labeling nucleic acid.

9. A conjugate comprising the non-fluorescent compound of any one of claims 1 to 4 as a quencher.

10. The conjugate of claim 9, wherein the conjugate is a nucleotide conjugate.

11. The conjugate of claim 9, wherein the conjugate is a probe or a primer.

12. A conjugate comprising:

a quencher comprising the non-fluorescent compound of any one of claims 1 to 4, and
a fluorophore.

13. A conjugate comprising:

a first quencher comprising the non-fluorescent compound of any one of claims 1 to 4,
a second quencher selected from azo, coumarin, cyanine, BODIPY, fluorescein, rhodamine, pyrene, carbopyronine, benzo[c,d] indole, oxazine, xanthene, thioxanthene, acridine, and derivatives thereof, and
a fluorophore.

14. The conjugate of claim 13, wherein the second quencher is at least one selected from dabcyl, Eclipse™, Black Hole Quencher™ BHQ0 (BHQnova), Black Hole Quencher™ BHQ1, Black Hole Quencher™ BHQ2, Black Hole Quencher™ BHQ3, BlackBerry™ Quencher 650 (BBQ650™), Iowa Black™ FQ (IABkFQ), SFC™ Q1, SFC™ Q2, Iowa Black™ RQ-n1, Iowa Black™ RQ n2, Iowa Black™ RQSp, TAMRA, Deep Dark Quencher I (DDQ I), Deep Dark Quencher II (DDQ II), QXL™520, QXL™570, QXL™610, QXL™670, IRQXL™, IRDye™, QC-1, QSY™, QSY™2, BMN-Q460, BMN-Q535, BMN-Q1, BMN-Q2, BMN-Q590, BMN-Q620, and BMN-Q651.

15. The conjugate of claim 12, wherein the fluorophore is at least one selected from coumarin, cyanine, BODIPY, fluorescein, rhodamine, pyrene, carbopyronine, oxazine, xanthene, thioxanthene, acridine, and derivatives thereof.

16. The conjugate of claim 15, wherein the fluorophore is at least one selected from 6-FAM™, TET™, JOE™, VIC®, HEX™, NED™, PET®, ROX™, TAMRA™, TET™, Texas Red®, SUN, MAX, ABY, JUN, LIZ, TAZ, CAL Fluor® Gold 540, CAL Fluor® Orange 560, CAL Fluor® Red 590, CAL Fluor® Red 610, CAL Fluor® Red 635, Cy® (cyanine) 3, Cy® 3.5, Cy® 5, Cy® 5.5, Cy® 7, Cy® 7.5, Quasar® 570, Quasar® 670, Quasar® 705, Rhodamine Green™, Rhodamine Red™, LightCycler® Cyan 500, LightCycler® Red 610, LightCycler® Red 640, LightCycler® Red 670, LightCycler® Red 705, Oregon Green® 488, Oregon Green® 500, Oregon Green® 514, the Alexa Fluor® dyes 350, 405, 488, 532, 546, 555, 568, 594, 610, 647, 680 and Alexa Fluor® 750, the BODIPY® dyes, Epoch Blue, AMCA, Marina Blue®, Pacific Blue™, Pacific Green™, Pacific Orange™, Yakima Yellow™, the ATTO dyes 390, 425, 465, 488, 495, 514, 520, 532, Rho6G, 542, 550, 565, Rho3B, Rho11, Rho12, Thio12, Rho101, 590, 594, Rho13, 610, 620, Rho14, 633, 643, 647, 647N, 655, Oxa12, 665, 680, 700, 725, ATTO 740, Texas red, Oyster 645, SFC™-V, SFC™-N, SFC™574, SFC™647, SFC™-C610, SFC™620, SFC™670, SFC™705, Chamel™560, Chamel™610, Chamel™670, and Chamel™705.

**17.** The conjugate of claim 12,
further comprising a minor groove binder (MGB).

**18.** A composition for detecting nucleic acid comprising the conjugate of claim 12.

**19.** A support for detecting nucleic acid comprising:

a quencher comprising the non-fluorescent compound of any one of claims 1 to 4;
a support; and
a linker connecting the quencher and the support.

**20.** The support for nucleic acid detection of claim 19, wherein the support is glass, cellulose, nylon, acrylamide gel, dextran, polystyrene, or resin.

**21.** A method for detecting nucleic acid comprising:

(a) preparing a reaction mixture comprising a target nucleic acid, reagents necessary to amplify the target nucleic acid, and the conjugate of claim 12;
(b) amplifying the target nucleic acid in the reaction mixture; and
(c) measuring fluorescence intensity of the reaction mixture.

**22.** The method of claim 21,
wherein step (b) comprises:

(b-1) extending the nucleotide conjugate hybridized to the target nucleic acid by a polymerase;
(b-2) separating a quencher and a fluorophore of the nucleotide conjugate from the target nucleic acid by exonuclease activity of the polymerase; and
(b-3) allowing the fluorophore separated from the quencher to emit fluorescence.

**23.** The method of claim 21,
further comprising:
(d) determining the amount of amplification of the target nucleic acid based on the fluorescence intensity measured in step (c).

**24.** The method of claim 21, wherein step (b) is performed by a method selected from strand displacement amplification (SDA), polymerase chain reaction (PCR), reverse transcription polymerase chain reaction (RT-PCR), real-time polymerase chain reaction, allele-specific polymerase chain reaction, ligase chain reaction (LCR), rolling circle amplification (RCA), isothermal multiple displacement amplification (IMDA), recombinase polymerase amplification (RPA), self-sustained sequence replication (3SR), single primer isothermal amplification (SPIA), multiple displacement amplification (MDA), whole genome amplification (WGA), cross-priming amplification (CPA), signal mediated amplification of RNA technology (SMART), transcription mediated amplification (TMA), nucleic acid sequence based amplification (NASBA), loop-mediated isothermal amplification (LAMP), and helicase dependent amplification (HDA).

FIG. 1

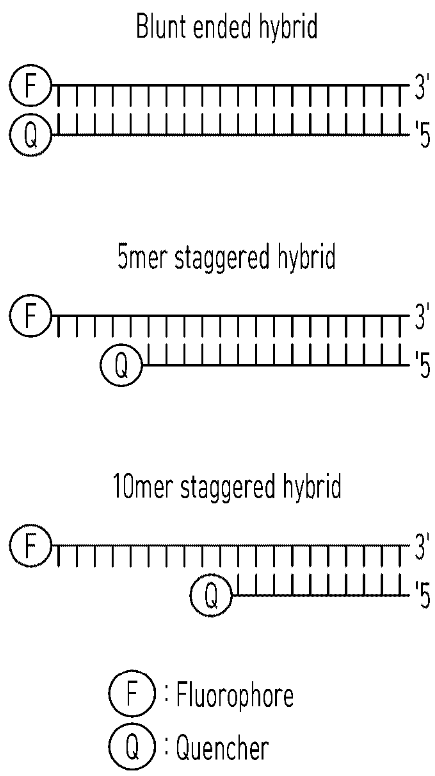
Blunt ended hybrid
F
Q
3'
'5
5mer staggered hybrid
F
Q
3'
'5
10mer staggered hybrid
F
Q
3'
'5
F : Fluorophore
Q : Quencher

FIG. 2

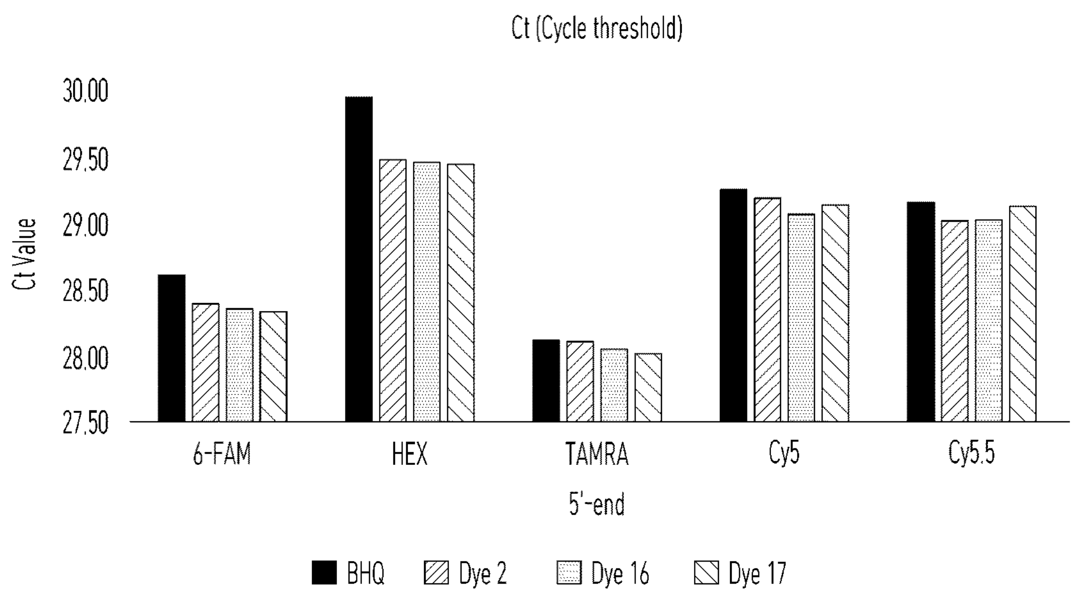
Ct (Cycle threshold)
Ct Value
30.00
29.50
29.00
28.50
28.00
27.50
6-FAM
HEX
TAMRA
Cy5
Cy5.5
5'-end
BHQ
Dye 2
Dye 16
Dye 17

FIG. 3

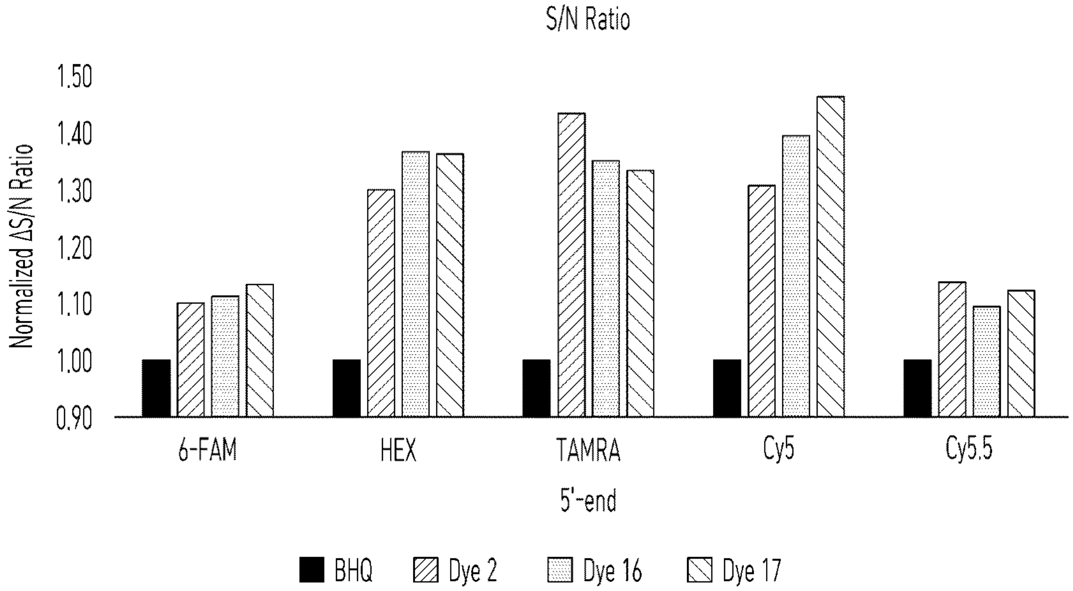
S/N Ratio
Normalized ΔS/N Ratio
1.50
1.40
1.30
1.20
1.10
1.00
0.90
6-FAM
HEX
TAMRA
Cy5
Cy5.5
5'-end
BHQ
Dye 2
Dye 16
Dye 17

FIG. 4

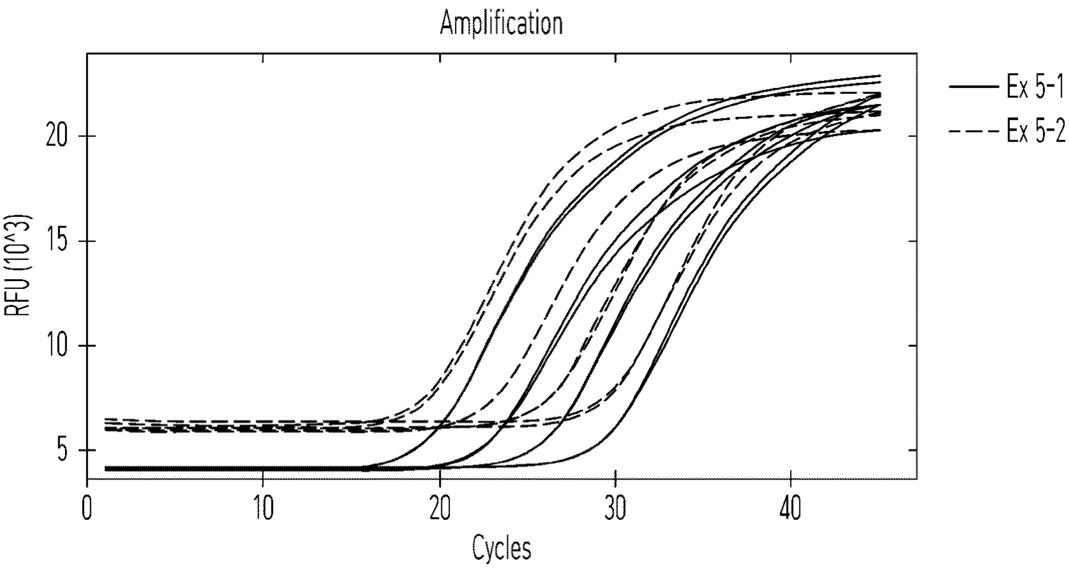
Amplification
RFU (10^3)
20
15
10
5
0
10
20
30
40
Cycles
Ex 5-1
Ex 5-2

FIG. 5

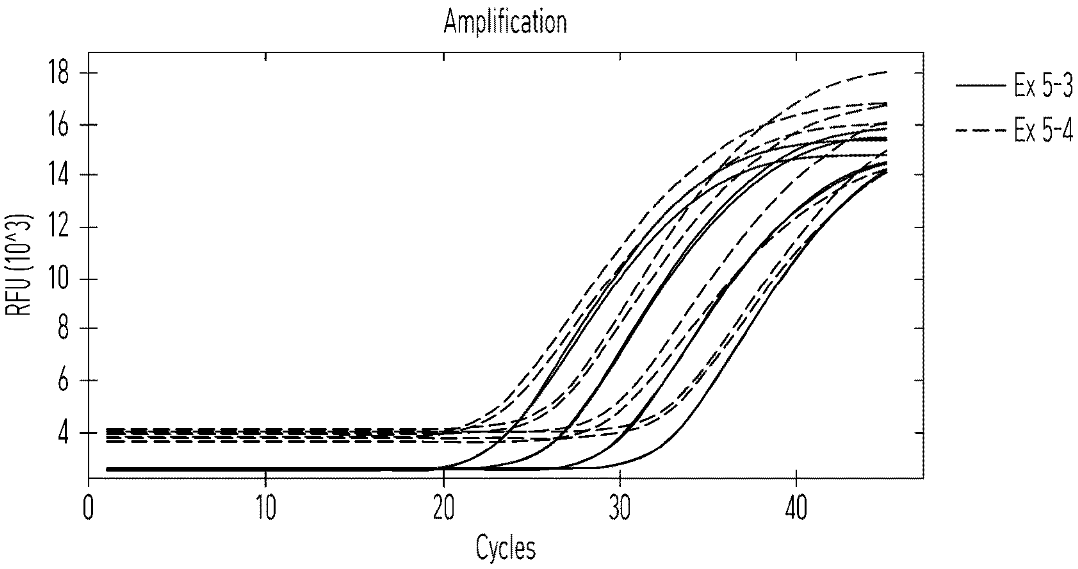
Amplification
RFU (10^3)
18
16
14
12
10
8
6
4
0
10
20
30
40
Cycles
Ex 5-3
Ex 5-4

FIG. 6

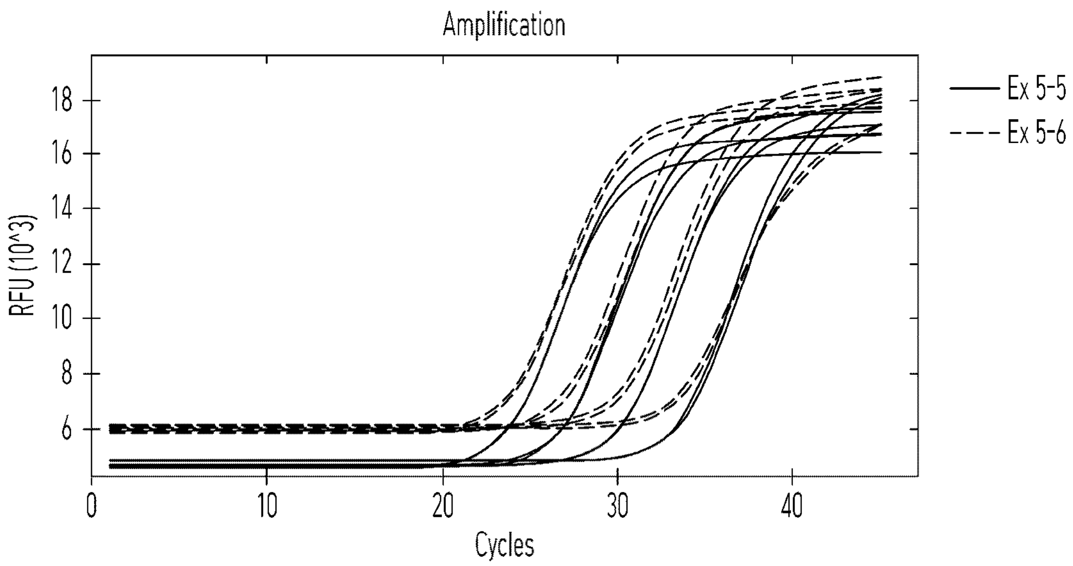
Amplification
RFU (10^3)
18
16
14
12
10
8
6
0
10
20
30
40
Cycles
Ex 5-5
Ex 5-6

FIG. 7

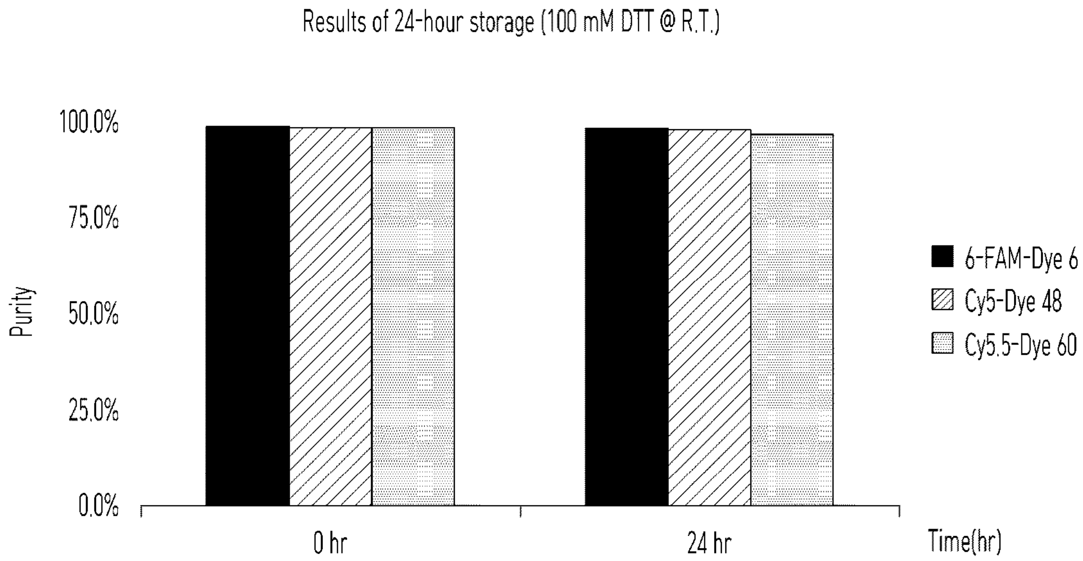
Results of 24-hour storage (100 mM DTT @ R.T.)
100.0%
75.0%
50.0%
25.0%
0.0%
Purity
0 hr
24 hr
Time(hr)
6-FAM-Dye 6
Cy5-Dye 48
Cy5.5-Dye 60

FIG. 8

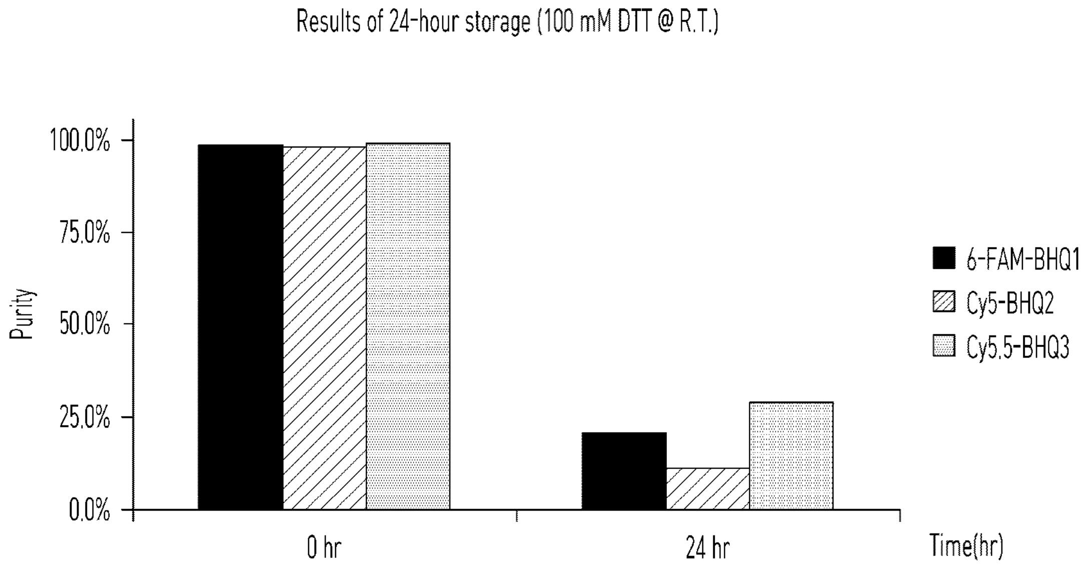
Results of 24-hour storage (100 mM DTT @ R.T.)
100.0%
75.0%
50.0%
25.0%
0.0%
Purity
0 hr
24 hr
Time(hr)
6-FAM-BHQ1
Cy5-BHQ2
Cy5.5-BHQ3

FIG. 9

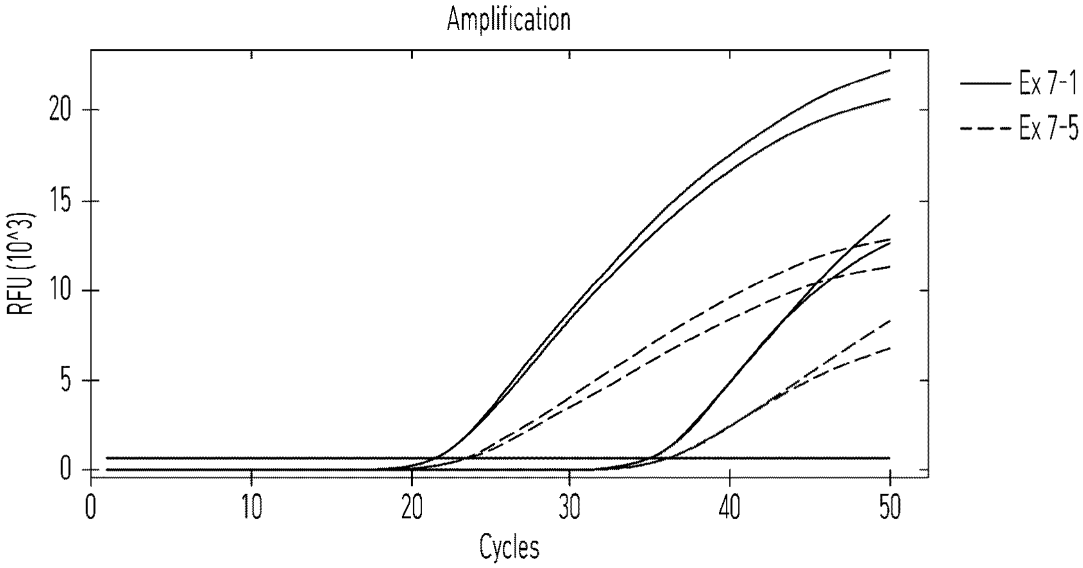
Amplification
RFU (10^3)
Cycles
Ex 7-1
Ex 7-5
0
5
10
15
20
0
10
20
30
40
50

FIG. 10

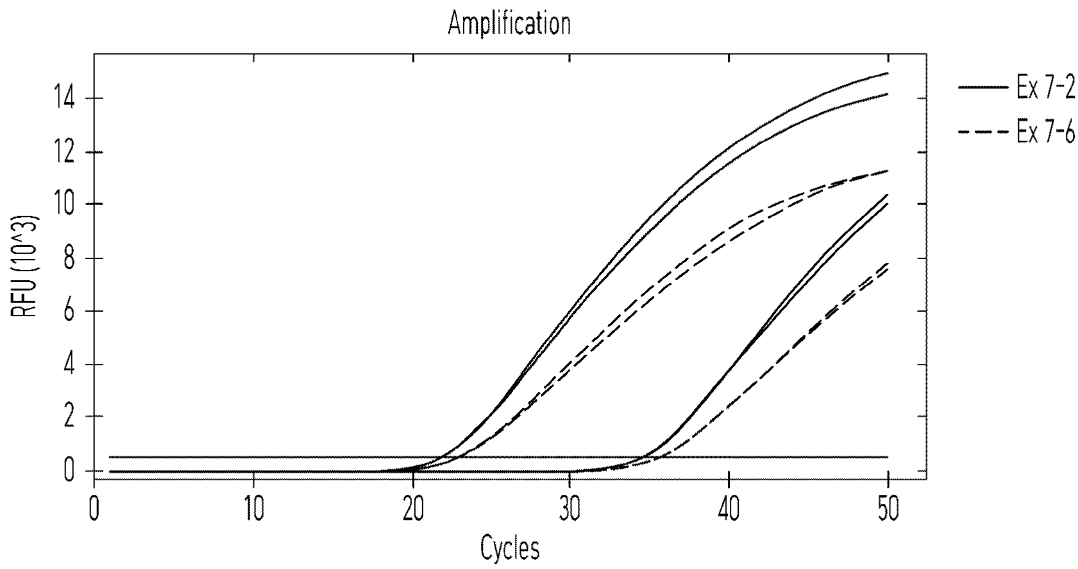
Amplification
RFU (10^3)
Cycles
Ex 7-2
Ex 7-6
0
2
4
6
8
10
12
14
0
10
20
30
40
50

FIG. 11

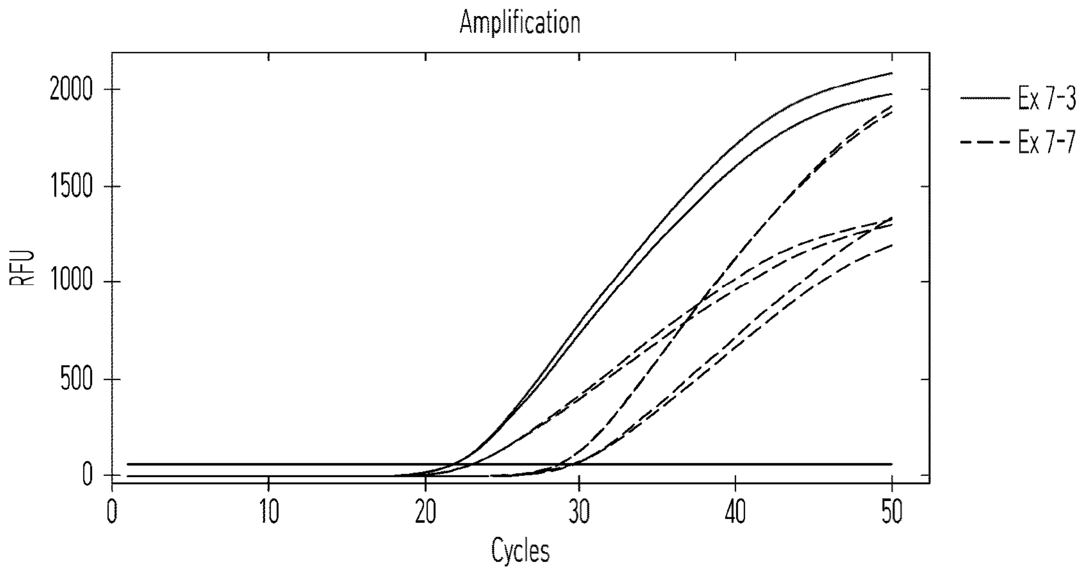
Amplification
2000
1500
1000
500
0
RFU
0
10
20
30
40
50
Cycles
Ex 7-3
Ex 7-7

FIG. 12

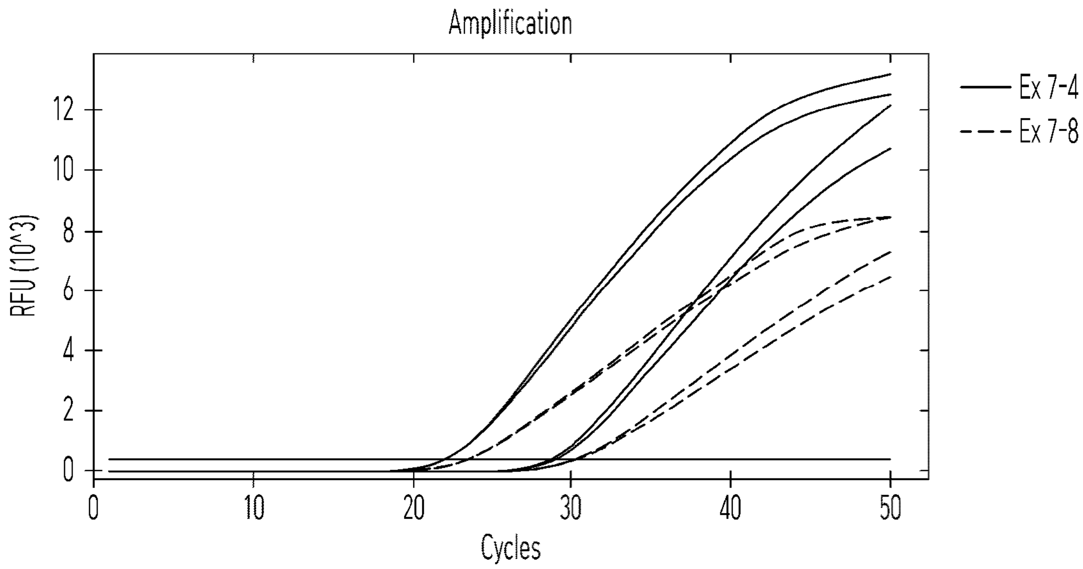
Amplification
12
10
8
6
4
2
0
RFU (10^3)
0
10
20
30
40
50
Cycles
Ex 7-4
Ex 7-8

FIG. 13

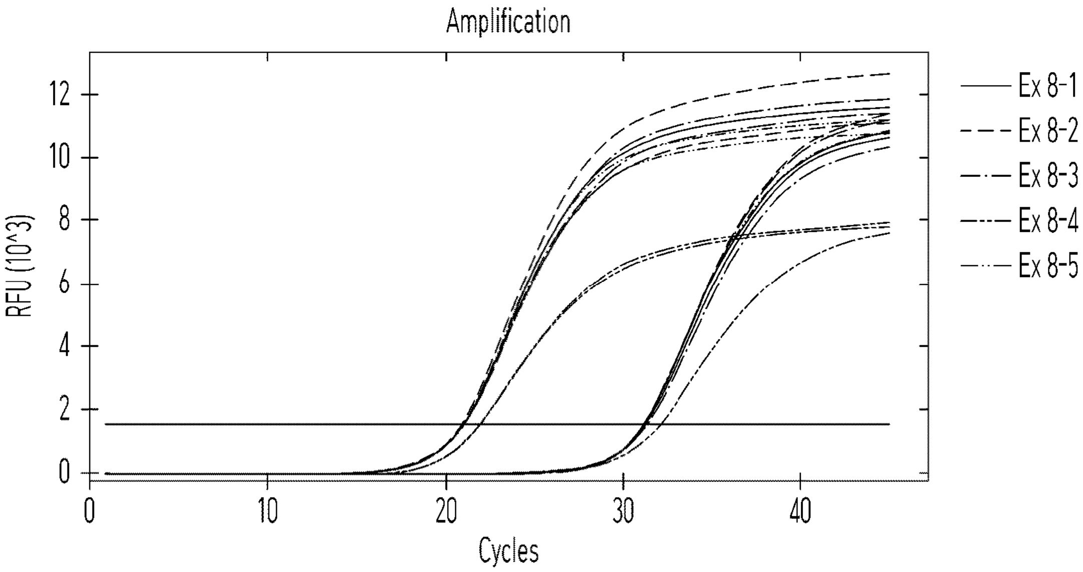
Amplification
RFU (10^3)
12
10
8
6
4
2
0
0
10
20
30
40
Cycles
Ex 8-1
Ex 8-2
Ex 8-3
Ex 8-4
Ex 8-5

**INTERNATIONAL SEARCH REPORT**

International application No.
**PCT/KR2024/005155**

**A. CLASSIFICATION OF SUBJECT MATTER**

**C07D 413/06**(2006.01)i; **C07D 417/06**(2006.01)i; **C07D 403/06**(2006.01)i; **C07D 403/14**(2006.01)i; **C07D 401/14**(2006.01)i; **C07D 487/04**(2006.01)i; **C07D 513/04**(2006.01)i; **C07D 495/04**(2006.01)i; **C07F 9/572**(2006.01)i; **C07D 498/04**(2006.01)i

According to International Patent Classification (IPC) or to both national classification and IPC

**B. FIELDS SEARCHED**

Minimum documentation searched (classification system followed by classification symbols)

C07D 413/06(2006.01); C08G 18/48(2006.01); C08G 18/75(2006.01); C09B 23/00(2006.01); C09B 23/08(2006.01); C09B 69/06(2006.01); C12N 15/09(2006.01); C12Q 1/37(2006.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), PubChem & keywords: 비형광성 화합물 (non-fluorescent compound), 소광 (quenching), 컨쥬게이트 (conjugate), 핵산 검출 (nucleic acid detection)

**C. DOCUMENTS CONSIDERED TO BE RELEVANT**

| Category* | Citation of document, with indication, where appropriate, of the relevant passages | Relevant to claim No. |
|---|---|---|
| X | WO 2007-005222 A2 (LI-COR, INC. et al.) 11 January 2007 (2007-01-11) See claim 1; and paragraphs [0005], [0023], [0060], [0084], [0085], [0099], [0100], [0119], [0138], [0154] and [0201]. | 1,2,5-20 |
| Y | | 21-24 |
| A | | 3,4 |
| Y | KR 10-1590175 B1 (SEEGENE, INC.) 29 January 2016 (2016-01-29) See claims 1 and 36; and paragraph [0184]. | 21-24 |
| A | WO 2020-066881 A1 (HAYASHIBARA CO., LTD.) 02 April 2020. See entire document. | 1-24 |
| A | WO 2022-200192 A1 (HOCHSCHULE NIEDERRHEIN) 29 September 2022 (2022-09-29) See entire document. | 1-24 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 July 2024** | **18 July 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/005155**

**C. DOCUMENTS CONSIDERED TO BE RELEVANT**

| Category* | Citation of document, with indication, where appropriate, of the relevant passages | Relevant to claim No. |
| --- | --- | --- |
| A | JP 2011-016879 A (ADEKA CORP.) 27 January 2011 (2011-01-27) See entire document. | 1-24 |

Form PCT/ISA/210 (second sheet) (July 2022)

**INTERNATIONAL SEARCH REPORT**

International application No.
**PCT/KR2024/005155**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2024/005155**

| Patent document cited in search report | Publication date (day/month/year) | Patent family member(s) | Publication date (day/month/year) |
|---|---|---|---|
| WO 2007-005222 A2 | 11 January 2007 | CA 2613386 A1 | 11 January 2007 |
| | | CA 2613386 C | 12 May 2015 |
| | | EP 1910554 A2 | 16 April 2008 |
| | | EP 1910554 B1 | 02 April 2014 |
| | | US 2007-0042398 A1 | 22 February 2007 |
| | | US 8227621 B2 | 24 July 2012 |
| | | WO 2007-005222 A3 | 13 September 2007 |
| | | WO 2007-005222 A8 | 17 January 2008 |
| KR 10-1590175 B1 | 29 January 2016 | EP 2480689 A1 | 01 August 2012 |
| | | EP 2480689 A4 | 15 May 2013 |
| | | JP 2013-505718 A | 21 February 2013 |
| | | JP 2015-133975 A | 27 July 2015 |
| | | JP 6099684 B2 | 22 March 2017 |
| | | KR 10-2012-0057657 A | 05 June 2012 |
| | | US 2012-0190030 A1 | 26 July 2012 |
| | | US 9447457 B2 | 20 September 2016 |
| | | WO 2011-037306 A1 | 31 March 2011 |
| WO 2020-066881 A1 | 02 April 2020 | JP 2020-050610 A | 02 April 2020 |
| | | TW 202214308 A | 16 April 2022 |
| WO 2022-200192 A1 | 29 September 2022 | DE 102021106964 A1 | 22 September 2022 |
| JP 2011-016879 A | 27 January 2011 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 11155713 B
- WO 2022191485 A1
- US 10473666 B2
- US 2011152538 A1
- US 9150922 B
- US 2015 A
- US 91509222015 B2
- US 200277487 A1
- KR 202067733 A
- CN 113336743 A
- US 20200224257 A1
- US 2022274960 A1
- KR 202067732 A
- WO 2017146187 A1
- EP 3882242 A1
- US 2020369703 A1
- KR 201690242 A
- CN 115304539 A
- KR 2015130206
- KR 2015130206 A1
- US 202259772 A1
- IN 202241025324
- EP 1221465 A1
- US 21667361937 A

### Non-patent literature cited in the description

- **WOJCIECHOWSKI, FILIP** ; **E. HUDSON** ; **ROBERT H**. *Current Topics in Medicinal Chemistry*, 2007, vol. 7 (7), 667-679
- *Dyes and Pigments*, 2012, vol. 93 (1-3), 1506-1511
- *Journal of Photochemistry and Photobiology A: Chemistry*, 2008, vol. 200 (2-3), 438-444
- *Organic and Biomolecular Chemistry*, 2011, vol. 9 (11), 4199-4204
- *Angewandte Chemie - International Edition*, 2020, vol. 59 (10), 3948-3951
- *Journal of the American Chemical Society*, 2001, vol. 123 (2), 361-362
- *Journal of Medicinal Chemistry*, 2022, vol. 65 (1), 811-823
- *Journal of the American Chemical Society*, 2011, vol. 133 (1), 51-55
- *Bioconjugate Chemistry*, 2019, vol. 30 (10), 2647-2663
- *Chemical Communications*, 2016, vol. 52 (90), 13307-13310
- *Bioconjugate Chemistry*, 2019, vol. 30 (10), 2647-2663
- *Journal of Organic Chemistry*, 2018, vol. 83 (8), 4389-4401
- *Dyes and Pigments*, 2022, vol. 197
- *Organic Letters*, 2019, vol. 21 (14), 5694-5698
- *Journal of Organic Chemistry USSR*, 1982, vol. 18 (2), 380-386
- *Journal of the American Chemical Society*, 2004, vol. 126 (27), 8364-8365
- *Bioorganic and Medicinal Chemistry*, 2016, vol. 24 (1), 26-32
- *Angewandte Chemie International Edition*, 2009, vol. 48, 4222-4225
- *Organic Letters*, 2001, vol. 3 (16), 2591-2594
- *Chemistry of Heterocyclic Compounds*, 1988, vol. 24, 87-92
- *Angew. Chem. Int. Ed*, 2009, vol. 48, 4222-4225
- *Journal of the Indian Chemical Society*, 1968, vol. 45, 799-809
- *Chemical Communications*, 2016, vol. 52 (90), 13307-13310
- *Journal of Organic Chemistry*, 2018, vol. 83 (8), 4389-4401
- *Dyes and Pigments*, 2014, vol. 101, 1-8
- *Journal of the American Chemical Society*, 2011, vol. 133 (40), 15870-15873